(19) Europäisches Patentamt
European Patent Office
Office européen des brevets

(11) Publication number: **0 376 040 A2**

# (12) EUROPEAN PATENT APPLICATION

(21) Application number: 89122840.5

(22) Date of filing: 11.12.89

(51) Int. Cl.5: C07K 5/06, C07K 5/08, C07K 5/02, A61K 37/64

(30) Priority: 27.12.88 US 289899

(43) Date of publication of application: 04.07.90 Bulletin 90/27

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Albright, Jay Donald
5 Clifford Court
Nanuet New York 10954(US)
Inventor: Sum, Fuk-Wah
5 Dixwell Road
New City New York 10956(US)
Inventor: Howell, Charles Frederick
28 Drake Lane
Upper Saddle River New Jersey 07458(US)

(74) Representative: Wächtershäuser, Günter, Dr.
Tal 29
D-8000 München 2(DE)

## (54) N-phosphinyl di-and tripeptides as renin inhibitors.

(57) The invention is new renin inhibitor dipeptide and tripeptide derivatives of the formula:

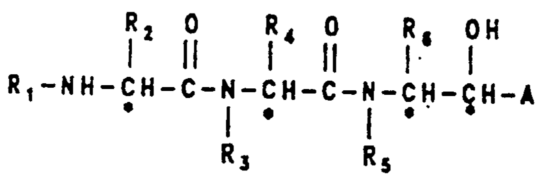

EP 0 376 040 A2

# RENIN INHIBITORS

## SUMMARY OF THE INVENTION

This invention relates to new dipeptide derivatives selected from those of general formula I,

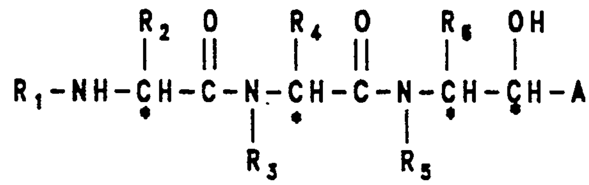

Formula I

wherein $R_1$ is

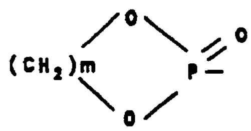 , 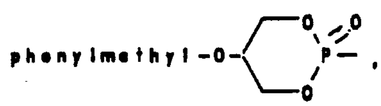 ,

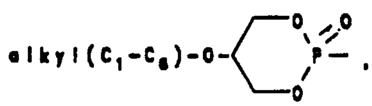 ,

$[alkyl(C_1\text{-}C_6)O]_2\overset{O}{\overset{\|}{P}}\text{-}$,

$(phenyl\text{-}O)_2\overset{O}{\overset{\|}{P}}\text{-}$,

$[alkyl(C_1\text{-}C_6)][alkyl(C_1\text{-}C_6)O]\overset{O}{\overset{\|}{P}}\text{-}$,

$(phenylmethyl\text{-}O)_2\overset{O}{\overset{\|}{P}}\text{-}$,

$[alkyl(C_1\text{-}C_6)O][phenyl\text{-}O]\overset{O}{\overset{\|}{P}}\text{-}$,

$[phenyl(CH_2)_n][alkyl(C_1\text{-}C_6)O]\overset{O}{\overset{\|}{P}}\text{-}$,

$[phenyl\ (CH_2)_n][phenyl\text{-}O]\overset{O}{\overset{\|}{P}}\text{-}$,

$[alkyl(C_1\text{-}C_6)][phenylmethyl\text{-}O]\overset{O}{\overset{\|}{P}}\text{-}$,

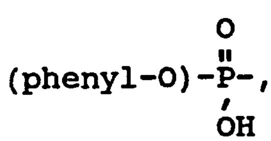

R-L-prolyl, R-L-thioprolyl, R-L-seryl, R-L-methionyl, R-L-alanyl, R-L-phenylalanyl, R-L-(S-benzyl)cysteinyl, R- L -histidyl or R-glycyl, where n is an integer 1-4; m is an integer 2-4 and R is selected from one of the groups defined by $R_1$; $R_2$ is phenylmethyl, $-CH_2$(2-thienyl), $-CH_2$(3-indolyl), 4-methoxybenzyl or $-CH_2$-naphthyl; $R_3$ is hydrogen or methyl; $R_4$ is 4-(imidazolyl)$CH_2X$-, alkyl($C_1$-$C_8$), $-(CH_2)_n-NH_2$, phenylmethyl, cyclohexylmethyl, -X-alkyl($C_1$-$C_8$), X-cyclohexyl, $-(CH_2)_n$-X-alkyl($C_1$-$C_3$), $-X-CH_2CH_2N$[alkyl-($C_1$-$C_3$)]$_2$ (where X is -O- or -S-) and moieties of the formulae:

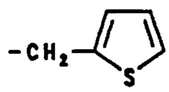
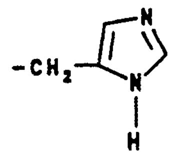
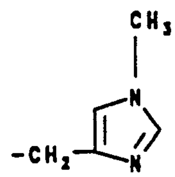

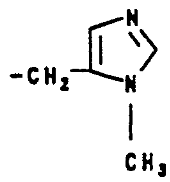
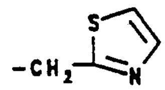
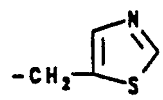

or 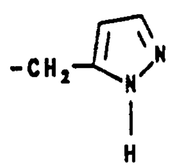

$R_5$ is hydrogen or methyl; $R_6$ is alkyl($C_1$-$C_6$), phenylmethyl, cyclohexylmethyl, $-(CH_2)_n$ -X-alkyl($C_1$-$C_3$) or

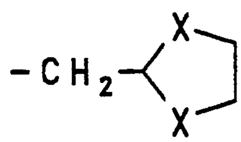

and A is

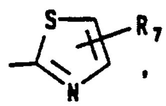, 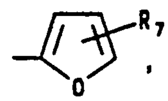, 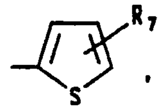, 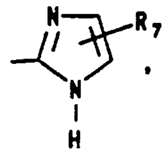,

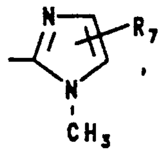, 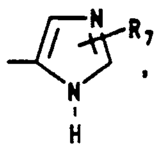, 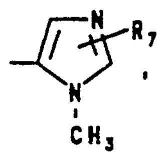, 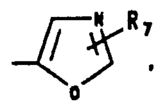,

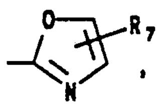, 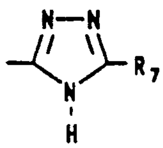, 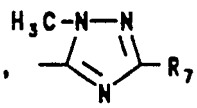, 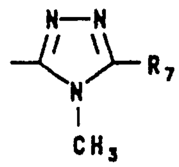,

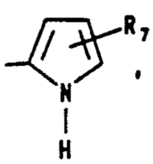, 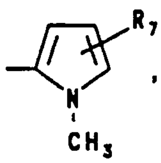, 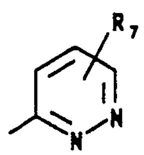, 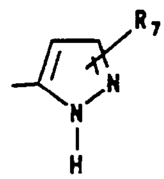,

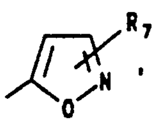, 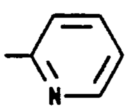, 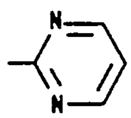, 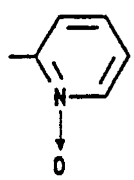,

or 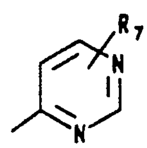

where $R_7$ is hydrogen, alkyl($C_1$-$C_3$), Br, Cl, F or $COR_8$, where $R_8$ is H, $CF_3$, $NH_2$, OH, -O-alkyl($C_1$-$C_4$), -NH-alkyl($C_1$-$C_4$), alkyl($C_1$-$C_6$), $CH_2OH$, -N[alkyl($C_1$-$C_3$)]$_2$ or

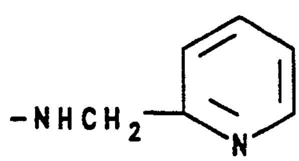.

In formula I, the asterisks denote asymmetric carbon atoms.

## DETAILED DESCRIPTION OF THE INVENTION

Within the group of compounds defined by formula I, certain subgroups of compounds are preferred. Broadly preferred are those compounds where the α-amino acids have the natural L configuration. Thus the most preferred are those compounds where the dipeptide or the tripeptide unit in formula I has the L, L or the L, L, L configuration. Especially preferred in the C-terminal units are compounds where the C-terminal units are selected from those of formula II,

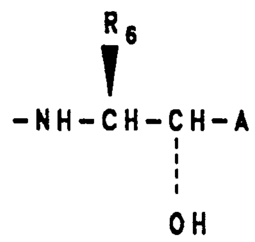

Formula II

with an anti(threo) relationship between the amino group and the hydroxyl group. Most preferred of the 1-amino-2-hydroxy compounds of formula II are those diastereomers with the 1s configuration.

Most preferred of the compounds of formula I, wherein the C terminal group is represented by formula II, are those compounds wherein $R_1$ is

$[alkyl(C_1-C_6)O]_2-\overset{O}{\overset{\|}{P}}-$,

$(phenyl-O)_2\overset{O}{\overset{\|}{P}}-$,

$[alkyl(C_1-C_6)][alkyl(C_1-C_6)O]\overset{O}{\overset{\|}{P}}-$,

$(phenylmethyl-O)_2\overset{O}{\overset{\|}{P}}-$,

$[alkyl(C_1-C_6)O]\ [phenyl-O]\ \overset{O}{\overset{\|}{P}}-$; $R_2$ is phenylmethyl, (3-indolyl)$CH_2$, (2-thienyl)$CH_2$ or $CH_2$-naphthyl; $R_3$ is hydrogen; $R_4$ is alkyl($C_1$-$C_8$),

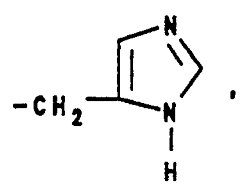 ,

O-alkyl($C_1$-$C_8$), S-alkyl($C_1$-$C_8$) or O-$CH_2CH_2N$[alkyl($C_1$-$C_3$)]$_2$; $R_5$ is hydrogen; $R_6$ is selected from the group consisting of alkyl($C_1$-$C_8$), cyclohexylmethyl or

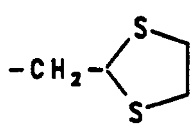 ; A is 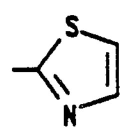 , 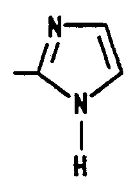 , 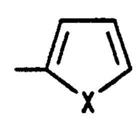 ,

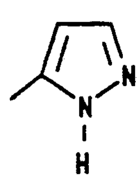 or 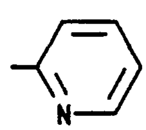

$R_7$ is as previously defined; and $R_8$ is $NH_2$, OH, -O-alkyl ($C_1$-$C_4$), NH-alkyl($C_1$-$C_4$) or lower alkyl($C_1$-$C_3$).

The products of formula I and the preferred subgroups can be prepared by various synthetic procedures.

For example, the products can be prepared by reacting an N-protected dipeptide of formula III:

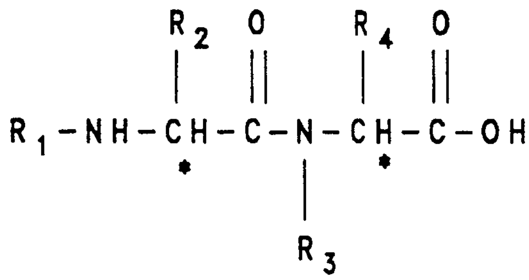

Formula III

or its chemical equivalent with a 1-amino-2-hydroxy compound of formula IV,

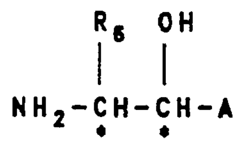

Formula IV

Thus a dipeptide or tripeptide of formula III is reacted with a peptide coupling reagent to convert the carboxyl group into an activated derivative which is then reacted with a compound of formula IV, or its chemical equivalent to give the products of this invention.

Preferred peptide coupling reagents are those which do not cause racemization at the carbons designated with asterisks. For example, appropriate peptide coupling reagents are:

1) N,N′-Dicyclohexylcarbodiimide plus 1-hydroxybenzotriazole
2) Benzotriazol-1-yloxytris (dimethylamino)phosphonium hexafluorophosphate (BOP-reagent)
3) N,N′-Bis[2-oxo-3-oxazolidinyl]phosphorodiamidic chloride (BOP-Cl)
4) Diphenylphosphinyl chloride (DPP-Cl)
5) Diethoxyphosphoryl cyanide
6) 2-Chloro-1-methylpyridinium iodide
7) Phenyl dichlorophosphate plus imidazole

In the compounds of formula III, where $R_4$ is 4-imidazolylmethyl, the imidazole nitrogen is blocked with an appropriate group such as tosyl, 2,4-dinitrophenyl, benzyl, or benzyloxymethyl, prior to coupling with compounds of formula IV. A suitable blocking group is chosen so that conditions for its removal are compatible with other structural features in the product of formula I.

Alternatively a dipeptide or tripeptide of formula III is activated with an appropriate peptide coupling reagent and then reacted with a compound of formula V in which the hydroxy group is protected with a removable blocking group Y. Suitable blocking groups are represented by trimethylsilyl, t-butyldimethylsilyl, tetrahydropyranyl, acetyl, benzoyl and the like. Removal of the hydroxyl blocking group then gives the compounds of formula I:

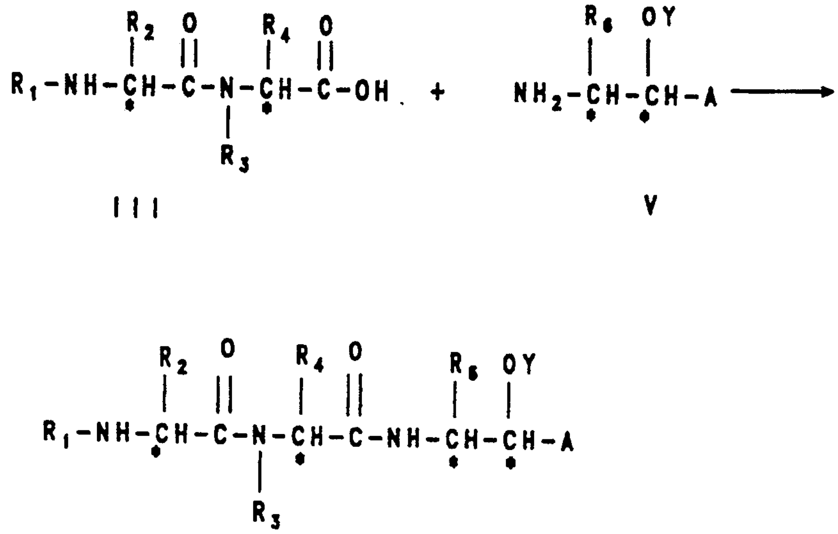

III

V

I(blocked)

Alternatively N-blocked peptide aldehydes of formula VI are prepared and reacted with a compound of formula VII where $M^+$ is a metal such as sodium, potassium, lithium and the like to give compounds of formula I.

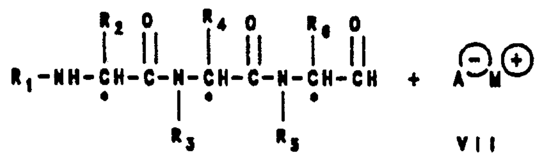

VII

VI

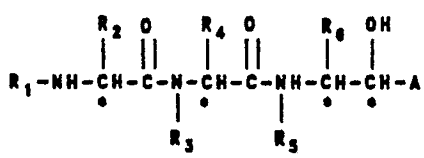

I

Preferably the peptide aldehydes of formula VI are reacted with trimethylsilyl derivatives of formula VIII in the presence of tetrabutylammonium fluoride, cesium fluoride or an equivalent source of fluoride anion giving the O-trimethylsilyl derivatives of formula IX or the desilylated derivatives of formula IX.

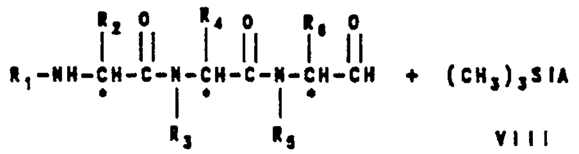

VIII

VI

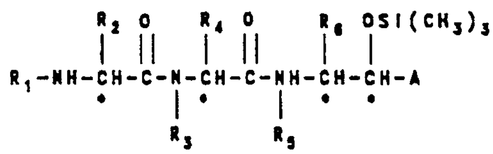

IX

Removal of the trimethylsilyl group under standard conditions such as treatment with dilute acid or tetrabutylammonium fluoride gives the compounds of formula I.

The intermediates of formula XI or XII are prepared by reacting an N-blocked-α-amino aldehyde of formula X wherein $R_9$ is t-butyloxycarbonyl, benzyloxycarbonyl, triphenylmethyl and the like with compounds of formula VII or VIII.

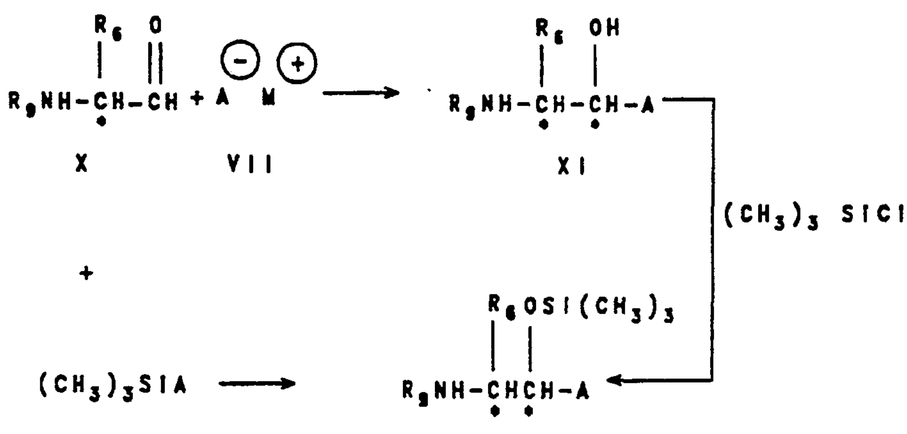


X VII XI VIII XII

The most preferred compounds of X are those with the L (S) configuration which give a pair of diastereomers on reaction with compound VII or VIII. The pair of diastereomers may be separated by chromatography on silica gel, and coupled to a peptide moiety of formula III as previously described.

As representative examples of the above process (Scheme A), 2-trimethylsilylthiazole 1 is reacted with L-t-butoxycarbonylphenylalanal 2 or L-t-butoxyxcarbonylleucinal 3 in dichloromethane to give diastereomers 4, 5 and 6, 7 respectively. Removal of the trimethyl silyl group with fluoride anion affords the free hydroxy compounds 4b, 5b, and 6b, 7b respectively. Separation of the diastereomers is carried out by chromatography. The most preferred diastereomers are (S)-2(tert-butoxycarbonyl)amino-3-phenyl-(R)-1-(2-thiazolyl)propan-1-ol 4b and (S)-2-(tert-butoxycarbonyl)amino-4-methyl-(R)-1-(2-thiazolyl)pentan-1-ol 6b.

## Scheme A

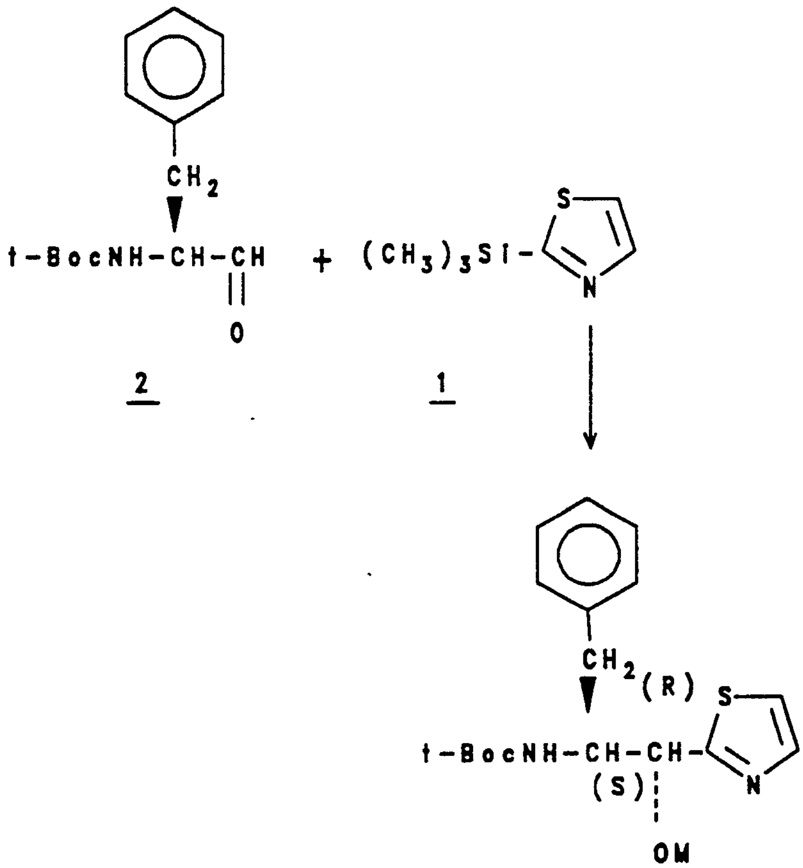

2

1

4a M=Si($CH_3$)$_3$
4b M=H

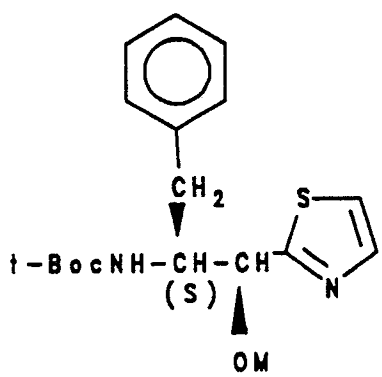

5a M=Si($CH_3$)$_3$
5b M=H

Scheme A (continued)

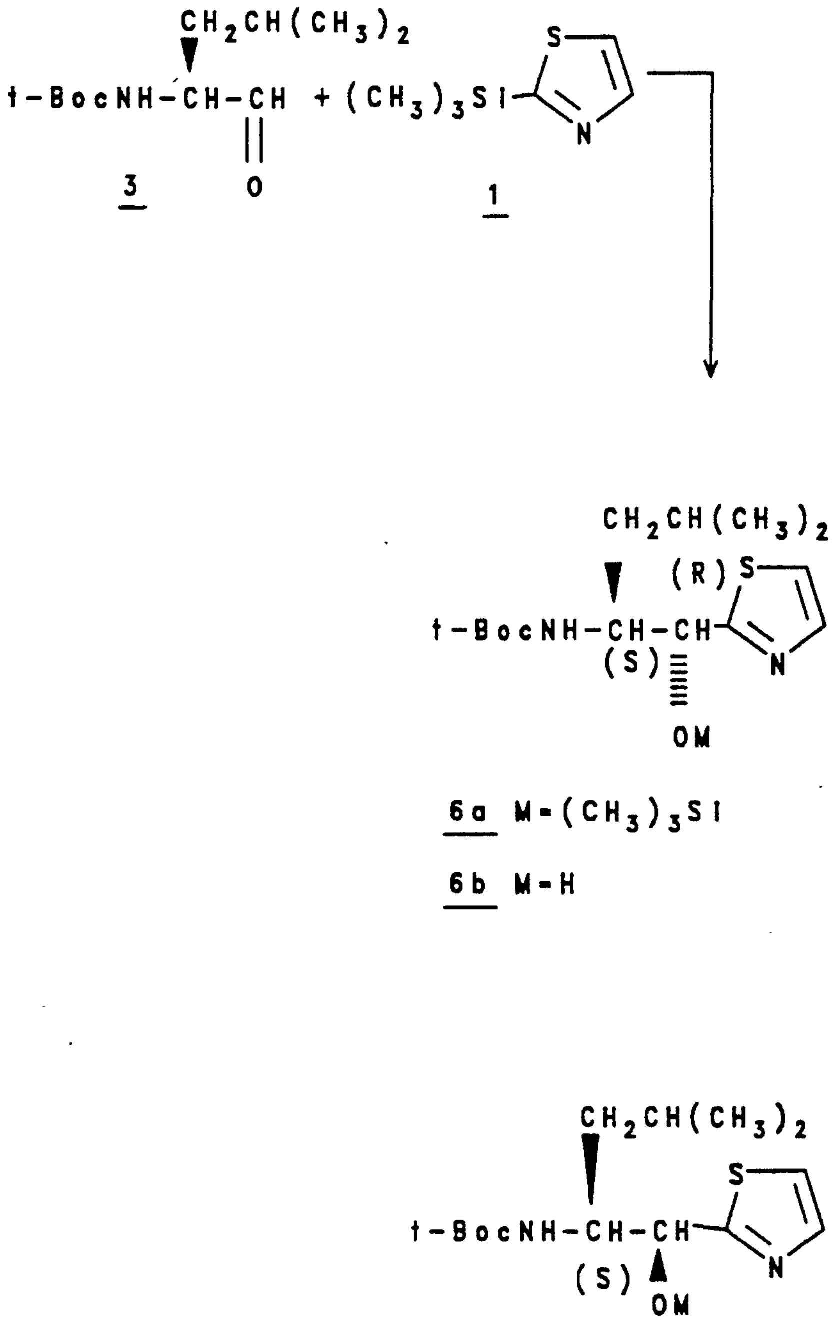

3

1

6a $M=(CH_3)_3Si$

6b $M=H$

7a $M=(CH_3)_3Si$

7b $M=H$

Alternatively, the compounds of formula I may be prepared by coupling sequentially N-blocked α-amino acids of formula XIII to compounds of formula V. The derivatives (Formula XIV) are then deblocked to give compounds of formula XV which are coupled with the N-terminal unit to give compounds of formula XVI, Scheme B. The hydroxyl blocking group (Y) is then removed to afford compounds of formula I. The sequence of reactions may be carried out wherein the blocking group (Y) is removed first and the

derivatives of formula XVII, containing a free hydroxyl group are coupled to the N-terminal unit of formula XVIII. The coupling reactions (Scheme B) may also be carried out with the compounds of formula V wherein the hydroxyl group is not blocked (Y = H) to give derivatives XIV(Y = H) and XV(Y = H).

## Scheme B

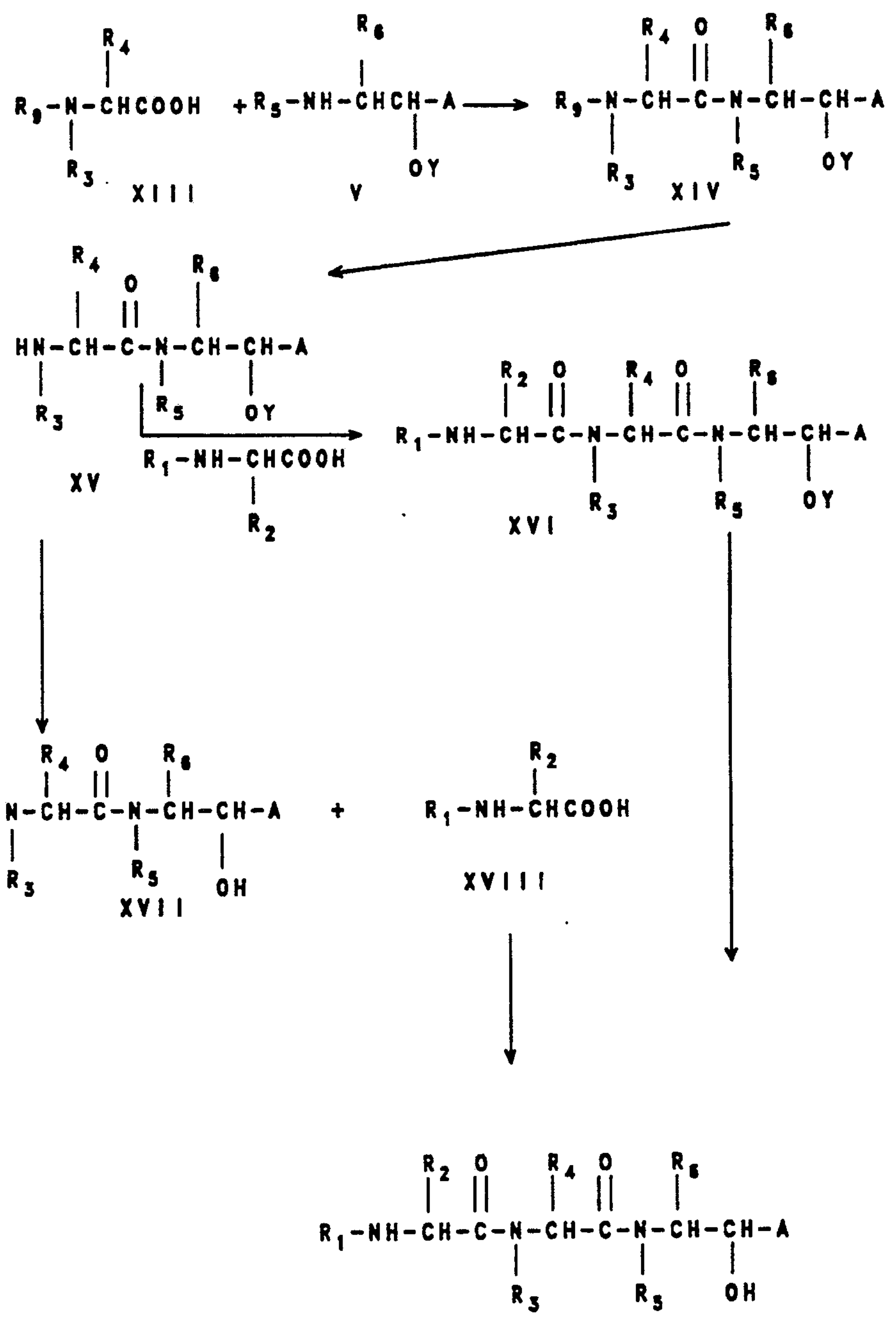

XIII V XIV

XV XVI

XVII XVIII

I

The compounds of formula I are conveniently prepared by first synthesizing the intermediates wherein

$R_1$ is a t-butoxycarbonyl or a benzyloxycarbonyl group, then removing the blocking group and reacting the free amino derivative with a diester of phosphorochloridic acid or a mono-ester of a phosphonochloridic acid.

The compounds of formula I are active inhibitors of renin.

Renin is an endopeptidase which plays an important role in the control of blood pressure. The renin angiotensin system is a multiregulated proteolytic cascade in which renin cleaves the protein substrate angiotensinogen to give angiotensin I. Angiotensin converting enzyme (ACE) catalyses the removal of the terminal dipeptide from the the decapeptide angiotensin I to form angiotensin II which exhibits potent pressor activity.

Renin is an aspartyl protease with high substrate specificity and is the first proteolytic step in the renin-angiotensin system which is involved in the control of blood pressure.

Renin inhibitors have been shown to lower blood pressure in primates, [J. Hypertension, 1, 399 (1983), J. Hypertension 1 (suppl 2), 189 (1983)] and in man, [Lancet II, 1486 (1983), Trans. Assoc. Am. Physicians, 96, 365 (1983), J. Hypertension, 3, 653 (1985)] and thus are potentially useful in the control of hypertension.

The novel compounds of formula I are new peptide renin inhibitors and are useful in the treatment of hypertension in warm-blooded animals, as established in the following test.

## Radioimmunoassay Screen For Renin Inhibitors

The *in vitro* method for the screening of anti-renin compounds involves, first, angiotensin I generation, and second, the quantitation of the angiotensin I produced by radioimmunoassay.

### Angiotensin I Generation

The incubation medium consisted of 20 µl of purified human plasma angiotensinogen (1); 10 µl of human kidney renin (2); 5 µl of phenylmethylsulfonyl fluoride; 10 µl of disodium EDTA (10 mM); 10 µl of antirenin compound ($5 \times 10^{-3}$, $5 \times 10^{-4}$, $5 \times 10^{-5}$) in dimethylformamide, or ethanol and a suitable amount of maleate buffer (77mM, pH 6.0) to make a final volume of 500 µl. The reaction mixture was incubated for one hour at 37° C and the enzymatic reaction was stopped by placing the tube in ice-cold water. The angiotensin I generated during the incubation was measured by a radioimmunoassay plasma renin activity kit (Clinical Assays, Inc.).

### Radioimmunoassay Procedure

The incubation medium consisted of either 100 µl aliquots of the above reaction mixture or a standard amount of angiotensin I; 1000 µl of phosphate buffer (100 mM, pH7.6) and 100 µl of ($^{125}$I)angiotensin in a gamma-coat, tube. After three hours of incubation at room temperature, the tubes were decanted, and the radioactivity of each tube was determined in a gamma counter. Duplicate determinations were performed for each incubation. The results were expressed in ng of angiotensin I generated per ml of generation medium per hour of incubation (ng/AI/ml/hr).

The results of this test on representative compounds of this invention appear in Table I, expressed as an $IC_{50}$.

(1) The human plasma angiotensinogen derived from the blood of a woman receiving oral contraceptive pills was purified by chromatography on a pepstatin-aminohexyl-agarose column.

(2) Human renin was prepared from human kidney.

TABLE I

N-Phosphinyl Di-and Tripeptides as Renin Inhibitors

| Compound | $IC_{50}$ Molar Concentration |
| --- | --- |
| N-[N-(Diethoxyphosphinyl)-L-phenylalanyl-L-leucyl]-(S)-2-amino-4-methyl-(R)-1-(2-thiazolyl)-pentan-1-ol | $4.2\times10^{-8}$ |
| N-[N-(Diphenoxyphosphinyl)-L-phenylalanyl-L-leucyl]-(S)-2-amino-4-methyl-(R)-1-(2-thiazolyl)-pentan-1-ol | $1.2\times10^{-6}$ |
| N-[N-[[Methoxy][phenoxy]phosphinyl]-L-phenylalanyl-L-leucyl]-(S)-2-amino-4-methyl-(R)-1-(2-thiazolyl)-pentan-1-ol | $6.0\times10^{-8}$ |
| N-[N-(R or S)[[Methoxy][phenylmethoxy]phosphinyl]-L-phenylalanyl-L-leucyl]-(S)-2-amino-3-cyclohexyl-(R)-1-(2-thiazolyl)propan-1-ol | $5.2\times10^{-9}$ |
| N-[N-(R or S)[[Methoxy][phenylmethoxy]phosphinyl]-L-phenylalanyl-L-leucyl]-(S)-2-amino-3-cyclohexyl-(R)-1-(2-thiazolyl)propan-1-ol | $6.0\times10^{-9}$ |
| N-[N-[[Hydroxy][phenoxy]phosphinyl]-L-phenylalanyl-L-leucyl]-(S)-2-amino-3-cyclohexyl-(R)-1-(2-thiazolyl)propan-1-ol | $5.4\times10^{-7}$ |
| N-[N-(Diethoxyphosphinyl)-L-phenylalanyl-L-leucyl]-(S)-2-amino-3-cyclohexyl-(R)-1-(2-thiazolyl)propan-1-ol | $2.6\times10^{-9}$ |
| N-[N-(Diethoxyphosphinyl)-L-phenylalanyl-L-leucyl]-(S)-2-amino-3-cyclohexyl-(R)-1-(2-furanyl)propan-1-ol | $1.7\times10^{-9}$ |
| N-[N-(Diethoxyphosphinyl)-L-phenylalanyl-L-leucyl]-(S)-2-amino-3-cyclohexyl-(R)-1-(2-imidazolyl)propan-1-ol | $1.1\times10^{-7}$ |

TABLE I (continued)

| Compound | $IC_{50}$ Molar Concentration |
|---|---|
| N-[N-(Diethoxyphosphinyl)-L-phenylalanyl-L-leucyl]-(S)-2-amino-3-cyclohexyl-(R)-1-(2-pyridinyl)propan-1-ol | $8.9x10^{-8}$ |
| N-[N-(Diethoxyphosphinyl)-L-phenylalanyl-L-leucyl]-(S)-2-amino-3-cyclohexyl-(R)-1-(5-methoxycarbonyl-2-thiazolyl)propan-1-ol | $2.6x10^{-9}$ |
| N-[N-(Diethoxyphosphinyl)-L-phenylalanyl-L-leucyl]-(S)-2-amino-3-cyclohexyl-(R)-1-(5-acetyl-2-furanyl)-propan-1-ol | $1.5x10^{-8}$ |
| N-[N-(Dibenzyloxyphosphinyl)-L-phenylalanyl-L-leucyl]-(S)-2-amino-3-cyclohexyl-(R)-1-(2-thiazolyl)propan-1-ol | $4.7x10^{-10}$ |
| N-[N-[(5-Methoxy-1,3,2-dioxaphosphorin-2-yl)-P-oxide]-L-phenylalanyl-L-leucyl]-(S)-2-amino-3-cyclohexyl-(R)-1-(2-thiazolyl)propan-1-ol | $1.7x10^{-7}$ |
| N-[N-Diethoxyphosphinyl]-L-phenylalanyl-L-histidyl]-(S)-2-amino-3-cyclohexyl-(R)-1-(2-imidazolyl)-propan-1-ol | $5.3x10^{-8}$ |
| N-[N-(Diethoxyphosphinyl)-L-phenylalanyl-L-histidyl]-(S)-2-amino-3-cyclohexyl-(R)-1-(2-thiazolyl)propan-1-ol | $5.1x10^{-9}$ |
| N-[N-(Diethoxyphosphinyl)-L-phenylalanyl-L-histidyl]-(S)-2-amino-3-cyclohexyl-(R)-1-(2-pyridinyl)propan-1-ol | $1.4x10^{-6}$ |

TABLE I (continued)

| Compound | $IC_{50}$ Molar Concentration |
|---|---|
| N-[N-(Diethoxyphosphinyl)-L-phenylalanyl-L-leucyl]-(S)-2-amino-3-cyclohexyl-(R)-1-(1H-pyrazol-3-yl)propan-1-ol | $2.8x10^{-9}$ |
| N-[N-(Diethoxyphosphinyl)-L-(4-methoxyphenyl)alanyl-L-leucyl]-(S)-2-amino-3-cyclohexyl-(R)-1-(2-furanyl)-propan-1-ol | $1.8x10^{-8}$ |

The novel compounds of the present invention have been found to be highly useful for lowering elevated blood pressure in mammals when administered in amounts ranging from about 5 mg to about 50 mg/kg of body weight per day.

The compounds of this invention are preferably administered by a parenteral route such as intravenous, intramuscular or subcutaneous, but may be administered orally if desired.

Compositions, according to the present invention, having the desired clarity, stability and adaptability for parenteral use are obtained by dissolving from 0.10% to 10% by weight of active compound in a vehicle consisting of a polyhydric aliphatic alcohol or mixtures of such alcohols. Especially satisfactory are glycerin, propylene glycol and polyethylene glycols. Although various mixtures of polyethylene glycols may be used, it is preferred to use a mixture having an average molecular weight of from about 200 to about 400.

In addition to the active compound, the parenteral solutions may also contain various preservatives to prevent bacterial and fungal contamination as well antioxidants to promote stability.

For intramuscular injection, the preferred concentration of active compound is 0.25 to 0.50 mg/ml of the finished compositions.

The novel compounds of this invention are equally adapted to intravenous administration when diluted with water or diluents employed in intravenous therapy such as isotonic glucose. For intravenous use, initial concentrations down to about 0.05 to 0.25 mg/ml of active compound are satisfactory.

The following specific examples illustrate the preparation of the compounds of this invention.

## Reference Example 1

### $N^{\alpha}$-[(Phenylmethoxy)carbonyl]- L -phenylalanimamide

An 18 g portion of $N^{\alpha}$-(phenylmethoxy)carbonyl] L-phenylalanine and 6.6 ml of N-methylmorpholine were dissolved in 150 ml of tetrahydrofuran and stirred at -10 °C as 8.04 ml of isobutylchloroformate was added. After one minute, ammonia gas was bubbled through the mixture until it was saturated. The mixture was stirred an additional 30 minutes and then poured into ice-water. The white solid was collected, giving 16.76 g of the desired compound, mp 164-165 °C.

## Reference Example 2

N-[(Phenylmethoxy)carbonyl]- L -phenylalanyl- D , L -2-hydroxyglycine

A solution of 16.8 g of $N^{\alpha}$-[phenylmethoxy)carbonyl]- L -phenylalaninamide, and 6.06 g of glyoxylic acid in 90 ml of acetone was refluxed for 6 hours, then poured into ice-water and stirred. The resulting white solid was collected and dried, giving 16.8 g of the desired compound, mp 137-141 ° C.

Reference Example 3

N-[(Phenylmethoxy)carbonyl]- L -phenylalanyl- D , L -2-(2-methylpropoxy)glycine, 2-methylpropyl ester

A mixture of 9.2 g of N-[(phenylmethoxy)carbonyl]- L -phenylalanyl- D , L -2-hydroxyglycine in 120 ml of isobutanol containing 0.5 ml of concentrated sulfuric acid was stirred for 30 minutes, then warmed on a steam bath for 4 hours, cooled and poured into cold saturated sodium bicarbonate solution. This mixture was extracted three times with ethyl acetate. The extracts were combined, dried, filtered and evaporated, giving a solid which was recrystallized from ethanol, giving 7.3 g of the desired compound. After recrystallization from aqueous ethanol the solid melted at 120-123 ° C.

Reference Example 4

N-[(Phenylmethoxy)carbonyl]- L -phenylalanyl- D , L -2-(2-methylpropoxy)glycine

A solution comprising 7.3 g of N-[(phenylmethoxy)carbonyl]- L -phenylalanyl- D , L -2-(2-methylpropoxy)glycine, 2-methylpropyl ester, 15 ml of 1N sodium hydroxide and 150 ml of methanol was stirred for 15 hours then acidified with 3N hydrochloric acid and evaporated to an oil. The addition of water produced a solid which was collected, giving 6.1 g of the desired compound, mp 99-109 ° C.

Reference Example 5

N-Methoxy-N-methyl- L -phenylalaninamide, trifluoroacetate

To 21.75 g of N-methoxy-N-methyl- L -(t-butoxycarbonyl)phenylalaninamide, chilled in an ice-methanol bath, was added with stirring, 50 ml of trifluoroacetic acid. After three hours the solvent was removed in vacuo. Ether was added twice and the solvent removed, then 100 ml of ether was added and the mixture was chilled. The resulting solid was collected, washed with ether and air dried, giving 19 g of the desired compound as crystals, mp 123-124 ° C.

Reference Example 6

N-[(Phenylmethoxy)carbonyl]- L -phenylalanyl- L -2-(2-methylpropoxy)glycyl-N-methoxy-N-methyl- L -phenylalaninamide, and N-[(Phenylmethoxy)carbonyl]- L -phenylalanyl- D -2-(2-methylpropoxy)glycyl-N-methoxy-N-methyl- L -phenylalaninamide

To a solution of 3 g of N-[(phenylmethoxy)carbonyl]- L-phenylalanyl- D, L -2-(2-methylpropoxy)-glycine, 1.73 g of 2-ethoxy-1(2H)-quinolinecarboxylic acid, ethyl ester and 70 ml of dichloromethane, was added a solution of 2.25 g of N-methoxy-N-methyl- L-phenylalaninamide trifluoroacetate and 1.2 ml of triethylamine in 35 ml of dichloromethane. This mixture was stirred for 20 hours and then evaporated. Chloroform was added to the residual oil and this solution was then washed three times with 3N hydrochloric acid, three times with saturated sodium bicarbonate solution and three times with saturated sodium chloride solution, then dried, filtered and evaporated giving 4.8 g of an oil. This oil was combined with 5.7 g of oil prepared in the same manner, placed on a 30x2 cm silica gel column and eluted with ethyl acetate:hexane (1:1).

The second fraction gave 1.16 g of N-[(phenylmethoxy)carbonyl]- L-phenylalanyl- L-2-(2-methylpropoxy)glycyl-N-methoxy-N-methyl- L-phenylalaninamide as an amorphous solid, Rf=7.55; $[\alpha]_D^{26}$ -11° ±3 (c = 0.35, methanol).

After eliminating intermediate fractions the second compound N-[(phenylmethoxy)carbonyl]- L -phenylalanyl- D -2-(2-methylpropoxy)glycyl-N-methoxy-N-methyl- L -phenylalaninamide was collected as 1.25 g of amorphous solid, Rf=0.33; $[\alpha]_D^{26}$ 0° (c = 0.3, methanol).

## Reference Example 7

### N-[(Phenylmethoxy)carbonyl]- L-phenylalanyl-N-[(S)-1-formyl-2-phenylethyl]- L-2-(2-methylpropoxy)-glycinamide

A solution of 1.16 g of N-[(phenylmethoxy)carbonyl]- L-phenylalanyl- L-2-(2-methylpropoxy)glycyl-N-methoxy-N-methyl- L-phenylalaninamide in 20 ml of ether and sufficient tetrahydrofuran to provide solution was added dropwise to a mixture of 0.1 g of lithium aluminum hydride in 20 ml of ether, with stirring under an argon atmosphere. When additon was complete, stirring was continued 4 hours longer, then the mixture was cooled and treated with a solution of 0.48 g of potassium bisulfate in 20 ml of water. More ether and water were added, the layers separated and the aqueous layer extracted three times with ether. The ether extracts and layer were combined, washed three times each with 3N hydrochloric acid, sodium bicarbonate solution and saturated sodium chloride solution, dried, filtered and evaporated, giving 0.53 g of the desired compound as a glass, $[\alpha]_D^{26}$ -19° ±3 (c = 0.32, methanol).

## Reference Example 8

### N-[(Phenylmethoxy)carbonyl]- L-phenylalanyl-N-[(S)-1-formyl-2-phenylethyl]- D-2-(2-methylpropoxy)-glycinamide

A 1.2 g portion of N-[(phenylmethoxy)carbonyl]- L-phenylalanyl- D-2-(2-methylpropoxy)glycyl-N-methoxy-N-methyl- L-phenylalaninamide was treated with lithium aluminum hydride in tetrahydrofuran as described in Reference Example 7, giving 0.76 g of the desired compound as a glass, which became an amorphous solid after trituration with hexane $[\alpha]_D^{26}$ 0° (c = 0.3, methanol).

## Reference Example 9

### N-Methoxy-N-methyl- L -leucinamide, trifluoroacetate

A mixture of 2 g of N-methoxy-N-methyl-t-butoxycarbonyl- L-leucinamide and 20 ml of trifluoroacetate

acid:water (9:1) was stirred at 0°C for 4 hours. The solvent was removed in vacuo and the oily residue triturated with ether. The resulting solid was collected and air dried, giving 1.7 g of the desired compound as a white solid, mp 96-98°C.

## Reference Example 10

N-[(Phenylmethoxy)carbonyl]- L-phenylalanyl-2- L-(2-methylpropoxy)glycyl-N-methoxy-N-methyl- L-leucinamide and N-[(Phenylmethoxy)carbonyl]- L-phenylalanyl- D-2-(2-methylpropoxy)glycyl-N-methoxy-N-methyl- L-leucinamide

To a solution of 3 g of N-[(phenylmethoxy)carbonyl]- L-phenylalanyl-(D, L)-2-(2-methylpropoxy)-glycine, 1.73 g of 2-ethoxy-1(2H)-quinolinecarboxylic acid, ethyl ester and 70 ml of dichloromethane, was added a solution of 2.08 g of N-methoxy-N-methyl- L-leucinamide, trifluoroacetate and 1.2 ml of triethylamine in 35 ml of dichloromethane. This mixture was stirred for 17 hours and then evaporated. Chloroform was added to the residual oil and the resulting solution was washed three times each with 3N hydrochloric acid, saturated sodium bicarbonate solution and saturated sodium chloride solution, then dried, filtered and evaporated. The residual oil was placed on a silica gel column (30x2 cm) and eluted with ethyl acetate:hexane (1:1), collecting 25 ml fractions. Fractons 8-10 were combined and evaporated, giving 0.76 g of N-[phenylmethoxy)carbonyl]- L-phenylalanyl- L-2-(2-methylpropoxy)glycyl-N-methoxy-N-methyl- L-leucinamide as an oil, Rf=0.60, $[\alpha]_D^{26}$ -29° ±3 (c = 0.37, methanol).

Fractions 12-17 were combined and evaporated, giving 1.16 g of N-[(phenylmethoxy)carbonyl]- L-phenylalanyl- D-2-(2-methylpropoxy)glycyl-N-methoxy-N-methyl- L-leucinamide as an oil, Rf=0.43; $[\alpha]_D^{26}$ -7° ±2 (c = 0.635, methanol).

## Reference Example 11

N-[(Phenylmethoxy)carbonyl]- L-phenylalanyl-N-[(S)-1-formyl-3-methylbutyl]- L-2-(2-methylpropoxy)-glycinamide

A solution of 0.74 g of N-[(phenylmethoxy)carbonyl]- L-phenylalanyl- L-2-(2-methylpropoxy)glycyl-N-methoxy-N-methyl- L-leucinamide in 10 ml of ether and 10 ml of tetrahydrofuran was added dropwise to a stirred mixture of lithium aluminum hydride in 20 ml of ether under an argon atmosphere. When addition was complete, the mixture was stirred 4 hours longer, then cooled and treated with a solution of 0.31 g of potassium bisulfate in 15 ml of water. The aqueous layer was separated, extracted with ether and the ether extracts combined with the ether layer. The ether solution was washed three times each with 6N hydrochloric acid, saturated sodium bicarbonate solution and saturated sodium chloride solution, dried, filtered and evaporated. The resulting glass was triturated with hexane, giving 0.28 g of the desired compound as a white amorphous solid $[\alpha]_D^{26}$ -23° ±7 (c = 0.13, methanol).

## Reference Example 12

N-[(Phenylmethoxy)carbonyl]- L-phenylalanyl-N-[(S)-1-formyl-3-methylbutyl]- D-2-(2-methylpropoxy)-glycinamide

A 1.16 g portion of N-[(phenylmethoxy)carbonyl]- L-phenylalanyl- D-2-(2-methylpropoxy)glycyl-N-methoxy-N-methyl- L-leucinamide was reacted with lithium aluminum hydride in ether as described in

Reference Example 7, giving 0.75 g of the desired compound as an amorphous solid, $[\alpha]_D^{26}$ 0° (c = 0.1, methanol).

## Reference Example 13

### N-[(Phenylmethoxy)carbonyl]-L-phenylalanyl-D, L-2-methoxyglycine, methyl ester

A solution of 0.4 g of N-[(phenylmethoxy)carbonyl]-L-phenylalanyl-D, L-2-hydroxyglycine, 50 ml of methanol and 0.3 ml of concentrated sulfuric acid was stirred for 2 days and then poured into cold saturated sodium bicarbonate solution. The resulting suspension was extracted with ethyl acetate. The extract was dried, filtered and evaporated. The residue was recrystallized from aqueous methanol, giving 0.25 g of the desired compound, mp 128-132° C.

## Reference Example 14

### N-[(Phenylmethoxy)carbonyl]-L-phenylalanyl-D, L-2-methoxyglycine

A solution of 0.47 g of D, L-2-methoxy-N-[(N-[(phenylmethoxy)carbonyl] L-phenylalanyl]glycine, methyl ester in 15 ml of methanol and 1.2 ml of 1N sodium hydroxide was stirred for one hour, then acidified and evaporated. Water was added to the residue. The resulting solid was recrystallized from aqueous methanol, giving 0.2 g of the desired compound as a solid, mp 75-90° C.

## Reference Example 15

### N-[(Phenylmethoxy)carbonyl]-L-phenylalanyl-D, L-2-ethoxyglycine, ethyl ester

A solution of 3.7 g of N-[(phenylmethoxy)carbonyl]-L-phenylalanyl-D, L-2-hydroxyglycine in 50 ml of ethanol and 0.3 ml of concentrated sulfuric acid was stirred for 18 hours and then refluxed for 48 hours. The clear solution was poured into cold saturated sodium bicarbonate solution and then extracted three times with ethyl acetate. The extracts were combined, washed with water, dried, filtered and evaporated. The oily residue solidified and was recrystallized from chloroform/hexane, giving 1.7 g of the desired compound, mp 99-110° C (dec).

## Reference Example 16

### N-[Phenylmethoxy)carbonyl]-L-phenylalanyl-D, L-2-ethoxyglycine

A solution of 1.5 g of N-[(phenylmethoxy)carbonyl]-L-phenylalanyl-D, L-2-ethoxyglycine, ethyl ester, 50 ml of methanol and 3.5 ml of 1N sodium hydroxide was stirred overnight, then acidified with 6N hydrochloric acid and evaporated to an oil. Water was added and the mixture stirred until the oil solidified. One recrystallization from chloroform/hexane gave one g of the desired compound as a white solid, mp 138-143° C (dec.).

## Reference Example 17

N-[(Phenylmethoxy)carbonyl]- L -phenylalanyl- D , L -2-(1-methylethoxy)glycine, 1-methylethyl ester

A mixture of 3.7 g of N-[(phenylmethoxy)carbonyl]- L -phenylalanyl- D , L -2-hydroxyglycine, 50 ml of isopropanol and 0.3 ml of concentrated sulfuric acid was stirred at room temperature for 17 hours, warmed on a steam bath for 1/2 hour and then stirred at room temperature for 48 hours. This mixture was filtered and the filtrate evaporated to an oil which solidified after washing with sodium bicarbonate solution and water. This solid was chromatographed on a 30x2 cm silica gel column, eluting with ethyl acetate:hexane (1:1), collecting 25 ml fractions. The first two fractions were combined and evaporated, giving 0.6 g of the desired compound, mp 103-111 °C; Rf=0.87; $[\alpha]_D^{26}$ +5 ° ±1 (c = 1.1 methanol).

## Reference Example 18

N-[(Phenylmethoxy)carbonyl]- L -phenylalanyl-D, L -2-(1-methylethoxy)glycine

A solution of 1.4 g of N-[(phenylmethoxy)carbonyl]- L -phenylalanyl- D, L -2-(1-methylethoxy)glycine, 1-methylethyl ester, 45 ml of methanol and 3 ml of 1N sodium hydroxide was stirred for 20 hours, then acidified with 6N hydrochloric acid and evaporated. Water was added to the residue and the mixture was stirred until a white solid separated. This solid was recrystallized from chloroform/hexane, giving 0.7 g of the desired compound as a white solid, mp 122-128 °C (dec.).

## Reference Example 19

N-[(Phenylmethoxy)carbonyl]- L -phenylalanyl- L -2-(1-methylethoxy)glycyl-N-methoxy-N-methyl- L -leucinamide

A solution of 1.37 g of N-methoxy-N-methyl- L -leucinamide, trifluoroacetate, 0.7 ml of triethylamine and 10 ml of dichloromethane was added to a solution of 1.9 g of N-[(phenylmethoxy)carbonyl]- L -phenylalanyl- D , L -2-(1-methylethoxy)glycine and 1.14 g of 2-ethoxy-1(2H)-quinolinecarboxylic acid, ethyl ester in 25 ml of dichloromethane. This mixture was stirred for 18 hours and then evaporated to an oil. The oil in ethyl acetate was washed three times each with 3N hydrochloric acid, sodium bicarbonate solution and sodium chloride solution, dried, filtered and evaporated. The oily residue was chromatographed on a 40x3 cm silica gel column eluting with ethyl acetate:hexane, collecting 50 ml fractions. Fractions 4-6 were combined and evaporated, giving 0.9 g of the desired compound as an amorphous solid, Rf=0.5; $[\alpha]_D^{26}$ -45 ° ±2 (c = 0.645, methanol).

## Reference Example 20

N-[(Phenylmethoxy)carbonyl]- L -phenylalanyl-N-[(S)-1-formyl-2-phenylethyl]- L -2-(2-methylpropoxy)-glycinamide

To 1.2 g of N-[(phenylmethoxy)carbonyl]- L -phenylalanyl- L -2-(2-methylpropoxy)glycyl-N-methoxy-N-methyl-L-phenylalaninamide in 20 ml of ether, cooled to 0°C under argon, was added 0.18 g of lithium aluminum hydride. After 20 minutes, 0.09 g of lithium aluminum hydride was added. After 15 minutes ethyl acetate was added and the mixture was worked up as described in Reference Example 7. The resulting glass was crystallized from hexane containing a small amount of ethyl acetate, giving 0.47 g of the desired compound as a crystalline solid, mp 164-168°C. A sample recrystallized from ethyl acetate/hexane gave crystals mp 172-174°C; $[\alpha]_D^{26}$ -38° ±1 (c = 0.1, methanol).

## Reference Example 21

### N-[(Phenylmethoxy)carbonyl]-L-phenylalanyl-N-(S)-1-formyl-3-methylbutyl]-L-2-(1-methylethoxy)glycinamide

A solution of 0.7 g of N-[(phenylmethoxy)carbonyl]-L-phenylalanyl-L-2-(1-methylethoxy)glycyl-N-methoxy-N-methylK L-leucinamide in 10 ml of ether and 10 ml of tetrahydrofuran was added to a stirred mixture of 0.6 g of lithium aluminum hydride in 20 ml of ether under an argon atmosphere. This mixture was stirred 4 hours, then cooled and treated with a solution of 0.31 g of potassium bisulfate in 25 ml of water. The layers were separated, the aqueous layer extracted once with ether and this extract combined with the ether layer. The ether solution was washed three times each with 3N hydrochloric acid, sodium bicarbonate solution and saturated sodium chloride solution, dried, filtered and evaporated. The residual glass was slurried several times in hexane and then thoroughly dried giving the desired compound as an amorphous solid $[\alpha]_D^{26}$ -24° ±4 (c = 0.26, methanol).

## Reference Example 22

### N-[(Phenylmethoxy)carbonyl]-L-phenylalanyl-D, L-2-(phenylmethoxy)glycine, phenylmethyl ester

A suspension of 37 g of N-[(phenylmethoxy)carbonyl]-L-phenylalanyl-D, L-2-hydroxyglycine in 250 ml of benzyl alcohol was treated with 3 ml of concentrated sulfuric acid, heated on a steam bath for 1.5 hours, diluted with hexane and chilled. The solid was collected, washed with ether and recrystallized from ethyl acetate/ether, giving 27.5 g of the desired compound, mp 130-140°C.

## Reference Example 23

### N-[(Phenylmethoxy)carbonyl]-L-phenylalanyl-D, L-2-(phenylmethoxy)glycine

To a suspension of 29.9 g of N-[(phenylmethoxy)carbonyl]-L-phenylalanyl-D, L-2-(phenylmethoxy)-glycine, phenylmethyl ester in 500 ml of methanol was added 60 ml of 1N sodium hydroxide with stirring. Stirring was continued for 1.5 hours then the mixture was heated to boiling to produce solution, filtered and the filtrate was acidified to pH 1.5 with 1N hydrochloric acid followed by the addition of water to the cloud point. The resulting suspension was cooled and the solid was collected and dried, giving 22.34 g of the desired compound, mp 139-142°C.

## Reference Example 24

(-)-N-[(Phenylmethoxy)carbonyl]- L -phenylalanyl-[R-(or S)]-2-(phenylmethoxy)glycyl-N-methoxy-N-methyl- L -phenylalaninamide and (+)-N-[(Phenylmethoxy)carbonyl]- L -phenylalanyl-[R(or S)]-2-(phenylmethoxy)glycyl-N-methoxy-N-methyl- L -phenylalaninamide

To a suspension of 12.47 g of N-[(phenylmethoxy)carbonyl]- L -phenylalanyl- D , L -2-(phenylmethoxy)glycine in 140 ml of dichloromethane was added 7.4 g of 2-ethoxy-1(2H)-quinolinecarboxylic acid, ethyl ester. The mixture was stirred 5 minutes, then a solution of 8.7 g of N-methoxy-N-methyl- L -phenylalaninamide, trifluoroacetate and 3.8 ml of triethylamine in 50 ml of dichloromethane was added and stirring was continued overnight. The solution was concentrated to an oil which was dissolved in 100 ml of ethyl acetate, washed with 2N hydrochloric acid, sodium bicarbonate solution and brine, dried, filtered and concentrated to an oil.

This oil, in two batches, was chromatographed by HPLC on a Waters Prep 520 column (silica gel) with ethyl acetate:dichloromethane (1:4) as eluent. After 10 hold-back volumes, the least polar component was eluted with ethyl acetate:dichloromethane (1:1). These fractions were concentrated in vacuo and the residue was crystallized first from dichloromethane/diisopropyl ether, then from ethyl acetate/diisopropyl ether, giving 4.0 g of (+)-N-[(phenylmethoxy)carbonyl]- L -phenylalanyl-[R(or S)]-2-(phenylmethoxy)glycyl-N-methoxy-N-methyl- L -phenylalaninamide, mp 135-140° C; $[\alpha]_D^{26}$ +17° ±0.5 (c = 0.12, chloroform).

The fractions containing the more polar component were concentrated in vacuo and the residue crystallized from diisopropyl ether/dichloromethane, giving 5.73 g of (-)-N-[(phenylmethoxy)carbonyl]- L -phenylal anyl-[R(or S)]-2-(phenylmethoxy)glycyl-N-methoxy-N-methyl- L -phenylalaninamide, mp 138-139° C; $[\alpha]_D^{26}$ -30° ±1 (c = 1.385, methanol).

## Reference Example 25

N-[(Phenylmethoxy)carbonyl]- L -phenylalanyl- D -2-(phenylmethoxy)glycyl-N-methoxy-N-methyl- L -leucinamide and N-[(Phenylmethoxy)carbonyl]- L -phenylalanyl- L -2-(phenylmethoxy)glycyl-N-methoxy-N-methyl- L -leucinamide

To a suspension of 13.9 g of N-[(phenylmethoxy)carbonyl]- L -phenylalanyl- D , L -2-(phenylmethoxy)-glycine in 60 ml of dichloromethane was added 7.4 g of 2-ethoxy-1(2H)-quinolinecarboxylic acid, ethyl ester. After the solution became complete, a solution of 8.8 g of N-methoxy-N-methyl- L -leucinamide, trifluoroacetate and 4.1 ml of triethylamine in 58 ml of dichloromethane was added. This solution was stirred overnight, then washed three times each with 60 ml portions of 2N hydrochloric acid, 1N sodium bicarbonate and sodium chloride solution. The organic layer was dried, filtered through a thin pad of dichloromethane washed hydrous magnesium silicate and concentrated in vacuo, giving 16.1 g of an oil. This oil was chromatographed by HPLC on a Water Prep 520 column (silica gel) with ethyl acetate:dichloromethane (1:4) as solvent. Two components (A & B) were isolated from the two main peaks.

Fractions containing component A were combined and concentrated in vacuo, giving an oil which was crystallized from ether, giving 4.46 g of N-[(phenylmethoxy)carbonyl]- L -phenylalanyl- L -2-(phenylmethoxy)glycyl-N-methoxy-N-methyl- L leucinamide, as white crystals, mp 88-89° C; $[\alpha]_D^{26}$ -39° ±1 (c = 0.96, methanol).

Fractions containing component B were combined and the solvent removed in vacuo, giving 7.1 g of N-[(phenylmethoxy)carbonyl]- L -phenylalanyl- D -2-(phenylmethoxy)glycyl-N-methoxy-N-methyl- L -leucinamide as an oil.

## Reference Example 26

N-[(Phenylmethoxy)carbonyl]- L -phenylalanyl-N-[(S)-1-formyl-3-methylbutyl]- D -2-(phenylmethoxy)glycinamide

An 8 g portion N-[(phenylmethoxy)carbonyl]- L -phenylalanyl- D ,-2-(phenylmethoxy)glycyl-N-methoxy-N-methyl- L -leucinamide was dissolved in 50 ml of anhydrous ether. To 25 ml of this solution, under nitrogen, was added 0.38 g of lithium aluminum hydride. The mixture was stirred for one hour, then quenched in ethyl acetate, treated with 37 ml of 0.35M potassium bisulfate, filtered through diatomaceous earth and washed with ether. The combined ether solutions were then washed three times with 3N hydrochloric acid, twice with 1N sodium bicarbonate and once with sodium chloride solution. The ethereal solution was dried, filtered and evaporated. The solid was crystallized from ether, giving 0.9 g of the desired compound, mp 109-114° C; $[\alpha]_D^{26}$ + 0.5° ±1 (c = 1.05, methanol).

## Reference Example 27

### N-[(Phenylmethoxy)carbonyl]- L -phenylalanyl-N-[(S)-1-formyl-3-methylbutyl]- L -2-(phenylmethoxy) glycinamide

To a suspension of 3.3 g of N-[(phenylmethoxy)carbonyl]- L -phenylalanyl- L-2-(phenylmethoxy)-glycyl-N-methoxy-N-methyl- L-leucinamide in 50 ml of ether at 3° C under nitrogen, was added 0.52 g of lithium aluminum hydride. The mixture was maintained at 0-6° C and after 20 minutes 0.26 g of lithium aluminum hydride was added. The mixture was maintained at 0° C for 15 minutes, then ethyl acetate and dichloromethane were added, followed by three 20 ml portions of 3N hydrochloric acid. The organic layer was separated, washed with three 20 ml portions of 1N sodium bicarbonate and brine, dried, diluted with ethyl acetate and filtered through a short bed of 200-340 mesh silica gel. The silica gel was washed with ethyl acetate and the combined filtrate and wash concentrated to dryness in vacuo. The residue was crystallized from ethyl acetate/diisopropyl ether, giving 1.35 g of the desired compound as crystals, mp 134-137° C; $[\alpha]_D^{26}$ -44° ±1 (c = 1.25, methanol).

## Reference Example 28

### N-[(Phenylmethoxy)carbonyl]- L -phenylalanyl-D, L -2-(cyclohexylmethoxy)glycine, cyclohexylmethyl ester

A mixture of 30 g of N-[(phenylmethoxy)carbonyl]- L -phenylalanyl- D , L -2-hydroxyglycine, 100 g of cyclohexylmethanol and 2.4 ml of concentrated sulfuric acid was stirred and heated at 90-100° C for 4 hours. The mixture was then cooled and stirred overnight at room temperature. Hexane was added and the solid collected, washed with hexane, cold water, saturated sodium bicarbonate solution and again with water. This solid was chromatographed on a silica gel column, eluting with dichloromethane:ethyl acetate (98:2). The product fractions were combined, evaporated and the residue triturated with hexane. Filtration gave 19.1 g of the desired compound as white crystals, mp 122-125° C; $[\alpha]_D^{26}$ -8° ±1 (c = 1.14, acetone).

## Reference Example 29

### N-[(Phenylmethoxy)carbonyl]- L -phenylalanyl-D, L -2-(cyclohexyloxy)glycine, cyclohexyl ester

A mixture of 3.7 g of N-[(phenylmethoxy)carbonyl]- L -phenylalanyl- D , L -2-hydroxyglycine, 50 ml of cyclohexanol and 0.3 ml of concentrated sulfuric acid was heated on a steam bath for 2 hours, then cooled overnight. A 50 ml portion of ethyl acetate was added, the mixture washed with sodium bicarbonate solution, brine and dried. The solution was filtered through a thin pad of hydrous magnesium silicate and the filtrate concentrated in vacuo to an oil. The oil was crystallized from hexane with chilling and

recrystallized from methanol, giving 2.2 g of the desired compound as white crystals, mp 106-118° C.

Reference Example 30

N-[(Phenylmethoxy)carbonyl]- L -phenylalanyl-D, L -2-(cyclohexyloxy)glycine

To a solution of 4.5 g of N-[(phenylmethoxy)carbonyl]- L -phenylalanyl- D, L -2-(cyclohexyloxy)-glycine, cyclohexyl ester in 40 ml of warm methanol was added 9.2 ml of 1N sodium hydroxide. This mixture was stirred 2 hours, then acidified with 5 ml of 2N hydrochloric acid and diluted with water to the cloud point. The mixture was diluted further with water, stirred, chilled and the solid collected. This solid was recrystallized from chloroform/hexane, giving 3.28 g of the desired compound, mp 119-121° C; $[\alpha]_D^{26}$ -3° ±2 (c = 0.539, methanol).

Reference Example 31

(S)-2-(tert-Butoxycarbonyl)amino-3-phenyl-(R,S)-1-(2-thiazolyl)propan-1-ol

To a solution of 8.28 g of 2-bromothiazole in 100 ml of ether at -78° C, under argon, was added 21.7 ml of n-butyl lithium in hexane. The mixture was stirred at -78° C for 45 minutes then 6.8 ml of trimethylsilyl chloride was added. This mixture was stirred at -78° C for one hour, warmed to -30° C over one hour, then 0° C over 1/2 hour and kept at 0° C for one hour. The reaction was quenched with 50 ml of 50% saturated sodium bicarbonate and then diluted with 50 ml of ether. The ether solution was washed with 50 ml of brine, dried, filtered and evaporated, giving 10.81 g of 2-trimethylsilylthiazole as an amber colored oil.

A solution of 10.55 g of phenyl dichlorophosphate in 50 ml of dichloromethane was added dropwise to a solution of 17.0 g of imidazole in 150 ml of dichloromethane. The resulting suspension was stirred under argon for 20 minutes, then 13.27 g of $N^{\alpha}$-tert-butyloxycarbonyl- L -phenylalanine was added in small portions and stirring was continued for one hour. A 5.85 g portion of N,O-dimethylhydroxylamine hydrochloride was added, stirring continued for 21 hours, then the mixture was diluted with 100 ml of dichloromethane and washed with three 100 ml portions of 1N hydrochloric acid, 100 ml of water, two 100 ml portions of 50% saturated sodium bicarbonate solution and 100 ml of brine. The mixture was then dried and evaporated, giving 15.87 g of N-methoxy-N-methyl $N^{\alpha}$-t-butoxycarbonyl-L-phenylalaninamide.

A suspension of 2.38 g of lithium aluminum hydride in 300 ml of ether at 0° C under argon was prepared and stirred. To this was added slowly a solution of 15.5 g of N-methoxy-N-methyl $N^{\alpha}$-t-butoxycarbonyl- L -phenylalaninamide in 80 ml of tetrahydrofuran. This mixture was stirred at 0° C for 2 hours, then a solution of 37 g of potassium biphosphate in 200 ml of water was added slowly. When addition was complete the mixture was stirred at room temperature for 30 minutes. The organic phase was separated. The aqueous phase was extracted with two 200 ml portions of ether. The combined organic phase and extracts was washed with 200 ml portions of 1N hydrochloric acid, 200 ml of sodium bicarbonate solution and 200 ml of brine, dried and evaporated. The residue was washed with petroleum ether (30-60°) giving 10.1 g of white solid. To a solution of 3.74 g of this solid in 20 ml of dichloromethane was added 5.5 g of 2-trimethylsilylthiazole. After stirring for 1.5 hours, another 1.57 g of 2-trimethylsilylthia zole was added and stirring was continued for 20.5 hours. A 20 ml solution of 1N tetrabutylammonium fluoride in tetrahydrofuran was added, the mixture was stirred 10 minutes and then the solvents were evaporated. The residue was taken up in 100 ml of ethyl acetate, washed with two 50 ml portions of water, then 50 ml of brine, dried and evaporated, giving 5.83 g of the desired compound as an amber colored gum.

Reference Example 32

(1S,2S)2-(t-Butoxycarbonyl)amino-3-phenyl-1-(2-thiazolyl)propan 1-ol, O-benzoate

A 5.6 g portion of 2-(t-butoxycarbonyl)amino-3-phenyl-(R,S)-1-(2-thiazolyl)propan-1-ol was dissolved in 30 ml of ether and treated successively with 2.5 ml of triethylamine and 2.1 ml of benzoyl chloride. The resulting suspension was stirred for 3 days and then filtered. The filtrate was stirred with 30 ml of 50% saturated potassium carbonate for 1/2 hour and then diluted with 30 ml of ether. The ether phase was separated, washed with two 30 ml portions of 0.5N hydrochloric acid, 30 ml of saturated potassium carbonate solution and brine, dried and evaporated. The residual gum was chromatographed on silica gel as a dichloromethane solution and then eluted with ethyl acetate:hexane (1:6). Fraction 1 gave 0.96 g of the (1S)-diastereomer, $[\alpha]_D^{26}$ -24° ±1 (c = 0.66, chloroform).

Fractions 2 is described in Reference Example 36.

## Reference Example 33

(1S,2S)-2-Amino-3-phenyl-1-(2-thiazolyl)propan-1-ol

To a solution of 0.44 g of (S)-2-(t-butoxycarbonyl)amino-3-phenyl-(S)-1-(2-thiazolyl) propan-1-ol, O-benzoate in 10 ml of methanol was added 2 ml of 5N sodium hydroxide. The mixture was stirred for one hour, then diluted with 5 ml of water and the methanol evap orated. The aqueous remainder was extracted with 20 ml of ethyl acetate. The organic extract was washed with brine, dried and evaporated, giving 0.37 g of white solid. A 0.27 g portion of this solid was dissolved in 2 ml of dichloromethane, stirred with 0.8 ml of trifluoroacetic acid for 5 hours, then 12 ml of 1N sodium hydroxide saturated with sodium chloride was added. After stirring 15 minutes the mixture was extracted with two 20 ml portions of dichloromethane. The extracts were combined, washed with 5 ml of sodium chloride, saturated 1N sodium hydroxide and 5 ml of brine, dried and evaporated, giving 0.2 g of the desired compound as a colorless gum.

## Reference Example 34

t-Butoxycarbonyl- L -phenylalanyl- L -leucine

A solution of 7.5 ml of phenyl dichlorophosphate in 50 ml of dichloromethane was added dropwise to a solution of 17 g of imidazole in 150 ml of dichloromethane. The resulting suspension was stirred under argon for 20 minutes and then cooled in an ice bath. A 9.09 g portion of L -leucine, methyl ester, hydrochloride was added in small portions. When addition was complete, the cooling bath was removed and the mixture stirred at room temperature for one hour. A solution of 13.27 g of tert.-butyloxycarbonyl-L -phenylalanine in 20 ml of dichloromethane was added, the mixture was stirred 15 hours, diluted with 100 ml of dichloromethane, washed with three 100 ml portions of 2N hydrochloric acid, 100 ml of water, two 100 ml portions of 0.5N sodium hydroxide and 100 ml of brine, dried and evaporated giving 18.55 g of t-butoxycarbonyl- L -phenylalanyl- L -leucine, methyl ester.

A solution of 3.72 g of t-butyloxycarbonyl-L -phenylalanyl- L -leucine, methyl ester in 20 ml of methanol was cooled to 0° C. To this was added dropwise, over 1/2 hour, 12 ml of 1N sodium hydroxide. The mix ture was stirred at 0° C for 4 hours, then refrigerated at 5° C for 14 hours, followed by stirring at 0° C for 6 hours. The methanol was evaporated at reduced pressure and room temperature and the aqueous suspension acidified to pH one with diluted hydrochloric acid. The suspension was extracted with two 40 ml portions of dichloromethane. The extracts were combined, washed with brine, dried and evaporated. The residue was washed with hexanes, giving 3.48 g of the desired compound.

## Reference Example 35

t-Butoxycarbonyl- L -phenylalanyl- L -leucyl-(1S,2S)-2-Amino-3-phenyl-1-(2-thiazolyl)propan-1-ol

A 0.2 g portion of imidazole and 0.13 g of phenyl dichlorophosphate in 2 ml of dichloromethane was stirred for 1/2 hour, then cooled to -15° C. A 0.23 g portion of t-butoxycarbonyl- L -phenylalanyl- L -leucine was added and the mixture was stirred at -12° to -15° C for 40 minutes. A solution of 0.12 g of (1S,2S)-2-amino-3-phenyl-1-(2-thiazolyl)propan-1-ol in one ml of dichloromethane was slowly added and stirring was continued at -12° C to -15° C for 20 hours. A 10 ml portion of 1N hydrochloric acid was added at -15° C and the mixture was stirred 15 minutes, then extracted with 20 ml of ethyl acetate. The organic solution was washed with 10 ml of 0.5N hydrochloric acid, two 10 ml portions of 0.5N potassium hydroxide and 10 ml of brine, dried and evaporated, giving 0.27 g of the desired compound, $[\alpha]_D^{26}$ -47° ±1 (c = 1.0, chloroform).

Reference Example 36

(S)-2-(t-Butoxycarbonyl)amino-3-phenyl-(R)-1-(2-thiazolyl)propan-1-ol, O-benzoate

The chromatographed fraction 2, from Reference Example 32, gave 2.76 g of the desired diastereomer; $[\alpha]_D^{26}$ -58° ±1 (c = 1.0, chloroform).

Reference Example 37

t-Butoxycarbonyl- L -phenylalanyl- L -leucyl-(1R,2S)-2-amino-3-phenyl-1-(2-thiazolyl)propan-1-ol

To a solution of 0.12 g of t-butoxycarbonyl- L -phenylalanyl- L -leucyl(1R,2S)-2-amino-3-phenyl-1-(2-thiazolyl)propan-1-ol, O-benzoate in 30 ml of methanol, was added 0.3 ml of 5N sodium hydroxide. This mixture was stirred for one hour, then diluted with 5 ml of water and the methanol evaporated. The aqueous residue was extracted with 20 ml of ethyl acetate. The organic extract was washed with brine, dried and evaporated. The solid was washed with hexane, giving 0.115 g of the desired compound as off-white crystals; $[\alpha]_D^{26}$ -55° ±2 (c = 0.42, chloroform).

Reference Example 38

(S)-2-Amino-3-phenyl-(R)-1-(2-thiazolyl)propan-1-ol, O-benzoate, trifluoroacetate

A mixture of 0.22 g of (S)-2-(t-butoxycarbonyl)amino-3-phenyl-(R)-1-(2-thiazolyl)propan-1-ol, O-benzoate and 0.4 ml of trifluoroacetic acid in one ml of dichloromethane was stirred for 3 hours, then the volatiles were removed *in vacuo*, giving 0.23 g of the desired compound as a gum, which was used without further purification in Reference Example 39.

Reference Example 39

t-Butoxycarbonyl-L-phenylalanyl-L-leucyl-(1R,2S)-2-amino-3-phenyl-1-(2-thiazolyl)propan-1-ol, O-benzoate

A mixture of 0.25 g of imidazole and 0.16 g of phenyl dichlorophosphate in 3 ml of dichloromethane was stirred for 1/2 hour, then cooled to -15° C. A 0.27 g portion of t-butoxycarbonyl-L-phenylalanyl-L-leucine was added and the mixture was stirred at -12° C to -15° for 20 minutes. A 0.23 g portion of (S)-2-amino-3-phenyl-(R)-1-(2-thiazolyl)propan-1-ol, O-ben zoate, trifluoroacetate in 0.5 ml of dichloromethane was added and stirring continued for 18 hours at -12° C to -15° C. The reaction was quenched with 10 ml of 0.5N hydrochloric acid and diluted with 30 ml of ethyl acetate. The organic solution was washed with 10 ml of 0.5N hydrochloric acid, twice with 15 ml of saturated potassium carbonate solution, dried and evaporated. The residue was washed with hexane, then with ether:hexane (1:3). The solid was extracted into 10 ml of ethyl acetate, filtered and evaporated. The residue was washed with ether:hexane (1:5) giving 0.25 g of the desired compound as a white solid $[\alpha]_D^{26}$ -65° ±2 (c = 0.545, chloroform).

## Reference Example 40

t-Butoxycarbonyl-L-cyclohexylalaninal

A 5.13 g portion of L-cyclohexylalanine was dissolved in a mixture of 60 ml of p-dioxane, 30 ml of water and 30 ml of 1N sodium hydroxide. To this solution, stirred and cooled in an ice bath, was added 7.19 g of di-t-butyldicarbonate. A suspension formed after a few minutes and the mixture was stirred at 0° C for one hour, then concentrated in vacuo to about 40 ml. A 150 ml portion of ethyl acetate was added and the mixture was stirred and cooled in an ice bath while the pH was adjusted to 2 with potassium hydrogen sulfate. The aqueous layer was separated and extracted with two 75 ml portions of ethyl acetate. The organic layer and extracts were combined, washed with two 100 ml portions of brine, dried and evaporated in vacuo, giving 8.0 g of $N^{\alpha}$-t-butoxycarbonyl-L-cyclohexylalanine as a colorless gum; $[\alpha]_D^{26}$ -2° ±1 (c = 1.0, chloroform).

To a solution of 9.52 g of imidazole in 80 ml of dichloromethane under argon, was added a solution of 6.22 g of phenyl dichlorophosphate in 20 ml of dichloromethane. After stirring one hour, the mixture was cooled to 0° C and a solution of 7.6 g of $N^{\alpha}$-t-butoxycar bonyl-L-cyclohexylalanine in 20 ml of dichloromethane was added over 10 minutes. This mixture was stirred for 1.5 hours at 0° to 5° C, then 3.28 g of N,O-dimethylhydroxylamine, hydrochloride was added. This mixture was stirred for 19 hours at 0° to 5° C, then diluted with 100 ml of dichloromethane and washed with 100 ml of 1N hydrochloric acid, 80 ml of 0.5N hydrochloric acid, two 80 ml portions 50% saturated potassium carbonate solution and 80 ml of brine. The organic layer was dried and evaporated, giving 8.38 g of N-methoxy-N-methyl $N^{\alpha}$-t-butoxycarbonyl-L-cyclohexylalaninamide as a colorless gum $[\alpha]_D^{26}$ -10° ±1 (c = 1.0, chloroform).

To a suspension of 1.25 g of lithium aluminum hydride in 100 ml of diethyl ether under argon, chilled to 0° C was added a solution of 7.85 g of N-methoxy-N-methyl $N^{\alpha}$-t-butoxycarbonyl-L-cyclohexylalaninamide in 20 ml of diethyl ether. This mixture was stirred at 0° C for 0.5 hour, then a solution of 19 g of potassium hydrogen sulfate in 100 ml of water was added very slowly. When addition was complete, stirring was continued for 0.5 hour without cooling. The aqueous layer was separated and extracted with three 50 ml portions of diethyl ether. The organic layer and extracts were combined, washed with two 50 ml portions of 1N hydrochloric acid, 50 ml of saturated sodium bicarbonate, 50 ml of brine, dried and the solvent removed in vacuo, giving 6.5 g of the desired compound as a colorless gum; $[\alpha]_D^{26}$ +12° ±1 (c = 1.135, chloroform).

## Reference Example 41

(S)-2-Amino-3-cyclohexyl-(R)-1-(2-thiazolyl)propan-1-ol, and (S)-2-Amino-3-cyclohexyl-(S)-1-(2-thiazolyl) propan-1-ol

To a solution of 6.35 g of 1,1-dimethylethyl-(S)(2-cyclohexyl-1-formylethyl)carbamate in 15 ml of dichloromethane under argon, was added 5.89 g of 2-trimethylsilylthiazole with stirring and cooling in an ice-water bath. The solution was next stirred under argon at room temperature for 2.5 days, then cooled in an ice bath and 38 ml of 1M tetra-n-butylammonium fluoride in tetrahydrofuran was added. After 15 minutes at room temperature the volatiles were removed in vacuo. The residue was partitioned between 150 ml of ethyl acetate and 50 ml of water. The organic layer was separated , washed with two 50 ml portions of water and 50 ml of brine, dried and the solvent removed in vacuo, giving 8.19 g of light yellow gum. A 7.48 g portion of this gum was dissolved in a mixture of 20 ml of dichloromethane and 8.5 ml of trifluoroacetic acid and stirred for 2 days. The mixture was poured into 120 ml of 1N sodium hydroxide. The organic layer was separated and the aqueous layer extracted with two 75 ml portions of dichloromethane. The organic layer and extracts were combined, washed with 60 ml of brine, dried and the solvent removed in vacuo. The resulting 5.69 g of solid residue was flash chromatographed on 220 g of silica gel (230-600 mesh). Elution with 4% methanol in ethyl acetate gave, in the first major fraction, 2.63 g of (S)-2-amino-3-cyclohexyl-(R)-1-(2-thiazolyl)propan-1-ol as a white solid, $[\alpha]_D^{26}$ -18° ±1 (c = 0.743, methanol).

Elution with 6% methanol in ethyl acetate gave 0.65 g (S)-2-amino-3-cyclohexyl-(S)-1-(2-thiazolyl)-propan-1-ol as a colorless gum.

## Reference Example 42

### (S)-2-Amino-4-methyl-(R)-1-(2-thiazolyl)pentan-1-ol

2-Trimethylsilylthiazole [A. Medici et al., Chem. Com. 655(1981)] was prepared as follows: To a one liter 3-necked flask, equipped with two rubber septa, low temperature(-100°C) thermometer and a magnetic stirrer and dried with a heat gun and a stream of dry nitrogen, was added via syringe 150 ml of freshly opened diethyl ether. A 32.8 g portion of 2-bromothiazole was added followed by another 50 ml of ether. The mixture was cooled to -65°C in a dry ice-isopropanol bath and 87 ml of 2.3M n-butyllithium in hexane was very slowly added using a 50 ml syringe equipped with a long No. 18 needle, while stirring and keeping the temperature below -50°C. The resulting dark brown solution was stirred at -65°C for one hour and then treated with 25.4 ml of trimethylsilyl chloride via a syringe. The mixture was stirred for 30 minutes each at -60C°, -30°C and 0C°. The mixture was washed with 200 ml of 0.5M sodium bicarbonate, then brine, dried, treated with charcoal, filtered on a bed of hydrous magnesium silicate and concentrated to an oil. The oil was slowly and carefully fractionated on a 10 cm Vigreux column, giving 18 g of 2-trimethylsilyl thiazole as a colorless oil, bp 55-60C°.

To a solution of 10.8 g of t-butoxycarbonyl- L -leucinal in 40 ml of dichloromethane was added 9.4 g of 2-trimethylsilyl thiazole in 10 ml of dichloromethane. The reaction was allowed to stand for 48 hours, then was cooled in an ice bath and treated cautiously with 30 ml of 1M tetra-n-butylammonium fluoride in tetrahydrofuran. This mixture was refluxed for 1.5 hours and then concentrated to an oil. The oil was dissolved in 100 ml of ethyl acetate, washed with 50 ml each of 2N citric acid, 1M sodium bicarbonate and brine and dried. The solution was treated with activated carbon, filtered through a pad of hydrous magnesium silicate and concentrated to an oil. This oil was dissolved in 50 ml of 10% ethyl acetate in dichloromethane and chromatographed on two columns in the same solvent. Refractive index was monitored at S 20, 250 ml/minute. The main peak in hold-back volumes 6-10 was cut into one liter portions which were separately evaporated. The first three of these all crystallized and the solids were recrystallized form isopropyl ether and hexane, giving 0.7 g of (S)-2-(t-butoxycarbonyl)amino-4-methyl-(S)-1-(2-thiazolyl)-pentan-1-ol as crystals, mp 121-122°C.

From the mother liquors of the first three cuts and the latter cuts there was obtained 5.7 g of (S)-2-(t-butoxycarbonyl)amino-4-methyl-(R)-1-(2-thiazolyl)pentan-1-ol as an oil.

To 5.2 g of this oil was added 100 ml of anhydrous 2N hydrochloric acid in ethyl acetate with stirring. After stirring one hour the mixture was diluted with 100 ml of hexane, cooled in an ice-methanol bath at -10°C and the solid was collected, giving 4.34 g of (S)-2-amino-4-methyl-(R)-1-(2-thiazolyl)pentan-1-ol, hydrochloride as crystals, mp 134-144C°.

To a solution of 3.5 g of this hydrochloride in 12 ml of water was added dropwise 1.8 ml of 15N ammonium hydroxide. Cooling and filtration gave 1.55 g of solid. Recrystallization from isopropanol gave the desired compound as crystals, mp 81-82°C; $[\alpha]_D^{26}$ -12° ±1 (c = 1.0, methanol).

Reference Example 43

t-Butoxycarbonyl- L -phenylalanyl- L-leucyl-(1R,2S)-2-amino-3-cyclohexyl-1-(2-thiazolyl)propan-1-ol

A mixture of 0.26 g of phenyl dichlorophosphate and 0.41 g of imidazole in 5 ml of dichloromethane was stirred for 0.5 hour and then cooled to -15 °C. A 0.45 g portion of $N^{\alpha}$-t-butoxycarbonyl- L -phenylalanyl- L -leucine was added and the mixture stirred for one hour. A 0.24 g portion of (1R,2S)-2-amino-3-cyclohexyl-1-(2-thiazolyl)propan-1-ol in one ml of dichloromethane was added, the mixture stirred at -15 °C for 3 days, then diluted with 20 ml of 0.5N hydrochloric acid and extracted with 40 ml of ethyl acetate. The extract was washed with 10 ml of 0.5N hydrochloric acid, three 10 ml of 0.5N sodium hydroxide and 10 ml of brine, dried and evaporated in vacuo. The residue was flash chromatographed on silica gel, eluting with ethyl acetate:hexane (1:1), giving 0.44 g of the desired compound as a white solid, $[\alpha]_D^{26}$ -48° ±1 (c = 1.68, methanol).

Reference Example 44

t-Butoxycarbonyl- L-phenylalanyl- L -leucyl-(1R,2S)-2-amino-4-methyl-1-(2-thiazolyl)pentan-1-ol

A mixture of 0.41 g of imidazole and 0.25 g of phenyl dichlorophosphate in 5 ml of dichloromethane was stirred for 0.5 hour and then cooled to -15 °C. A 0.45 g portion of t-butoxycarbonyl- L -phenylalanyl- L-leucine was added and the mixture stirred at -15 °C for one hour. A solution of 0.20 g of (S)-2-amino-4-methyl-(R)-1-(2-thiazolyl)pentan-1-ol in one ml of dichloromethane was added and the mixture stirred at -12° to -15 °C for two days, then diluted with 20 ml of 0.5N hydrochloric acid and extracted with 40 ml of ethyl acetate. The extract was washed with 10 ml of 0.5N hydrochloric acid, three x 10 ml of 0.5N sodium hydroxide, 10 ml of brine, dried and evaporated in vacuo. The residue was chromatographed on silica gel, eluting with ethyl acetate:hexane (1:1). giving 0.46 g of the desired compound as white crystals, $[\alpha]_D^{26}$ -25° ±1 (c = 0.8, chloroform).

Reference Example 45

t-Butoxycarbonyl- L -phenylalanyl- L -histidyl-(1R,2S)-2-amino-4-methyl-1-(2-thiazolyl)pentan-1-ol

A mixture of 3.44 g of $N^{\alpha}$-t-butoxycarbonyl-N-imidazole-tosyl- L -histidine, 1.44 g of diethyl cyanophosphate and 1.3 ml of triethylamine in 50 ml of dichloromethane was stirred at 0 °C for one hour. A 1.4 g portion of (S)-2-amino-4-methyl-(R)-1-(2-thiazolyl)pentan-1-ol was added and stirring was continued at 0 °C for 20 hours then for one hour at room temperature. The mixture was diluted with dichloromethane and washed with 50% saturated sodium bicarbonate. The aqueous phase was separated and extracted with dichloromethane. The organic phase and extract were combined, washed with water and brine, dried and evaporated in vacuo to a gum. This gum was dissolved in 70 ml of methanol and 2.84 g of 1-hydroxybenzotriazole added. The mixture was stirred for 21 hours and the solvent removed in vacuo. The residue was dissolved in ethyl acetate, washed with 1N sodium hydroxide, brine, dried and the solvent removed in vacuo. The residue was chromatographed on a flash silica gel column, eluting with 5% methanol in ethyl acetate, giving 1.33 g of $N^{\alpha}$-t-butoxycarbonyl- L -histidyl-(1R,2S)-2-amino-4-methyl-1-(2-thiazolyl)pentan-1-ol; $[\alpha]_D^{26}$ -34° ±1 (c = 0.848, methanol).

A 1.1 g portion of the above compound was dissolved in a mixture of 5 ml of dichloromethane and 1.9 ml of trifluoroacetic acid and stirred for 16 hours. The solution was treated with 15 ml of 1N sodium hydroxide which was saturated with sodium chloride and extracted with 3x30 ml of dichloromethane. The

extracts were combined, dried and evaporate in vacuo. The residue was triturated with ether-hexane, giving L -histidyl-(1R,2S)-2-amino-4-methyl-1-(2-thiazolyl)pentan-1-ol as a solid which is used in the next step.

A mixture of 0.65 g of phenyl dichlorophosphate and 1.02 g of imidazole in 20 ml of dichloromethane was stirred for 0.5 hour, then cooled to 0° C and 0.8 g of $N^{\alpha}$-t-butoxycarbonyl- L -phenylalanine added. This mixture was stirred at 0° C for one hour and the L-histidyl-(1R,2S)-2-amino-4-methyl-1-(2-thiazolyl)-pentan-1-ol (prepared above) in 5 ml of tetrahydrofuran was added. This mixture was stirred at room temperature 16 hours, then diluted with 1N aqueous potassium carbonate, the volatiles removed in vacuo and the resulting suspension filtered. The precipitate was washed with water, dried in vacuo and then flash chromatographed on silica gel eluting first with 5% methanol in ethyl acetate, then 10% methanol in ethyl acetate. The second eluate gave a solid which was triturated with hexane-ether, giving 0.4 g of the desired compound as an off-white solid, $[\alpha]_D^{26}$ -30° ±2 (c = 0.53, methanol).

## Reference Example 46

t-Butoxycarbonyl- L -phenylalanyl- L -histidyl-(1R,2S)-2-amino-3-cyclohexyl-1-(2-thiazolyl)propan-1-ol

A mixture of 0.45 g of phenyl dichlorophosphate and 0.71 g of imidazole in 10 ml of dichloromethane under argon, was allowed to stand 0.5 hour, then cooled to 0° C and 0.86 g of $N^{\alpha}$-t-butoxycarbonyl-N-imidazole-tosyl- L -histidine was added. The mixture was stirred for 2 hours at 0° C, then a solution of 0.48 g of (S)-2-amino-3-cyclohexyl-(R)-1-(2-thiazolyl)propan-1-ol in 2 ml of dichloromethane was added. The mixture was stirred at 0° C for 17 hours and then at room temperature for one day. A 30 ml portion of dichloromethane was added and the solution washed with 30 ml of 50% saturated sodium bicarbonate. The aqueous layer was separated and extracted with 2x20 ml of dichloromethane. The organic layer and extracts were combined, washed with 30 ml of water and 30 ml of brine, dried and the solvent removed. The residue was washed with 4x10 ml of hexane:ether (4:1) giving 1.34 g of white solid.

To 1.2 g of the above solid in 30 ml of methanol was added 0.77 g of 1-hydroxybenzotriazole. This mixture was stirred for 20 hours, then the solvent was removed in vacuo. The residue was dissolved in 60 ml of ethyl acetate, washed with 3x10 ml of 1N sodium hydroxide and 10 ml of brine, dried and the solvent removed. The residue was flash chromatographed on silica gel, eluting with 5% methanol in ethyl acetate and gave 0.61 g of t-butoxycarbonyl- L-histidyl-(1R,2S)-2-amino-3-cyclohexyl-1-(2-thiazolyl)propan-1-ol as a white solid; $[\alpha]_D^{26}$ -55° ±2 (c = 0.46, methanol).

A 0.48 g portion of the above compound in 2 ml of dichloromethane and 0.8 ml of trifluoroacetic acid was stirred for 20 hours, then treated with 15 ml of sodium chloride saturated 1N sodium hydroxide and extracted with 3x30 ml of dichloromethane. The extracts were combined, dried and evaporated. The residue was triturated with ether-hexane, giving 0.35 g of L -histidyl-(1R,2S)-2-amino-3-cyclohexyl-1-(2-thiazolyl)-propan-1-ol as a white solid; $[\alpha]_D^{26}$ -41° ±3 (c = 0.288, methanol).

A mixture of 0.13 g of phenyl dichlorophosphate and 0.20 g of imidazole in 4 ml of dichloromethane was stirred for 0.5 hour, then cooled to 0° C and 0.16 g of $N^{\alpha}$-t-butoxycarbonyl- L -phenylalanine added. After stirring one hour at 0° C to 5° C, a solution of 0.20 g of L -histidyl-(1R,2S)-2-amino-3-cyclohexyl-1-(2-thiazolyl)propan-1-ol in one ml of tetrahydrofuran was added. The mixture was stirred for 1.5 hour at 0° to 5° C, then at room temperature for 18 hours, diluted with 15 ml of 1M potassium carbonate solution and the volatiles removed in vacuo. The resulting suspension was filtered and the solid washed with 3x10 ml of water, then dried in vacuo. This solid was triturated with 5 ml of ether and filtered, giving 0.2 g of white solid. This solid was flash chromatographed on silica gel, eluting with 5% methanol in ethyl acetate followed by 10% methanol in ethyl acetate which gave 25 mg of N-[N-(t-butoxycarbonyl- L -phenylalanyl- L -histidyl-(1R,2S)-2-amino-3-cyclohexyl-1-(2-thiazolyl)propan-1-ol as a white solid; $[\alpha]_D^{26}$ -37° ±2 (c = 0.387, methanol).

## Reference Example 47

N-[N-[(Phenylmethoxy)carbonyl]- L -phenylalanyl- L -2-(2-methylpropoxy)glycyl]-(1R,2S)-2-amino-3-cyclohexyl-1-(2-thiazolyl)propan-1-ol, and N-[N-[(Phenylmethoxy)carbonyl]- L -phenylalanyl- D -2-(2-methylpropoxy)glycyl]-(1R,2S)-2-amino-3-cyclohexyl-1-(2-thiazolyl)propan-1-ol

To a solution of 0.19 g of N,N-carbonyldiimidazole in 3 ml of dichloromethane under argon, was added 0.51 g of N-[(phenylmethoxy)carbonyl]- L -phenylalanyl- D , L -2-(2-methylpropoxy)glycine. The solution was stirred for one hour and then 0.24 g of (S)-2-amino-3-cyclohexyl-(R)-1-(2-thiazolyl)propan-1-ol was added. After stirring for one day, a solution of 0.26 g of N-[(phenylmethoxy)carbonyl]- L -phenylalanyl- D , L -2-(2-methylpropoxy)glycine activated with 0.10 g of N,N-carbonyldiimidazole was added. After stirring 21 hours this addition was repeated. The solution was stirred overnight, then diluted with 40 ml of ethyl acetate and washed with 2x20 ml of 0.5N hydrochloric acid, 2x20 ml of 50% saturated potassium carbonate solution and 20 ml of brine. The organic layer was dried and evaporated in vacuo. The residue was chromatographed on silica gel, eluting with ethyl acetate:hexane (2:3), giving 0.21 g of N-[N-[-(phenylmethoxy)carbonyl]- L -phenylalanyl- L -2-(2-methylpropoxy)glycyl]-(1R,2S)-2-amino-3-cyclohexyl-1-(2-thiazolyl)propan-1-ol as a white solid; $[\alpha]_D^{26}$ -48° ±3° (c = 0.365, methanol) and 0.14 g of N-[N-[-(phenylmethoxy)carbonyl]- L -phenylalanyl- D -2-(2-methylpropoxy)glycyl]-(1R,2S)-2-amino-3-cyclohexyl-1-(2-thiazolyl)propan-1-ol as a white solid; $[\alpha]_D^{26}$ -19° ±3 (c = 0.366, methanol).

## Reference Example 48

N-[N-(tert-butoxycarbonyl)- L -prolyl- L -phenylalanyl- L -leucyl]-(S)-2-amino-4-methyl-(R)-1-(2-thiazolyl) pentan-1-ol

A mixture of 65 mg of phenyl dichlorophosphate and 102 mg of imidazole in 2 ml of dichloromethane was stirred at room temperature under argon for 0.5 hour. $N^{\alpha}$-tert-butoxycarbonyl- L -proline (65 mg) was added and the mixture stirred for 0.5 hour, then 115 mg of N-( L -phenylalanyl- L -leucyl)-(S)-2-amino-4-methyl-(R)-1-(2-thiazolyl)pentan-1-ol was added and the mixture stirred at room temperature for 2.5 days. The mixture was quenched with 5 ml of 1N hydrochloric acid and diluted with 15 ml of dichloromethane. The organic layer was separated, washed with 5 ml of 0.5N hydrochloric acid, dried and the solvent removed. The residue was dissolved in 2 ml of methanol and treated with 0.5 ml of 1N sodium hydroxide. After 2 hours water was added and the methanol removed under vacuum.

The aqueous residue was extracted with ethyl acetate, the organic extract washed with saturated sodium bicarbonate solution dried and filtered. The filtrate was passed through a pad of hydrous magnesium silicate and the pad washed with ethyl acetate. The filtrate was evaporated under vacuum to give 0.14 g of white solid. The solid was washed with hexane and ether to give the compound as a white solid, $[\alpha]_D^{26}$ -64° (c = 0.421; methanol).

## Reference Example 49

N-( L -phenylalanyl- L -leucyl)-(S)-2-amino-4-methyl-(R)-1-(2-thiazolyl)pentan-1-ol

A solution of 0.28 g of N-(tert-butoxycarbonyl- L -phenylalanyl- L -leucyl)-(S)-2-amino-4-methyl-(R)-1-(2-thiazolyl)pentan-1-ol in one ml of dichloromethane and 0.39 ml of trifluoroacetic acid was stirred at room temperature for 20 hours. To the mixture was added 5 ml of 1N sodium hydroxide and the mixture was extracted with two 10 ml portions of dichloromethane. The extract was washed with saturated sodium chloride solution, dried and the solvent removed. The residual white solid (0.23 g) was washed with hexane to give the compound as a white solid.

## Reference Example 50

N-[N-(Phenylmethoxycarbonyl)-L-(S-benzyl)cysteinyl-L-phenylalanyl-L-leucyl]-(S)-2-amino-4-methyl-(R)-1-(2-thiazolyl)pentan-1-ol

A mixture of 39 mg of phenyl dichlorophosphate and 61 mg of imidazole in 2 ml of dichloromethane under argon was stirred at room temperature for 0.5 hour. To the mixture cooled to 0° C was added 62 mg of $N^{\alpha}$-(benzyloxycarbonyl)-L-(S-benzyl)cysteine. The mixture was stirred at 0° C for 0.5 hour and 69 mg of N-(L-phenylalanyl-L-leucyl)-(S)-2-amino-4-methyl-(R)-1-(2-thiazolyl)pentan-1-ol was added. After stirring at 0° C for 2 hours, the mixture was stirred at room temperature for 16 hours. The mixture was worked-up as described for Reference Example 48. The crude solid was washed with ether to give 95 mg of the compound as a white solid; $[\alpha]_D^{26}$ -51° ±3 (c = 0.344, methanol).

Reference Example 51

N,N-Diethyl-5-[(S)-2-tert-Butoxycarbonylamino-(R,S)-1-hydroxy-4-methylpentyl]-2-thiophenecarboxamide

A mixture of 0.285 g of 5-[(S)-2-tert-butoxycarbonylamino-(R,S)-1-hydroxy-4-methylpentyl]-2-thiophenecarboxylic acid and 0.36 g of N,N-carbonyldiimidazole in 5 ml of tetrahydrofuran was stirred at room temperature for one hour. To the solution was added 0.4 ml of dry distilled diethylamine and the mixture was stirred overnight. The solvent was removed under vacuum and the residue extracted with ethyl acetate. The extract was washed with 1N hydrochloric acid, saturated sodium bicarbonate solution, saturated sodium chloride solution and dried. The solvent was removed under vacuum and the residue purified on preparative thick layer silica gel plates with hexane-ethyl acetate (4:1) as solvent to give 0.21 g of compound as a gum.
Anal. Calcd: C, 60.3; H, 8.6; N, 7.0; S, 8.0;
Found: C,60.0; H, 8.6; N, 6.8; S, 7.7.

Reference Example 52

N,N-Diethyl 5-[(S)-2-benzyloxycarbonylamino-(R,S)-1-hydroxy-4-methylpentyl]-2-thiophenecarboxamide

A mixture of 40 mg of 5-[(S)-2-benzyloxycarbonylamino-(R,S)-1-hydroxy-4-methylpentyl]-2-thiophenecarboxylic acid and N,N-carbonyldiimidazole in one ml of tetrahydrofuran was stirred one hour and then 0.1 ml of dry distilled diethylamine was added. After stirring overnight, the solution was concentrated under vacuum. The residue was extracted with ethyl acetate and the combined extracts were washed with 1N hydrochloric acid, saturated sodium bicarbonate solution and saturated sodium chloride solution, and dried. The solvent was removed and the residue purified by thick layer chromatography on silica gel plates with hexane-ethyl acetate-acetic acid (1:1:0.001) as solvent to give 9.1 mg of compound; RF 0.51, silica gel with solvent hexane-ethyl acetate-acetic acid (1:1:0.0005).

Reference Example 53

5-[(S)-2-tert-Butoxycarbonylamino-(R,S)-1-hydroxy-4-methylpentyl]-2-thiophene-carboxylic acid

A solution of 3.9 ml of dry distilled diisopropylamine in 10 ml of dry tetrahydrofuran under argon was chilled to -70° C and to the solution was added 11.81 ml of 2.36 M n-butyllithium in tetrahydrofuran via syringe. The solution was stirred for 20 minutes and then a solution of 1.7 g of 2-thiophenecarboxylic acid in 10 ml of tetrahydrofuran was added (via syringe). After stirring for 40 minutes at -70° C, a solution of 1.5 g of N-tert-butoxycarbonylleucinal in 10 ml of tetrahydrofuran was added. The mixture was stirred at -70° C for one hour and quenched with 10% ammonium chloride solution. The mixture was allowed to warm to room temperature and the solvent removed under vacuum. The residue was extracted with ether. The combined ether extracts were washed with 1N hydrochloric acid, saturated sodium bicarbonate solution and with saturated sodium chloride solution and dried. The solvent was removed under vacuum and the residue chromatographed an a silica gel column with hexane-ethyl acetate-acetic acid (1:1:0.002) as eluent. The fractions containing product were combined and the solvent removed to give 0.28 g of gum: RF 0.36 on TLC (silica gel) with hexane-ethyl acetate-acetic acid (1:1:0.002) as solvent.

## Reference Example 54

### (S)-2-tert-Butoxycarbonylamino-4-methyl-(R,S)-1-(2-furanyl)pentan-1-ol

To 0.545 ml of furan in 10 ml of of dry tetrahydrofuran under argon cooled to -20° C was added 3.35 ml of 2.36 M n-butyllithium in hexane. The solution was allowed to warm to 10° C and stirred for 2 hours. The solution was chilled to -70° C (dry ice-acetone) and 1.70 g of N-t-butoxycarbonyl- L -leucinal in 10 ml of tetrahydrofuran was added. After one hour at -70° C, 10 ml of 10% ammonium chloride was added. The mixture was concentrated under vacuum, diluted with water and extracted with ether. The combined ether extracts were dried and concentrated. The residue was purified twice by thick layer chromatography on silica gel plates to give 0.20 g of product as a gum; RF 0.22 on thin layer chromatography (silica gel) with hexane-ethyl acetate (4:1) as solvent.

## Reference Example 55

### (S)-2-tert-Butoxycarbonylamino-4-methyl-(R,S)-1-(2-thienyl)pentan-1-ol

To a solution of 1.4 g of thiophene in 20 ml of dry tetrahydrofuran under argon was added 7.05 ml of 2.36 M n-butyllithium in tetrahydrofuran. The solution was stirred at room temperature for 45 minutes and then cooled to -70° C (dry-ice acetone bath). A solution of 1.79 g of N-tert-butoxycarbonyl- L -leucinal in 10 ml of dry tetrahydrofuran was added via syringe. The mixture was stirred (-68° C) for one hour and quenched with 10% ammonium chloride solution. After warming to room temperature, the solvent (tetrahydrofuran) was removed under vacuum. The residual aqueous mixture was extracted with ether. The combined ether extracts were washed with 50 ml of 1N hydrochloric acid, saturated sodium bicarbonate solution, saturated sodium chloride solution and dried. The solvent was removed and the residue chromatographed on a silica gel column with hexane-ethyl acetate (gradient elution) as solvent. The product was eluted with hexane-ethyl acetate (4:1). The fractions containing product were combined and the solvent removed under vacuum to give 1.15 g of gum; RF 0.34 on TLC (silica gel) with hexane-ethyl acetate (4:1).

## Reference Example 56

### 5-[(S)-2-Benzyloxycarbonylamino-(R,S)-1-hydroxy-4-methylpentyl]-2-thiophenecarboxylic acid

To a solution of 3.74 ml of dry distilled diisopropylamine in 10 ml of dry tetrahydrofuran at -70° C under argon was added 11.33 ml of 2.36 M n-butyllithium in tetrahydrofuran via a syringe. After stirring 20 minutes, a solution of 1.71 g of 2-thiophenecarboxylic acid in 10 ml of tetrahydrofuran was added via a syringe. The mixture was stirred at -70° C for 40 minutes and then 1.666 g of N-(benzyloxycarbonyl)-L-leucinal in 10 ml of tetrahydrofuran was added. The solution was stirred at -70° C for one hour and quenched with 10% ammonium chloride solution. The mixture was allowed to warm to room temperature and the tetrahydrofuran removed under vacuum. The residual aqueous mixture was extracted with ether and the combined ether extracts washed with 1N hydrochloric acid and saturated sodium chloride solution. The extract was dried and the solvent removed. The residue was chromatographed on a silica gel column with hexane:ethyl acetate (1:1) containing 0.5% acetic acid. The fractions containing product were combined to give 0.23 g of crystals, mp. 113-117° C.

Mass Spec. (FAB-low res.) 378 (M+H)

Anal. Calcd. for $C_{19}H_{23}NSO_5$: C, 60.5; H, 6.1; N, 3.7;

Found: C, 60.5; H, 6.5; N, 4.0.

## Reference Example 57

### N-[N-(tert-Butoxycarbonyl)- L-phenylalanyl- L-leucyl]-(S)-2-amino-4-methyl-(R,S)-1-(2-furanyl)pentan-1-ol

A one mmole sample of (S)-2-tert-butoxycarbonylamino-4-methyl-(R,S)-1-(2-furanyl)pentan-1-ol is stirred with 5 equivalents of trifluoracetic acid for 16 hours. The mixture is poured into 6 ml of 1N sodium hydroxide and extracted with dichloromethane. The extract is dried and the solvent removed. The (S)-2-amino-4-methyl(R,S)-1-(2-furanyl)pentan-1-ol is used without purification and coupled to tert-butoxycarbonyl-L-phenylalanyl- L-leucine as described previously.

The product is purified by column chromatographed an silica gel (ethyl acetate-hexane as solvent) to give a solid.

## Reference Example 58

### N,N-Diethyl N-[N-(tert-Butoxycarbonyl)- L phenylalanyl- L-leucyl]-5-[(S)-2-amino-(R,S)-1-hydroxy-4-methylpentyl]-2-thiophene-carboxamide

A one mmole sample of N,N-diethyl 5-[(S)-2-tert-butoxycarbonylamino-(R,S)-1-hydroxy-4-methylpentyl]-2-thiophenecarboxamide is stirred with 5 equivalents of trifluoroacetic acid for 24 hours. The mixture is poured into 6 ml of 1N sodium hydroxide and extracted with dichloromethane. The extract is dried and the solvent removed. The crude N,N-diethyl 5-[(S)-2-amino-(R,S)-1-hydroxy-4-methylpentyl]-2-thiophenecarboxamide is used without purification and is coupled with tert-butoxycarbonyl- L -phenylalanyl- L -leucine as described previously. The crude product is purified by chromatography on silica gel (ethyl acetate-hexane) to give a solid.

## Reference Example 59

### N-[N-(tert-Butoxycarbonyl)- L -phenylalanyl- L -leucyl]-(S)-2-amino-4-methyl-(R,S)-1-(2-thienyl)pentan-1-ol

A one mmole sample of (S)-2-tert-butoxycarbonylamino-4-methyl-(R,S)-1-(2-thienyl)pentan-1-ol is stirred with 5 equivalents of trifluoroacetic acid for 24 hours. The mixture is poured into 6 ml of 1N sodium

hydroxide and extracted with dichloromethane. The organic extract is dried and the solvent removed. The crude (S)-2-amino-4-methyl-(R,S)-1-(2-thienyl)pentan-1-ol is used without purification and coupled to tert-butoxycarbonyl-L-phenylalanyl-L-leucine as described previously. The product is purified by chromatography on silica gel (ethyl acetate-hexane as solvent) to give a solid.

## Reference Example 60

### (S)-2-Amino-4-methyl-(R)-1-(1-methyl-1H-1,2,4-triazol-5-yl)pentan-1-ol

To a suspension of 15.2 g of sodium hydride (60% in oil)(washed with hexane) in 100 ml of tetrahydrofuran under argon was added slowly at 0° to 10° C a solution of 25 g of 1,2,4-triazole in 100 ml of methanol. To this solution was added dropwise 25 ml of methyl iodide over 0.5 hour. The mixture was stirred at 0° C for one hour and then refluxed for 15 minutes. The solution was concentrated under reduced pressure until sodium iodide began to precipitate. The mixture was diluted with 100 ml each of ether and dichloromethane, filtered and the filtrate concentrated to dryness. The residual solid was triturated with dichloromethane (100 ml) and filtered. The filtrate was dried over molecular sieves (3A) and passed through a thin pad of hydrous magnesium silicate. The solvent was removed under vacuum and the residue sublimed to give 7.72 g of 1-methyl-1,2,4-triazole, mp 18-20° C.

To a solution of 5.3 g of 1-methyl-1,2,4-triazole in 100 ml of tetrahydrofuran under nitrogen at -75° C was added via syringe 25.6 ml of n-butyllithium. The solution was stirred 1.5 hour at -75° C and then 6.9 g of N-tert-butoxycarbonyl-L-leucinal in 25 ml of tetrahydrofuran at -75° C was added. The mixture was stirred 1/2 hour each at -75° C and at -23° C. The mixture was treated with 10 ml of saturated ammonium chloride and concentrated. The residue in 100 ml of ethyl acetate was washed with 50 ml each of water, 2N citric acid, 1M sodium bicarbonate, brine and dried. The solution was filtered and concentrated to give 8.41 g (88%) of a colorless oil which partly crystallized. Two recrystallization from iso-propyl acetate afforded 1.85 of (S)-2-(tert-butoxycarbonyl)amino-4-methyl-(R)-1-(1-methyl-1H-1,2,4-triazol-5-yl)pentan-1-ol, mp 144-145° C $[\alpha]_D^{26}$ -26° ±1 (c = 1.1, methanol).

The combined mother liquor fraction (5.47 g) was chromatographed on silica gel with ethyl acetate-dichloromethane (2:3) as solvent on a Waters Prep 500 instrument. Fractions containing the less polar diastereomer were combined and crystallized form ether/hexane to give 0.67 g (7% yield) of (S)-2-tert-butoxycarbonyl)amino-4-methyl-(S)-1-(1-methyl-1H,1,2,4-triazol-5-yl)pentan-1-ol as crystals, mp 114-116° C; $[\alpha]_D^{26}$ -38° ±1 (c = 1.2, methanol).

To a suspension of 2.34 g of (S)-2-(tert-butoxycarbonyl)amino-4-methyl-(R)-1-(1-methyl-1H,1,2,4-triazol-5-yl)pentan-1-ol in 3.5 ml of ethyl acetate was added 25 ml of anhydrous 2N hydrogen chloride in ethyl acetate. The solution was stirred one hour at room temperature and the solvent removed under vacuum. The residue was dissolved in 3 ml of water and one ml of concentrated ammonium hydroxide added. The mixture was extracted with three 12 ml portions of dichloromethane. The extract was dried and the solvent removed under vacuum to give 1.75 g of product as an oil.

## Reference Example 61

### N-[N-(Benzyloxycarbonyl)-L-phenylalanyl-L-leucyl]-(S)-2-amino-4-methyl-(R)-1-(1-methyl-1H-1,2,4-triazol-5-yl)pentan-1-ol

To a suspension of 1.13 g of N-benzyloxycarbonyl-L-phenylalanyl-L-leucine in 3 ml of dichloromethane was added 0.39 ml of triethylamine followed by 1.21 g of benzotriazolyloxytris(dimethylamino)phosphonium hexafluorophosphate . To this solution was added 0.52 g of (S)-2-amino-4-methyl-(R)-1-(1-methyl-1H-1,2,4-triazol-5-yl)pentan-1-ol. After stirring for 3 hours the solvent was removed under vacuum. The residue was dissolved in ethyl acetate, and washed with 1N hydrochloric acid, 1M sodium bicarbonate and with brine. Concentration of the ethyl acetate solution under vacuum gave 1.36 g of

an oil which was chromatographed on five 20x20x0.2cm preparative thin layer silica gel plates developed in ethyl acetate, and eluted with methanol. Concentration and filtering through hydrous magnesium silicate gave 0.26 g of a glass; $[\alpha]_D^{26}$ -7° ±1 (c = 1.0, methanol).

## Reference Example 62

N-[N-(Benzyloxycarbonyl)- L -phenylalanyl- L -2-(2-methylpropoxy)glycyl]-(S)-2-amino-4-methyl-(R)-1-(1-methyl-1H-1,2,4-triazol-5-yl)pentan-1-ol and N-[N-(Benzyloxycarbonyl)- L -phenylalanyl- D -2-(2-methylpropoxy)glycyl]-(S)-2-amino-4-methyl-(R)-1-(1-methyl-1H-1,2,4-triazol-5-yl)pentan-1-ol

To a stirred suspension of 2.34 g of N-(benzyloxycarbonyl)- L -phenylalanyl-( D , L )-2-(2-methylpropoxy)glycine in 6 ml of dichloromethane was added 0.78 ml of triethylamine and 2.42 g of benzotriazol-1-yloxytris(dimethylamino)phosphonium hexafluorophosphate. After 3 minutes, 1.04 g of (S)-2-amino 4-methyl-(R)-1-(1-methyl-1H-1,2,4-triazol-5-yl)-pentan-1-ol in 6 ml of dichloro methane was added. After stirring overnight, the solution was refluxed 30 minutes and concentrated. The residue, dissolved in 50 ml of ethyl acetate, was washed with 25 ml each of 1N hydrochloric acid, 1M sodium bicarbonate and brine. The organic layer was dried and concentrated to give 4 g of an oily mixture of diastereomers.

Preparative high pressure liquid chromatography on silica gel with 4:1 hexane-ethyl acetate gave a less polar fraction, 0.66 g, m.p 70-74° C, $[\alpha]_D^{26}$ -34° ±1 (c = 1.2, methanol) identified as N-[N-(benzyloxycarbonyl)- L -phenylalanyl- L -2-(2-methylpropoxy)glycyl]-(S)-2-amino-4-methyl-(R)-1-(1-methyl-1H-1,2,4-triazol-5-yl)-pentan-1-ol.

The more polar isomer, 0.7 g, mp 135-139° C, $[\alpha]_D^{26}$ -4° ±1 (c = 1, methanol) was identified as N-[N-(benzyloxycarbonyl)- L -phenylalanyl- D -2-(2-methylpropoxy)glycyl]-(S)-2-amino-4-methyl-(R)-1-(1-methyl-1H-1,2,4-triazol-5-yl)-pentan-1-ol.

## Reference Example 63

N-[N-(Benzyloxycarbonyl)- L -phenylalanyl- L -2-(2-methylpropoxy)glycyl]-(S)-2-amino-3-phenyl-(R)-1-(2-thiazolyl)propan-1-ol and N-[N-(Benzyloxycarbonyl)- L -phenylalanyl- D -2-(2-methylpropoxy) glycyl]-(S)-2-amino-3-phenyl-(R)-1-(2-thiazolyl)propan-1-ol

A solution of 0.51 g of N-benzyloxycarbonyl)- L -phenylalanyl-( D , L )-2-(2-methylpropoxy)glycine in 2 ml of dichloromethane was added to a prepared solution of 0.26 g of phenyl dichlorophosphate and 0.40 g of imidazole in 4 ml of dichloromethane (as described in Reference Example 39). To this solution was added 0.24 g of (S)-2-amino-3-phenyl-(R)-1-(2-thiazolyl)propan-1-ol and the mixture stirred for 24 hours at room temperature. To the mixture was added 20 ml of 0.5N hydrochloric acid and 30 ml of ethyl acetate. The organic layer was separated and washed with 15 ml of 0.5N hydrochloric acid, 30 ml of saturated potassium carbonate and 15 ml of brine. The organic layer was dried and the solvent removed to give 1.04 g of solid. This solid was chromatographed by flash chromatography on silica gel (230-400 mesh) with ethyl acetate-hexane (1:1) as solvent to give 0.28 g of the L -, L - isomer (first fraction): $[\alpha]_D^{26}$ -23° ±1 (c = 1.04, chloroform) and 0.10 g of the L , D isomer (second fraction): $[\alpha]_D^{26}$ -43° ±2 (c = 0.57, chloroform).

## Reference Example 64

N-[N-(Benzyloxycarbonyl)- L -phenylalanyl- L -2-(2-methylpropoxy)glycyl]-(S)-2-amino-3-phenyl-(R)-1-(2-thiazolyl)propan-1-ol

To a stirred suspension of 0.18 g of N-[(phenylmethoxy)carbonyl]- L -phenylalanyl-N-[(S)-1-formyl-2-phenylethyl ]- L -2-(2-methylpropoxy)glycinamide (Reference Example 20) in 0.5 ml of dichloromethane was added 51 mg of 2-trimethylsilylthiazole in 0.5 ml of dichloromethane. After 3 hours the milky suspension was diluted with 2 ml of dichloromethane and stirred overnight. The mixture was refluxed 15 minutes and then cooled in an ice bath. Tetra-n-butylammonium fluoride (0.5 ml of 2.5 M in tetrahydrofuran) was added and the solution refluxed 1.5 hours. The solution was concentrated and the residue in 5 ml of ethyl acetate was washed with 5 ml each of 2N citric acid, 1M sodium bicarbonate and brine. The solution was dried, filtered through a thin layer of hydrous magnesium silicate and concentrated to give 0.13 g of a gum. This gum was chromatographed on three 20x20x0.2 cm preparative silica gel plates with hexane-ethyl acetate (1:1) for development and ethyl acetate for elution. Evaporation of the elution solvent (ethyl acetate) gave 25 mg of the product as a gum which was identical to the L -, L isomer of Reference Example 63 (pmr spectrum). Comparison by thin layer chromato graphy on silica gel with ethyl acetate-hexane (1:1) showed identical RF's.

## Reference Example 65

### N[N-(Benzyloxycarbonyl)- L -phenylalanyl- L -2-(cyclohexyloxy)glycyl]-(S)-2-amino-4-methyl-(R)-1-(2-thiazolyl)pentan-1-ol and N-[N-(Benzyloxycarbonyl)- L -phenylalanyl- D -2-(cyclohexyloxy)glycyl]-(S)-2-amino-4-methyl-(R)-1-(2-thiazolyl)pentan-1-ol

To a suspension of 2.3 g of N-[N-(benzyloxycarbonyl-L -phenylalanyl]-( D , L )-2-(cyclohexyloxy)-glycine in 6 ml of dichloromethane was added 0.71 ml of triethylamine. To this solution was added 2.25 g of benzotriazol-1-yloxytris(dimethylamino)phosphonium hexafluorophosphate followed 2 minutes later by the addition of one g of (S)-2-amino-4-methyl-(R)-1-(2-thiazolyl)pentan-1-ol.

After stirring overnight, the solution was refluxed one hour and concentrated under vacuum. The residue was dissolved in 50 ml of ethyl acetate and washed successively with 1N hydrochloric acid, 1M sodium bicarbonate and brine. The colorless solution was dried, filtered through a thin pad of hydrous magnesium silicate and concentrated to an oil (4.55 g). The preceding oil in 30 ml of hexane-ethyl acetate (1:1) was filtered and chromatographed, with solvent ethyl acetate-hexane (1:1), on two prep 500 silica gel columns in series at high pressure. Hold-back volumes 4-7 (fraction A) and 9-12 (fraction B) contained the products. The solutions were concentrated separately to give from fraction A 1.1 g of N-[N-(benzyloxycarbonyl)-L -phenylalanyl- L -2-(cyclohexyloxy)glycyl]-(S)-2-amino-4-methyl-(R)-1-(2-thiazolyl)pentan-1-ol as a glass; $[\alpha]_D^{26}$ -54° ±1 (c = 1.3, methanol); Rf 0.39 on silica gel plates with solvent ethyl acetate-hexane (1:1).

Concentration of fraction B gave 1.11 g of N-[N-(benzyloxycarbonyl)- L -phenylalanyl- D -2-(cyclohexyloxy)glycyl]-(S)-2-amino-4-methyl-(R)-1-(2-thiazolyl)pentan-1-ol as a glass; $[\alpha]_D^{26}$ -3° ±1 (c = 1.4, methanol); Rf 0.19 on silica gel plates with solvent ethyl acetate-hexane (1:1).

## Reference Example 66

### N-[N-(Benzyloxycarbonyl)- L -phenylalanyl- L -2-(1-methylethoxy)glycyl]-(S)-2-amino-4-methyl-(R)-1-(2-thiazolyl)pentan-1-ol and N-[N-(Benzyloxycarbonyl)- L -phenylalanyl- D -2-(1-methylethoxy)glycyl]-(S)-2-amino-4-methyl-(R)-1-(2-thiazolyl)pentan-1-ol

To a suspension of 1.7 g of N-[N-(benzyloxycarbonyl)- L -phenylalanyl-( D , L )-2-(1-methylethoxy)-glycine in 4 ml of dichloromethane was added 0.56 ml of triethylamine. To the resulting solution was added 1.8 g of benzotriazol-1-yloxytris(dimethylamino)phosphonium hexafluorophosphate followed 2 minutes later by the addition of 0.8 g of (S)-2-amino-4-methyl-(R)-1-(2-thiazolyl)pentan-1-ol in one ml of dichloromethane. The reaction was stirred overnight, refluxed 30 minutes, and diluted with 50 ml of ethyl acetate. After washing with 10 ml each of 1N hydrochloric acid, 1M sodium bicarbonate and brine, this solution was dried and filtered with charcoal through a thin pad of hydrous magnesium silicate. The filtrate was concentrated

under vacuum to give 2.5 g of a gum. High pressure liquid chromatography (two prep-500 silica gel columns) in hexane-ethyl acetate (1:1) gave two well-separated (refraction index detection) peaks. Concentration of hold-back volumes 6-9 (fraction A) gave 0.76 g of N-[N-(benzyloxycarbonyl)- L -phenylalanyl- L -2-(1-methylethoxy)glycyl]-(S)-2-amino-4-methyl-(R)-1-(2-thiazolyl)pentan-1-ol as a glassy foam; $[\alpha]_D^{26}$ -56° ±1 (c = 1.5, methanol) Rf 0.32 on silica gel plates with ethyl acetate-hexane (1:1) as solvent.

Concentration of hold-back volume 12-15 (fraction B) gave a solid which was crystallized from isopropyl acetate to give 0.83 g of N-[N-(benzyloxycarbonyl)- L-phenylalanyl- D -2-(1-methylethoxy)glycyl]-(S)-2-amino-4-methyl-(R)-1-(2-thiazolyl)pentan-1-ol as crystals, mp 163-164° C; $[\alpha]_D^{26}$ -8° ±2 (c = 0.5 methanol); Rf 0.20 on silica gel plates with ethyl acetate-hexane (1:1) as solvent.

## Reference Example 67

### N-[N-[(Phenylmethoxy)carbonyl]- L -phenylalanyl- L -valyl]-(S)-2-amino-4-methyl)-(R)-1-(2-thiazolyl) pentan-1-ol

To a mixture of 0.42 g of N-[(phenylmethoxy)carbonyl]- L -phenylalanyl- L -valine and 0.15 ml of triethylamine in 2 ml of dichloromethane was added 0.47 g of benzotriazol-1-yloxytris(dimethylamino)-phosphonium hexafluorophosphate. After 2 minutes, 0.20 g of (S)-2-amino-4-methyl-(R)-1-(2-thiazolyl)-pentan-1-ol was added. This mixture was stirred overnight, then concentrated in vacuo to dryness. The residue was dissolved in 40 ml of ethyl acetate and washed twice with 5 ml each of 1N hydrochloric acid, 1M sodium bicarbonate and saturated sodium chloride solution. The organic layer was dried and the solvent removed in vacuo. The residue was dissolved in hot isopropyl acetate and filtered through a thin pad of hydrous magnesium silicate. The pad was washed with isopropyl acetate. The filtrate and wash were combined, chilled and the solid collected, giving 0.37 g of the desired compound as crystals, mp 181-182° C; $[\alpha]_D^{26}$ -42° ±1 (c = 1.082, methanol).

## Reference Example 68

### N-[N[(Phenylmethoxy)carbonyl]- L -tryptophyl- L -leucyl]-(S)-2-amino-4-methyl-(R)-1-(2-thiazolyl)pentan-1-ol

To a mixture of 0.47 g of N-[(phenylmethoxy)carbonyl]- L -tryptophyl- L -leucine and 15 ml of dry tri ethylamine in 2 ml of dichloromethane was added 0.47 g of benzotriazol-1-yloxytris(dimethylamino)-phosphonium hexafluorophosphate. After stirring 2 minutes 0.2 g of (S)-2-amino-4-methyl-(R)-1-(2-thiazolyl)-pentan-1-ol and 0.5 ml of dichloromethane were added. This mixture was stirred overnight, then refluxed for 0.5 hour and concentrated to dryness in vacuo. The residue was dissolved in 20 ml of ethyl acetate and washed with 5 ml each of 1N hydrochloric acid, 1M sodium bicarbonate and saturated sodium chloride solution. The organic layer was dried and passed through a pad of hydrous magnesium silicate. The pad was washed with ethyl acetate and the combined filtrate and wash concentrated in vacuo. The residue was crystallized from isopropyl ether-isopropyl acetate, giving 0.2 g of the desired compound as crystals, mp 162-165° C; $[\alpha]_D^{26}$ -38° ±1 (c = 1.024 methanol).

## Reference Example 69

### N-[N-[(Phenylmethoxy)carbonyl]- L -phenylalanyl- L -methionyl]-(S)-2-amino-4-methyl-(R)-1-(2-thiazolyl) pentan-1-ol

To a mixture of 0.45 g of N-[phenylmethoxy)carbonyl]- L -phenylalanyl- L -methionine and 0.15 ml of dry triethylamine in 2 ml of dichloromethane was added 0.47 g of benzotriazol-1-yloxytris(dimethylamino)-phosphonium hexafluorophosphate. After stirring for one minute, 0.2 g of (S)-2-amino-4-methyl-(R)-1-2-thiazolyl)pentan-1-ol was added. This mixture was stirred overnight, then refluxed for 15 minutes and concentrated in vacuo. The residue was dissolved in 20 ml of ethyl acetate and washed with 5 ml each of 1N hydrochloric acid, 1M sodium bicarbonate and saturated sodium chloride. The organic layer was dried and passed through a thin pad of hydrous magnesium silicate. The pad was washed with ethyl acetate and the combined filtrate and wash concentrated in vacuo to an oil. This oil was crystallized from isopropyl ether-isopropyl acetate, giving 0.35 g of the desired compound as crystals mp 145-150° C; $[\alpha]_D^{26}$ -31° ±1 (c = 1.024, methanol).

## Reference Example 70

### N-[N-(tert-butoxycarbonyl)glycyl- L -phenylalanyl- L -methionyl]-(S)-2-amino-4-methyl-(R)-(2-thiazolyl) pentan-1-ol

To a mixture of 0.94 g of N-[N-(tert-butoxycarbonyl)-glycyl- L -phenylalanyl]- L -methionine and 0.29 ml of dry triethylamine in 4 ml of dichloromethane was added 0.92 g of benzotriazol-1-yloxytris-(dimethylamino)-phosphonium hexafluorophosphate and 2 ml of dichloromethane. After stirring for 2 minutes, 0.6 g of (S)-2-amino-4-methyl-(R)-1-(2-thiazolyl)pentan-1-ol in 2 ml of dichloromethane was added. This mixture was stirred overnight, then concentrated in vacuo to dryness. To the residue was added 20 ml of ethyl acetate. This solution was washed with 5 ml each of 1N hydrochloric acid, 1M sodium bicarbonate and saturated sodium chloride solution. The organic layer was dried and evaporated. The residue was dissolved in dichloromethane, filtered through hydrous magnesium silicate and washed with dichloromethane. The filtrate and wash were combined, concentrated in vacuo and the residue crystallized from 10 ml of isopropyl ether and 10 ml of isopropyl acetate, giving 1.09 g of the desired compound as crystals, mp 170-175° C; $[\alpha]_D^{26}$ -18° ±1 (c = 0.878, methanol).

## Reference Example 71

### (S)-2-Amino-3-cyclohexyl-(R)-1-(2-thiazolyl)-1-[(t-butyl)(dimethyl)silyloxy]propane, and (S)-2-(t-Butoxycarbonyl)amino-3-cyclohexyl-(R)-1-(2-thiazolyl)-1-[(t-butyl)(dimethyl)silyloxy]propane

A) To a stirred solution of 14.3 g of t-butyldimethylsilyl chloride in 30 ml of dry N,N-dimethylformamide, under argon, was added 18.8 g of imidazole in small portions. This mixture was stirred for 15 minutes, then a solution of 15.8 g of (S)-2-(t-butoxycarbonyl)amino-3-cyclohexyl-(R,S)-1-(2-thiazolyl)propan-1-ol (prepared as described in Reference Example 41) in 15 ml of N,N-dimethylformamide was added. This mixture was stirred for 2 days, then diluted with 600 ml of hexane and washed twice with 300 ml of water, twice with 200 ml of saturated sodium bicarbonate solution and 200 ml of saturated sodium chloride solution. The organic layer was dried and evaporated in vacuo. The residue was flash chromatographed on silica gel with ethyl acetate:hexane (1:10) as solvent, giving 10.28 g of (S)-2-(t-butoxycarbonyl)amino-3-cyclohexyl-(R)-1-(2-thiazolyl)-1-[(t-butyl)(dimethyl)silyloxy]propane as a colorless gum; $[\alpha]_D^{26}$ -12° ±1 (c = 1.122, methanol), and 2.24 g of (S)-2-(t-butoxycarbonyl)amino-3-cyclohexyl-(S)-1-(2-thiazolyl)-1-[(t-butyl)(dimethyl)silyloxy]propane as a sticky white solid; $[\alpha]_D^{26}$ -43° ±1 (c = 1.047, methanol).

B) A solution of 9.80 g of (S)-2-t-butoxycarbonyl)amino-3-cyclohexyl-(R)-1-(2-thiazolyl)-1 [(t-butyl)(dimethyl)silyloxy]propane in 50 ml of dichloromethane was cooled to 0° C and 16.6 ml of trifluoroacetic acid was added with stirring. The mixture was then stirred at room temperature for 4 hours, poured into 120 ml of ice-cold 2N sodium hydroxide with stirring and 100 ml of dichloromethane added. The aqueous layer was separated and extracted with 100 ml of dichloromethane. The organic layer and extract were combined, dried and the solvent removed in vacuo, giving 7.58 g of (S)-2-amino-3-cyclohexyl-(R)-1-(2-thiazolyl)-1-[(t-butyl)(dimethyl)silyloxy]propane as a white solid, mp 63-65° C: $[\alpha]_D^{26}$ +20° ±1 (c = 1.11, methanol).

## Reference Example 72

### N-(L-Phenylalanyl-L-leucyl)-(S)-2-amino-3-cyclohexyl-(R)-1-(2-thiazolyl)-1-[(t-butyl)(dimethyl)silyloxy]propane

To a solution of 10.2 g of imidazole in 120 ml of dichloromethane was added 6.46 g of phenyl dichlorophosphate in 10 ml of dichloromethane. The suspension was stirred for 0.5 hour, then cooled to -15° C and 11.34 g of t-butoxycarbonyl-L-phenylalanyl-L-leucine added in portions. The mixture was stirred at -15° C for one hour, then a solution of 7.08 g of (S)-2-amino-3-cyclohexyl-(R)-1-(2-thiazolyl)-1-[(t-butyl)(dimethyl)silyloxy]propane in 20 ml of dichloromethane was added slowly. This mixture was stirred at -15° C for 28 hours, then quenched with 200 ml of water. The organic layer was separated and the solvent removed in vacuo. The residue was dissolved in 250 ml of ethyl acetate and washed successively with 100 ml of 1N hydrochloric acid, 100 ml of 0.5N hydrochloric acid, 50 ml of saturated sodium chloride, two 100 ml portions of 0.5N sodium hydroxide and finally 50 ml of saturated sodium chloride solution. The organic layer was dried and the solvent removed in vacuo, giving 14.72 g of N-[N-(t-butoxycarbonyl)-L-phenylalanyl-L-leucyl]-(S)-2-amino-3-cyclohexyl-(R)-1-(2-thiazolyl)-1-[(t-butyl)(dimethyl)silyloxy]propane as a white foamy solid.

A 3.57 g portion of the above solid was dissolved in 10 ml of dichloromethane, the solution cooled to 0° C and 3.9 ml of trifluoroacetic added. The cooling bath was removed, the mixture stirred at room temperature for 4 hours, then poured into ice-cold 2N sodium hydroxide with stirring and diluted with dichloromethane. The organic layer was separated, dried and the solvent removed, giving 3.04 g of the desired compound as a foamy white solid.

## Reference Example 73

### (S)-2-Amino-4-methyl-(R)-1-(2-thienyl)pentan-1-ol

A 0.81 g portion of (S)-2-(tert-butoxycarbonyl)amino-4-methyl-(R,S)-1-(2-thienyl)pentan-1-ol (Reference Example 55) was dissolved in 5 ml of dichloromethane and 2.1 ml of trifluoroacetic acid added. This mixture was stirred for 3 hours, then poured with stirring into 15 ml of ice-cold 2N sodium hydroxide. The mixture was diluted with 25 ml of dichloromethane, the organic layer separated and the aqueous layer extracted with 20 ml of dichloromethane. The organic layer and extract were combined, washed with saturated sodium chloride solution, dried and the solvent removed in vacuo. The residue was chromatographed on a silica gel column with ethyl acetate:hexane (1:4), giving 0.72 g of (4S-trans)-4-(2-methylpropyl)-5-(2-thienyl)-2-oxazolidinone as a white solid $[\alpha]_D^{26}$ -141° ±2 (c = 0.570 methanol).

A 0.23 g portion of the above solid was dissolved in 5 ml of ethanol and 5 ml of 1N sodium hydroxide added. The solution was refluxed for 16 hours and then concentrated in vacuo. The residue was extracted with two 10 ml portions of dichloromethane. The extracts were combined, dried and the solvent removed in vacuo, giving 0.2 g of the desired compound; Rf 0.045 [silica gel; ethyl acetate:hexane (1:2)].

## Reference Example 74

### (S)-2-Amino-3-cyclohexyl-(R)-1-(2-thienyl)propan-1-ol

To a solution of 1.57 g of N-methoxy-N-methyl $N^{\alpha}$-t-butoxycarbonyl- L -cyclohexylalaninamide (prepared as described in Reference Example 40) in 10 ml of diethyl ether, cooled to -78° C, was added under argon 2.1 ml of 2.35M n-butyllithium in hexane. After stirring for one hour, the mixture was allowed to warm to 0° C. To this was added a solution of 2-lithiothiophene in ether (prepared from 0.64 g of thiophene in 5 ml of ether and 3.2 ml of 3.25M n-butyllithium in hexane at 0° C for one hour). This mixture was stirred at 0° C for 2 hours, then quenched with 15 ml of 1N hydrochloric acid and diluted with 25 ml of ether. The organic layer was separated, washed successively with 15 ml of 1N hydrochloric acid, 10 ml of water and 15 ml of saturated sodium bicarbonate, dried and filtered through a short pad of hydrous magnesium silicate. The filter pad was washed with ether, the filtrate and wash combined and evaporated in vacuo. The residue was washed with hexane and then chromatographed on 50 g of silica gel with ethyl acetate:hexane (1:20) as solvent giving 1.2 g of solid. Crystallization from hexane containing a trace of ether gave (S)-1,1-dimethylethyl-[1-(cyclohexylmethyl)-2-oxo-2-thienylethyl]carbamate as crystals; $[\alpha]_D^{26}$ +24° ±1 (c = 1.10, methanol).

A solution of 0.51 g of the above compound in 8 ml of dry tetrahydrofuran was cooled to -78° C under argon and 3 ml of 1.0M potassium tri-sec-butylborohydride in tetrahydrofuran was added dropwise. This mixture was stirred at -78° C for 4 hours, then quenched with 5 ml of saturated aqueous ammonium chloride, warmed to room temperature and the organic solvent removed in vacuo. The aqueous residue was diluted with 5 ml of water and 20 ml of ethyl acetate. The organic layer was separated and washed successively with two 5 ml portions of saturated aqueous ammonium chloride, 5 ml of saturated aqueous sodium bicarbonate and 5 ml of saturated aqueous sodium chloride solution, dried and the solvent removed in vacuo to give (S)-2-(tert-butoxycarbonylamino)-3-cyclohexyl-(R,S)-1-(2-thienyl)propan-1-ol as a gum.

To an 18.4 g sample of the preceding gum in 330 ml of dichloromethane cooled to 0° C was added 16.75 ml of trifluoroacetic acid. The solution was stirred overnight, cooled to 0° C and ice cold 1N sodium hydroxide (approximately 300 ml) was added. The organic layer was separated and the aqueous layer extracted with two 350 ml portions of dichloromethane. The organic layer and extracts were combined, washed with two 250 ml portions of brine, dried ($Na_2SO_4$) and the solvent removed in vacuum to give 14.5 g of solid.

Trituration with 200 ml of hot hexane, cooling to room temperature and filtering gave 7.5 g of crystals of (4S-trans) 4-(cyclohexylmethyl)-5-(2-thienyl)-2-oxazolidinone as crystals, mp 105-108° C.

A mixture of 7.0 g of the preceding compound in 13 ml of ethanol and 132 ml of 1N sodium hydroxide was refluxed for 17 hours. The solvent was removed under vacuum and the residue extracted twice with 200 ml of dichloromethane. The combined extracts were dried ($Na_2SO_4$) and the solvent removed under vacuum to give 4.64 g of crystals, mp 62-64° C; $[\alpha]_D^{26}$ -35° ±1 (c = 1.145, $CH_3OH$).

## Reference Example 75

### N-[N-(tert-Butoxycarbonyl)-N-methyl- L -leucyl]-(S)-2-amino-4-methyl-1-(2-thiazolyl)pentan-1-ol

To a mixture of 0.26 g of N-(tert-butoxycarbonyl)-N-methyl- L -leucine in 2 ml of dichloromethane was added 0.15 ml of triethylamine and 0.47 g of benzotriazol-1-yloxytris(dimethylamino)phosphonium hexafluorophosphate (BOP-reagent). After one minute, 0.20 g of (S)-2-amino-4-methyl-(R)-1-(2-thiazolyl)pentan-1-ol was added and the mixture was stirred at room temperature overnight. The mixture was refluxed for 15 minutes, diluted with 20 ml of ethyl acetate and washed (twice) with 5 ml each of 1N hydrochloric acid, and IM sodium bicarbonate. The organic layer was filtered through a thin pad of hydrous magnesium silicate and the pad washed with ethyl acetate. The filtrate concentrated to give 0.41 g of gum; $[\alpha]_D^{26}$ -68° ±3 (c = 0.315, methanol).

## Reference Example 76

### N-[N-Methyl- L -leucyl]-(S)-2-amino-4-methyl-(R)-1-(2-thiazolyl)pentan-1-ol

To 0.41 g of N-[N-(tert-butoxycarbonyl)-N-methyl- L -leucyl]-(S)-2-amino-4-methyl-(R)-1-(2-thiazolyl)-pentan-1-ol was added 4 ml of anhydrous 2N hydrochloric acid in ethyl acetate. The mixture was stirred for 2 hours and the solvent removed under vacuum. The residue was dissolved in water and concentrated ammonium hydroxide added. The solid formed, was filtered and then washed with cold water to give 0.095 g of crystals, mp 66-67° C; Rf 0.05 on silica gel plate with ethyl acetate-methanol (9:1) as solvent. The sample was distilled (onto cold finger) under vacuum to give crystals, mp 86-87° C; $[\alpha]_D^{26}$ -41° ±1 (c = 0.943, methanol).

## Reference Example 77

### N-( L -leucyl)-(S)-2-amino-4-methyl-(R)-1-(2-thiazolyl)pentan-1-ol

To a solution of 1.76 g of imidazole in 15 ml of dichloromethane under nitrogen was added 1.1 g of phenyl dichlorophosphate (phenyl phosphorodichloridate) in 5 ml of dichloromethane and the solution cooled to 0° C. A solution of 1.25 g of $N^{\alpha}$-tert-butoxycarbonyl- L -leucine hydrate in 6 ml of dichloromethane was dried over magnesium sulfate and filtered. The filtrate was added to the first solution and the mixture stirred one hour at 0° C. To this mixture was added 1.05 g of (S)-2-amino-4-methyl-(R)-1-(2-thiazolyl)pentan-1-ol. After stirring at room temperature overnight the mixture was refluxed one hour and filtered. The filtrate was washed with 10 ml each of 2N hydrochloric acid, 1M sodium bicarbonate and dried over magnesium sulfate. The filtrate was passed through a thin pad of hydrous magnesium silicate and the pad washed with dichloromethane. The filtrate was concentrated under vacuum to give 1.71 g of N-[N-(tert-butoxycarbonyl)- L -leucyl]-(S)-2-amino-4- methyl-(R)-1-(2-thiazolyl)pentan-1-ol as an oil; Rf 0.43 on silica gel plate with ethyl acetate-hexane (1:1) as solvent.

To the preceding oil (1.71 g) in one ml of ethyl acetate was added 16 ml of anhydrous 2N hydrochloric acid in ethyl acetate. After stirring 3 hours at room temperature, one ml of hexane was added and the mixture cooled 0° C and filtered to give 1.4 g of the product as the hydrochloride, mp 136-140° C. The hydrochloride was dissolved in water (4 ml) and ammonium hydroxide (2 ml) added. Cooling gave crystals which were filtered and washed with cold water to give 0.97 g of product as crystals, mp 112-116° C. Recrystallization from diisopropyl ether gave 0.67 g of crystals, mp 112-114° C; $[\alpha]_D^{26}$ -34° ±1 (c = 0.976, methanol).

## Reference Example 78

### ± N-(tert-Butoxycarbonyl)-3-(2-thienyl)alanine

To a stirred mixture of 1.7 g of 3-(2-thienyl)- D , L -alanine in 20 ml of dioxane was added 10 ml of water. The mixture was cooled (ice-water bath) and 2.4 g of 2-tert-butoxycarbonyloxyimino)-2-phenylacetonitrile (BOC-ON) was added. After stirring for 0.5 hours, the mixture was concentrated to 15 ml and acidified to pH 2 to 3 with 0.35M potassium hydrogen sulfate. The crystals which separated were filtered, washed with cold water and air dried to give 2.2 g of crystals, mp 113-114° C.

## Reference Example 79

### N-( L -leucyl)-(S)-2-amino-3-cyclohexyl(R)-1-(2-pyridinyl)propan-1-ol

A solution of 0.75 g of $N^{\alpha}$-tert-butoxycarbonyl- L -leucine monohydrate in dichloromethane was stirred

with anhydrous magnesium sulfate for 2 hours, filtered and the filtrate concentrated to a volume of 15 ml. This solution was chilled to 0 °C and 0.488 g of N,N-carbonyldiimidazole added. The solution was stirred at 0 °C for one hour and 0.469 g of (S)-2-amino-3-cyclohexyl-(R)-1-(2-pyridinyl)propan-1-ol added. The mixture was stirred at room temperature overnight, washed twice with saturated sodium bicarbonate, water, brine and dried ($MgSO_4$). The solvent was remcved under reduced pressure to give 1.0 g of a white foam. This foam was dissolved in chloroform-methanol (99:1) and the combined filtrates concentrated under vacuum to give 0.90 g of N-[N-(tert-butoxycarbonyl)- L -leucyl]-(S)-2-amino-3-cyclohexyl-(R)-1-(2-pyridinyl)propan-1-ol as a clear foam.

To a 0.30 g sample of the preceding compound in 5 ml of dichloromethane was added 0.516 ml of trifluoroacetic acid and the mixture stirred overnight. The solution was poured into ice-cold sodium hydroxide (1.61 ml of 5N sodium hydroxide in 5 ml of water), stirred and the organic layer separated. The aqueous layer was extracted twice with dichloromethane and the organic layer and extracts combined and dried($MgSO_4$). The solvent was removed under vacuum. The residue was crystallized from diisopropylether and recrystallized from diisopropylether to give 0.102 g of white crystals, mp 98-100 °C.

## Reference Example 80

### N-( L -Leucyl)-(S)-2-amino-3-cyclohexyl-(R)-1-(2-thiazolyl)propan-1-ol

A sample of 0.26 g of $N^{\alpha}$-tert-butoxycarbonyl- L -leucine monohydrate was dried with magnesium sulfate in 2 ml of dichloromethane. The mixture was filtered and 0.15 ml of dry triethylamine and 0.47 g of benzotriazol-1-yloxytris(dimethylamino)phosphonium hexafluorophosphate (BOP) added. After one minute, 0.24 g of (S)-2-amino-3-cyclohexyl-(R)-1-(2-thiazolyl)propan-1-ol was added and the mixture stirred overnight and refluxed 2 hours. The mixture was concentrated and the residue in ethyl acetate was washed with 1N hydrochloric acid, sodium carbonate solution and brine. The organic layer was dried ($MgSO_4$) and concentrated to give 0.44 g of crudeN-[N-(tert-butoxycarbonyl)- L -leucyl]-(S)-2-amino-3-cyclohexyl-(R)-1-(2-thiazolyl)propan-1-ol as a glass.

The preceding compound was added to 5 ml of 2N anhydrous hydrochloric acid in ethyl acetate and the mixture stirred for 2 hours. The mixture (containing solid) was diluted with 5 ml of hexane, filtered and the solid washed with hexane. The solid was dissolved in 1 ml of water and 0.7 ml of concentrated ammonium hydroxide added. The solid which separated was filtered, and dried to give 0.27 g of solid, mp 140-141 °C.

## Reference Example 81

### N-( L -Histidyl)-(S)-2-amino-4-methyl-(R)-1-(2-thiazolyl)pentan-1-ol

To a suspension of 2.4 g of $N^{\alpha}$-tert-butoxycarbonyl- L -histidine in 24 ml of dichloromethane was added 1.3 ml of triethylamine and then 4.2 g of benzotriazol-1-yloxytris(dimethylamino)phosphonium hexafluorophosphate (BOP). After stirring at room temperature for 2 minutes, 1.73 g of (S)-2-amino-4-methyl-(R)-1-(2-thiazolyl)pentan-1-ol was added. The mixture was stirred for 4 days and refluxed for one hour and the solvent removed under vacuum to give an oil. This oil in 20 ml of ethyl acetate was washed with 10 ml each of water and 1M sodium bicarbonate. The organic layer was washed with 5 ml of 2N citric acid, 20 ml of 1M sodium bicarbonate, brine and dried ($MgSO_4$). The solvent was removed under vacuum to give 2.7 g of N-[N-(tert-butoxycarbonyl)- L -histidyl]-(S)-2-amino-4-methyl-(R)-1-(2-thiazolyl)pentan-1-ol as a foam.

The preceding compound (2.7 g) was dissolved in 5 ml of ethyl acetate under argon and 30 ml of 2N anhydrous hydrochloric acid in ethyl acetate was added by way of a syringe. After stirring for 2 hours crys tals had separated and the mixture was diluted with 35 ml of hexane. The mixture was filtered to give 4.5 g of solid. This solid was dissolved in 9 ml of water and made basic with 3 ml of concentrated ammonium

hydroxide. The mixture was extracted with ethyl acetate and the extract dried ($MgSO_4$). The solvent was removed under vacuum to give 1.23 g of solid (foam). A sample on standing crystallized to give crystals, mp 118 126 °C.

## Rreference Example 82

### N ( L -Histidyl)-(S)-2-amino-3-cyclohexyl-(R)-1-(2-thiazolyl)propan-1-ol

To a suspension of 0.59 g of $N^{\alpha}$-tert-butoxycarbonyl- L -histidine in 6 ml of dichloromethane was added 0.32 ml of triethylamine and 1.02 g of benzotriazol-1-yloxytris(dimethylamino)phosphonium hexafluorophosphate (BOP). After stirring for one minute, 0.53 g of (S)-2-amino-3-cyclohexyl-(R)-1-(2-thiazolyl)-propan-1-ol was added and the mixture was stirred at room temperature overnight. The mixture was concentrated under vacuum to give an oil. This oil in 20 ml of ethyl acetate was washed once with 5 ml of water, three times with 5 ml portions of 1M sodium bicarbonate and with 5 ml of brine. The organic layer was dried ($MgSO_4$) and filtered through 8.0 g of hydrous magnesium silicate. The hydrous magnesium silicate was washed with three 150 ml portions of ethyl acetate. The filtrate and ethyl acetate washes were combined and the solvent removed to give 1.0 g of solid. This solid was dissolved in dichloromethane and the solution filtered through diatomaceous earth. The filtrate was evaporated under reduced pressure to give 0.98 g of N-[$N^{\alpha}$-(tert-butoxycarbonyl)- L -histidyl]-(S)-2-amino-3-cyclohexyl(R)-1-(2-thiazolyl)propan-1-ol as a hard glass. The preceding compound (0.98 g) in one ml of ethyl acetate was treated with 10 ml of 2N anhydrous hydrochloric acid in ethyl acetate. After stirring for 2 hours 10 ml of hexane was added and the mixture filtered to give crystals. The crystals were dissolved in 3 ml of water and 0.4 ml of 15N ammonium hydroxide added. The mixture was extracted with ethyl acetate and the extract dried ($MgSO_4$). The solvent was removed to give 0.40 g of solid.

## Reference Example 83

### (S)-2-Amino-3-cyclohexyl-(R)-1-(2-pyridinyl)propan-1-ol

To a solution of 3.16 g of 2-bromopyridine in 20 ml of dry tetrahydrofuran cooled to -78 °C under argon was added 11.6 ml of 2.0M n-butyllithium in tetrahydrofuran. After 5 minutes, 3.14 g of N-methoxy-N-methyl-$N^{\alpha}$-tert-butoxycarbonyl- L -cyclohexylalaninamide in 20 ml of dry tetrahydrofuran was added. The dark solution was stirred at -78 °C for one hour. To the mixture was added 20 ml of saturated aqueous sodium sulfate solution. After warming to room temperature the mixture was poured into 50 ml of water and extracted with ethyl acetate. The organic layer was dried ($Na_2SO_4$) and the solvent removed. The residue was chromatographed on a silica gel column to give 2.01 g of 1,1-dimethylethyl-(S)[1-(cyclohexylmethyl)-2-oxo-2-(2-pyridinyl)ethyl]carbamate as an oil; $[\alpha]_D^{26}$ +135 ° ±1 (c = 0.812, methanol).

The preceding compound (6.49 g) was dissolved in 80 ml of tetrahydrofuran and the solution cooled to -78 °C. To this solution was added 40 ml of 1.0M lithium tri-sec-butyl borohydride in tetrahydrofuran over 10 minutes. The mixture was stirred at -78 °C for 3.5 hours and allowed to warm to room temperature. The solution was cooled to -8 °C, diluted with 20 ml of water and 20 ml of hydrogen peroxide added dropwise (temperature rose to 45 °C). The mixture was extracted with ethyl acetate, dried ($MgSO_4$) and the solvent removed to give 6.38 g of oil. This oil was chromatographed over silica gel with ethyl acetate-hexane (3:7) to give 3.12 g of (S)-2-(tert-butoxycarbonyl)amino-3-cyclohexyl-(R)-1-(2-pyridinyl)propan-1-ol an an oil. The oil was crystallized from hexane to give 1.5 g of crystals, mp 68-69 °C; $[\alpha]_D^{26}$ +9 ° ±1 (c = 1.07, methanol).

To a 1.0 g sample of the preceding compound in 2 ml of dichloromethane was added 2 ml of trifluoroacetic acid. After stirring for 6 hours 15 ml of 2N sodium hydroxide was added. The mixture was extracted with three 5 ml portions of dichloromethane, and the combined extracts dried ($K_2CO_3$). The solvent was removed to give 0.59 g of oil which was crystallized from diisopropyl ether to give 0.27 g of product as crystals, mp 59-60 °C.

## Reference Example 84

### N-[L-Histidyl]-(S)-2-amino-3-cyclohexyl-(R)-1-(2-pyridinyl)propan-1-ol

To 0.56 g of $N^{\alpha}$-tert-butoxycarbonyl-L-histidine in 2 ml of dichloromethane was added 0.30 ml of triethylamine and 0.97 g of benzotriazol-1-yloxytris(dimethylamino)phosphonium hexafluorophosphate. After 2 minutes, 0.47 g of (S)-2-amino-3-cyclohexyl-(R)-1-(2-pyridinyl)propan-1-ol in one ml of dichloromethane was added. The mixture was stirred overnight and refluxed for 5 minutes. The mixture was concentrated under vacuum and the residue in 10 ml of ethyl acetate washed with water (2 ml), 2M sodium carbonate (6 ml) and with brine (2 ml). The organic layer was dried ($Na_2SO_4$) and concentrated. The residue was dissolved in formic acid and the solution extracted with dichloromethane. The aqueous layer was treated with concentrated ammonium hydroxide and the mixture filtered to give 0.8 g of N-[N-(tert-butoxycarbonyl)-L-histidyl]-(S)-2-amino-3-cyclohexyl-(R)-1-(2-pyridinyl)propan-1-ol as an amorphous solid.

To the preceding compound (1.03 g) in 2 ml of dichloromethane cooled to 0 °C added 2 ml of trifluoroacetic acid. After 3 hours, 15 ml of 2N sodium hydroxide was added while cooling in an ice bath. The mixture was extracted with three 5 ml portions of dichloromethane . The extract was dried($Na_2SO_4$) and the solvent removed under vacuum to give 0.4 g of solid. This solid was chromatographed on five 20 x 20 x 0.2 cm thick layer silica gel plates with dichloromethane-methanol-ammonium hydroxide (9:1.2:0.2) as solvent to give 0.19 g of the product of the Example as an amorphous solid; $[\alpha]_D^{26}$ +4° (c = 1.00, methanol).

A mixture of 0.41 g of $N^{\alpha}$-tert-butoxycarbonyl-L-histidine and 0.28 g of N,N-carbonyldiimidazole in 2 ml of tetrahydrofuran was stirred one hour and warmed briefly. To the solution was added 0.34 g of (S)-2-amino-3-cyclohexyl-(R)-1-(2-pyridinyl)propan-1-ol. The mixture was stirred overnight. To the mixture was added a mixture of 28 mg of $N^{\alpha}$-tert-butoxycarbonyl-L-histidine and 28 mg of N,N-carbonyldiimidazole in 0.2 ml of tetrahydrofuran which had been stirred 2 hours. The mixture was stirred for 2 hours and the solvent removed under vacuum. The residue in 5 ml of ethyl acetate was washed three times with 2 ml portions of 1M sodium carbonate, water and brine. The organic layer was dried ($Na_2SO_4$) and the solvent removed to give 0.66 g of foam. This foam was dissolved in 1.3 ml of dichloromethane, cooled to 0 °C and 1.3 ml of trifluoroacetic acid added. The solution was stirred for 2 days and 20 ml of 2N sodium hydroxide added. The mixture was extracted with three 10 ml portions of dichloromethane. The extract was concentrated under vacuum to give 0.39 g of solid. The solid was chromatographed on five 20 x 20 x 0.2 cm thick layer silica gel plates with dichloromethane-methanol-ammonium hydroxide (9:1.2:0.2) as solvent to give 0.14 g of the product of the Example as an amorphous solid.

## Reference Example 85

### N-[N-π-(Benzyloxymethyl)-L-histidyl]-(S)-2-amino-3-cyclohexyl-(R)-1-(2-thiazolyl)-1-(tert-butyldimethylsilyloxy)propane

To a suspension of 1.10 g of $N^{\alpha}$-tert-butoxycarbonyl-N-π-(benzyloxymethyl)-L-histidine in 3 ml of dichloromethane was added 0.41 ml of triethylamine. To this solution was added 1.28 g of benzotriazol-1-yloxytris(dimethylamino)phosphonium hexafluorophosphate. After stirring for 1.5 minutes, 0.87 g of (S)-2-amino-3-cyclohexyl-(R)-1-(2-thiazolyl)-1-[(tert-butyldimethyl)silyloxy]propane and 0.5 ml of dichloromethane. The mixture was stirred at room temperature overnight and the solvent removed under vacuum. The residue in 20 ml of ethyl acetate was washed three times each with 1N hydrochloric acid and 1M sodium bicarbonate. The organic layer was dried ($Na_2SO_4$) and the solvent removed and the residual solid dried to give 1.68 g of glassy solid.

The preceding solid (1.68 g) in 2 ml of dichloromethane was chilled to 0 °C and 2 ml of trifluoroacetic acid added. The mixture was allowed to warm to room temperature and was stirred overnight. The solvent was removed under reduced pressure and the residual gum dissolved in 10 ml of ethyl acetate. The solution was washed twice with 10 ml of 7.5N ammonium hydroxide and brine. The organic layer was dried

($Na_2SO_4$) and the solvent removed to give 1.4 g of product as an amorphous solid.

Reference Example 86

N-( L -Histidyl)-(S)-2-amino-4-methyl-(R)-1-(2-thiazolyl)pentan-1-ol

A mixture of 3.44 g of $N^{\alpha}$-tert-butoxycarbonyl-N-imidazole-tosyl- L -histidine, 1.44 g of diethyl cyanophosphate and 1.3 ml of triethylamine in 50 ml of dichloromethane was stirred at 0° C for one hour. A 1.4 g portion of (S)-2-amino-4-methyl-(R)-1-(2-thiazolyl)pentan-1-ol was added and stirring was continued at 0° C for 20 hours then for one hour at room temperature. The mixture was diluted with dichloromethane and washed with 50% saturated sodium bicarbonate. The aqueous phase was separated and extracted with dichloromethane. The organic phase and extract were combined, washed with water and brine, dried and evaporated in vacuo to a gum. This gum was dissolved in 70 ml of methanol and 2.84 g of 1-hydroxybenzotriazole added. The mixture was stirred for 21 hours and the solvent removed in vacuo. The residue was dissolved in ethyl acetate, washed with 1N sodium hydroxide, brine, dried and the solvent removed in vacuo. The residue was chromatographed on a flash silica gel column, eluting with 5% methanol in ethyl acetate, to give 1.33 g of $N^{\alpha}$-tert-butoxycarbonyl- L -histidyl-(1R,2S)-2-amino-4-methyl-1-(2-thiazolyl)pentan-1-ol; $[\alpha]_D^{26}$ -34° ±1 (c = 0.848, methanol).

A 1.1 g portion of the above compound was dissolved in a mixture of 5 ml of dichloromethane and 1.9 ml of trifluoroacetic acid and stirred for 16 hours. The solution was treated with 15 ml of 1N sodium hydroxide which was saturated with sodium chloride and extracted with 3 x 30 ml of dichloromethane. The extracts were combined, dried and evaporated in vacuo. The residue was triturated with ether-hexane , giving L -histidyl-(1R,2S)-2-amino-4-methyl-1-(2-thiazolyl)pentan-1-ol as a solid.

Reference Example 87

N-( L -Histidyl)-(S)-2-amino-3-cyclohexyl-(R)-1-(2-thiazolyl)propan-1-ol

A mixture of 0.45 g of phenyl dichlorophosphate and 0.71 g of imidazole in 10 ml of dichloromethane under argon, was allowed to stand 0.5 hour, then cooled to 0° C and 0.86 g of $N^{\alpha}$-tert-butoxycarbonyl- N-imidazole-tosyl- L -histidine was added. The mixture was stirred for 2 hours at 0° C, then a solution of 0.48 g of (S)-2-amino-3-cyclohexyl-(R)-1-(2-thiazolyl)propan-1-ol in 2 ml of dichloromethane was added. The mixture was stirred at 0° C for 17 hours and then at room temperature for one day. A 30 ml portion of dichloromethane was added and the solution washed with 30 ml of 50% saturated sodium bicarbonate. The aqueous layer was separated and extracted with 2 x 20 ml of dichloromethane. The organic layer and extracts were combined, washed with 30 ml of water and 30 ml of brine, dried and the solvent removed. The residue was washed with 4 x 10 ml of hexane:ether (4:1) to give 1.34 g of white solid.

To 1.2 g of the above solid in 30 ml of methanol was added 0.77 g of 1-hydroxybenzotriazole. This mixture was stirred for 20 hours, then the solvent was removed in vacuo. The residue was dissolved in 60 ml of ethyl acetate, washed with 3 x 10 ml of 1N sodium hydroxide and 10 ml of brine, dried and the solvent removed. The residue was flash chromatographed on silica gel, eluting with 5% methanol in ethyl acetate and gave 0.61 g of tert-butoxycarbonyl- L -histidyl-(1R,2S)-2-amino-3-cyclohexyl-1-(2-thiazol-1)-propan-1-ol as a white solid; $[\alpha]_D^{26}$ -55° ±2 (c = 0.46, methanol).

A 0.48 g portion of the above compound in 2 ml of dichloromethane and 0.8 ml of trifluoroacetic acid was stirred for 20 hours, then treated with 15 ml of sodium chloride saturated 1N sodium hydroxide and extracted with 3 x 30 ml of dichloromethane. The extracts were combined, dried and evaporated. The residue was triturated with ether-hexane, giving 0.35 g of L -histidyl-(1R,2S)-2-amino-3-cyclohexyl-1-(2-thiazolyl)propan-1-ol as a white solid; $[\alpha]_D^{26}$ -41° ±3 (c = 0.288, methanol).

## Reference Example 88

### (S)-2-Amino-4-(methylthio)-(R)-1-(2-thiazolyl)butan-1-ol

To a mixture of 17.6 g of imidazole in 150 ml of dichloromethane was added dropwise 11.0 g of phenyl dichlorophosphate. The mixture was cooled to 0° C and 12.4 g of $N^{\alpha}$-tert-butoxycarbonyl- L -methionine in 60 ml of dichloromethane was added at a rate to maintain the temperature of the reaction at 0° C to 5° C. The mixture was stirred at 0° C for one hour and 5.1 g of N-methoxy-N-methylamine hydrochloride added. The mixture was stirred overnight at room temperature, filtered and the solid washed with dichloromethane. The filtrate was washed with 100 ml each of 2N citric acid, 1M sodium bicarbonate, saturated sodium chloride and dried ($MgSO_4$). The solvent was removed under reduced pressure to give 11.3 g of colorless oil; $[\alpha]_D^{26}$ -45° ±1 (c = 0.989, methanol).

The preceding compound (11.3 g) was dissolved in 320 ml of ether under argon and 1.85 g of solid lithium aluminumhydride (LAH) added in small portions (solution becomes warm and refluxes). After 0.5 hour the solution was cooled in an ice bath and 320 ml of 1M potassium hydrogen sulfate ($KHSO_4$) added slowly. The organic layer was separated and the aqueous layer extracted three times with 200 ml of ether. The organic layer and extracts were combined and washed with three 100 ml portions of 3N hydrochloric acid, two 100 ml portions of 1M sodium bicarbonate and 50 ml of brine. The organic layer was dried ($MgSO_4$) and the solvent removed under vacuum to give 8.68 g of $N^{\alpha}$-tert-butoxycarbonyl- L -methioninal as an oil.

To a solution of 6.8 g of the preceding compound in 20 ml of dichloromethane was added 4.7 g of 2-trimethylsilylthiazole in 5 ml of dichloromethane (reaction temperature rose to 42° C). The mixture was stirred under argon at room temperature overnight. The solution was cooled to 0° C and 13 ml of a 1.0M solution of tetrabutylammonium fluoride in tetrahydrofuran added dropwise. The mixture was refluxed on a steam bath for 1.5 hours and the solvent removed under vacuum. The residue in 40 ml of ethyl acetate was washed with 25 ml each of 2N citric acid, 1M sodium bicarbonate, brine and dried ($MgSO_4$). The solvent was removed to give 10.4 g of an oil.

To the preceding oil (10.4 g) in 10 ml of ethyl acetate was added 10 equivalents of 2N anhydrous hydrochloric acid in ethyl acetate. After stirring at room temperature for 2 hours, the mixture was cooled to 0° C, diluted with 50 ml of hexane, chilled and filtered. The solid was quickly dissolved in 15 ml of water and 4 ml of concentrated ammonium hydroxide added dropwise. The mixture was extracted three times with dichloromethane and the extract dried ($MgSO_4$) and the solvent removed to give 1.72 g of oil. The mother liquors from the 2N HCl-ethyl acetate treatment were extracted with 1N aqueous hydrochloric acid and the aqueous layer treated with concentrated ammonium hydroxide. The mixture was extracted with dichloromethane to give an additional 0.82 g of oil.

The combined oil (1.72 g plus 0.82 g) was chromatographed on silica gel by HPLC on Water-prep 500 instrument with 5% methanol in ethyl acetate containing 3% N-methylmorpholine as solvent. The fractions containing product were combined and a portion of the oil obtained was sublimed to give 0.159 g of crystals, mp 63-65° C; $[\alpha]_D^{26}$ -29° ±1 (c = 1.01, methanol).

## Reference Example 89

### N-( L -Leucyl)-(S)-2-amino-4-(methylthio)-(R)-1-(2-thiazolyl)butan-1-ol

To a sample of 1.6 mmol of $N^{\alpha}$-(tert-butoxycarbonyl)- L -leucine in 1.6 ml of dichloromethane (dried over magnesium sulfate) was added 0.22 ml of triethyl amine and 0.71 g of benzotriazol-1-yloxytris-(dimethylamino)phosphonium hexafluorophosphate. After one minute 0.32 g of (S)-2-amino-4-(methylthio)-(R)-1-(2-thiazolyl)butan-1-ol was added. The mixture was stirred overnight and the solvent removed under vacuum to give 0.60 g of N-[N-(tert-butoxycarbonyl)- L -leucyl]-(S)-2-amino-4-(methylthio)-(R)-1-(2-thiazolyl)butan-1-ol as an oil.

To the preceding compound (0.60 g) in 2 ml of ethyl acetate was added 7.5 ml of 2N anhydrous

hydrochloric in ethyl acetate. The mixture was stirred at room temperature for 2 hours and 10 ml of hexane added. The mixture was chilled and filtered to give a yellow solid. This solid was dissolved in 2 ml of water and 0.3 ml of concentrated ammonium hydroxide added. The mixture was filtered to give 0.29 g of the product of the Example as crystals, mp 83-85° C.

## Reference Example 90

### N-( L -Histidyl)-(S)-2-amino-4-methyl-(R)-1-(2-thiazolyl)pentan-1-ol

To a suspension of 2.4 g of $N^{\alpha}$-tert-butoxycarbonyl- L-histidine in 24 ml of dichloromethane was added 1.3 ml of triethylamine, followed by the addition of 4.2 g of benzotriazol-1-yloxytris(dimethylamino)-phosphonium hexafluorophosphate (BOP). The mixture was stirred under argon for 2 minutes and 1.73 g of (S)-2-amino-4-methyl-(R)-1-(2-thiazolyl)pentan-1-ol added. The mixture was stirred for 4 days and the solvent removed. The residue was dissolved in 20 ml of ethyl acetate and the solution washed with 10 ml of water, three 10 ml portions of 1M sodium bicarbonate, 5 ml of 2N citric acid and 20 ml of 1M sodium bicarbonate. The organic layer was dried ($MgSO_4$) and then filtered through a thin pad of hydrous magnesium silicate (pad washed with ethyl acetate). The filtrate was concentrated under vacuum to give 2.7 g of N-[N-(tert-butoxy carbonyl)- L -histidyl]-(S)-2-amino-4-methyl-(R)-1-(2-thiazolyl)pentan-1-ol as a foam. This foam (2.7 g) was dissolved in 5 ml of ethyl acetate and to the solution was added 30 ml of 2N anhydrous hydrochloric acid in ethyl acetate. The mixture was stirred for 2 hours (crystals separated) and 35 ml of hexane added. The mixture was filtered to give 4.55 g of N-( L -histidyl)-(S)-2-amino-4-methyl-(R)-1-(2-thiazolyl)pentan-1-ol, dihydrochloride as white hygroscopic crystals. The crystals were dissolved in 9 ml of water and 3 ml of concentrated ammonium hydroxide added. The mixture was extracted with ethyl acetate, the extract dried ($MgSO_4$) and the solvent removed under vacuum to give 1.23 g of N-( L-histidyl)-(S)-2-amino-4-methyl-(R)-1-(2-thiazolyl)pentan-1-ol as a foam.

## Reference Example 91

### N-( L -Histidyl)-(S)-2-amino-3-cyclohexyl-(R)-1-(2-thiazolyl)-1-(tert-butyldimethylsilyloxy)propane

To a solution of 25 ml of 3.9 g of imidazole in 25 ml of dichloromethane was added 1.7 ml of phenyl dichlorophosphate. After stirring under nitrogen at room temperature for 20 minutes, the mixture was chilled to 0° C and 2.94 g of $N^{\alpha}$-tert-butoxycarbonyl- L -histidine was added. The mixture was stirred at 0° C for one hour and 3.68 g of (S)-2-amino-3-cyclohexyl-(R)-1-(2-thiazolyl)-1-(tert-butyldimethylsilyloxy)propane in 5 ml of dichloromethane added. The mixture was stirred at room temperature overnight, filtered and the filtrate concentrated under vacuum. The residue in 50 ml of ethyl acetate was washed (three times) with 10 ml of 2M sodium carbonate, with brine. The aqueous layer was extracted with ethyl acetate. The organic layer and ethyl acetate extracts were combined and the solvent removed to give 7.0 g of a foam (after drying under vacuum). This solid was dissolved in 10 ml of dichloromethane and the solution cooled to 0° C. To the solution was added 10 ml of trifluoroacetic acid. The solution was stirred at room temperature over night and then refluxed for 2 hours. The solution was cooled to 0° C and added to 130 ml of 2N sodium hydroxide. The mixture was filtered and the organic layer separated. The aqueous layer was extracted with ethyl acetate. The organic layer and extracts were combined and dried ($Na_2SO_4$ ). The solvent was removed under vacuum to give 4.93 g of solid. This solid was chromatographed on silica gel with a Waters-prep 500 HPLC apparatus with dichloromethane-methanol-triethylamine (95:4:1) as solvent. The fractions containing product were combined, the solvent removed and the solid dissolved in ethyl acetate. The solution was washed three times with 5 ml portions of brine, dried ($Na_2SO_4$) and filtered through diatomaceous earth. The filtrate was concentrated and the residue dried under vacuum to give 3.92 g of foam: $[\alpha]_D^{26}$ -43° ±1 (c = 1.099, methanol); FAB mass spectrum - M+H = 492.

## Reference Example 92

## (S)-2-Amino-3-cyclohexyl-(R)-1-(2-furanyl)propan-1-ol

A. 1,1-Dimethylethyl (S)-[1-(cyclohexylmethyl)-2-(2-furanyl)-2-oxoethyl]carbamate

A solution of 1.57 g of N-methoxy-N-methyl $N^{\alpha}$-t-butoxycarbonyl- L -cyclohexylalaninamide in 15 ml of dry tetrahydrofuran was cooled to -78° C under argon. To the solution was added dropwise 5.9 ml of secondary butyllithium (0.85M in hexane). The viscous mixture was stirred at -78° C for 1.5 hours and then warmed to 0° C and stirred for 5 minutes. (Solution A)

A solution of 0.73 ml of furan in 5 ml of dry tetrahydrofuran was cooled to 0° C and 3.8 ml of n-butyllithium (2.35M in hexane) added. The yellow suspension was stirred at 0° C for 1.7 hours and then allowed to warm to room temperature for 15 minutes. (yellow solution B)

The yellow solution B was added to solution A and the mixture stirred at 0° C for 1.5 hours. The mixture was quenched with 5 ml of saturated aqueous ammonium chloride and the solvent tetrahydrofuran removed under vacuum. The residue was diluted with 50 ml of ethyl acetate and 20 ml of 1N hydrochloric acid. The organic phase was separated and washed successively with 20 ml of 1N hydrochloric acid, 20 ml of water, 20 ml of saturated sodium bicarbonate, 20 ml of brine and dried over sodium sulfate. The solvent was removed under vacuum to give 1.63 g of a light brown gum. This gum was dissolved in ether-hexane (1:5) and the solution filtered through a thin pad of hydrous magnesium silicate. The pad was washed with ether-hexane (1:5) and the filtrate concentrated. The residue was triturated with hexane to give 1.23 g of light yellow crystals; $[\alpha]_D^{26}$ +41° ±1 (c = 1.14, methanol).

B. (S)-2-(N-tert-Butoxycarbonyl)amino-3-cyclohexyl-(R,S)-1-(2-furanyl)propan-1-ol

A solution of 0.16 g of 1,1-dimethylethyl-(S)-[1-(cyclohexylmethyl)-2-(2-furanyl)-2-oxoethyl]carbamate in 2 ml of dry tetrahydrofuran and 0.2 ml of methanol was cooled to 0° C under argon and 23 mg of sodium borohydride added. The solution was stirred at 0° C for one hour and quenched with 2 ml of saturated aqueous ammonium chloride. The organic solvent was removed under vacuum and the residue diluted with 5 ml of saturated aqueous ammonium chloride. The organic solvent was removed under vacuum and the residue diluted with 5 ml of water and extracted with 10 ml of ethyl acetate. The organic layer was separated, washed successively with 5 ml of 0.5N hydrochloric acid, 5 ml of saturated sodium bicarbonate, 5 ml of brine and dried over sodium sulfate. The solvent was removed under vacuum to give 0.19 g of gummy solid.

C. (4S-trans)-4-(Cyclohexylmethyl)-5-(2-furanyl)-2-oxazolidione

To a solution of 0.23 g of (S)-2-(N-tert-butoxycarbonyl)amino-3-cyclohexyl-(R,S)-1-(2-furanyl)propan-1-ol in 3 ml of dichloromethane was added 0.06 ml of trifluoroacetic acid. The solution was stirred for 23 hours at room temperature, washed with 1N sodium hydroxide, dried over sodium sulfate and the solvent removed to give 0.17 g of solid. This solid was dissolved in dichloromethane-ethyl acetate (9:1) and filtered through a thin pad of hydrous magnesium silicate. The filter pad was washed with two 10 ml portions of dichloromethane-ethyl acetate (9:1) and the filtrate and washes combined. The solvent was removed and residual solid washed with hexane to give 0.10 g of white crystals; $[\alpha]_D^{26}$ -124° ±2 (c = 0.417, methanol).

D. (S)-2-Amino-3-cyclohexyl-(R)-1-(2-furanyl)propan-1-ol

A 0.15 g sample of (4S-trans)-4-(cyclohexylmethyl)-5-(2-furanyl)-2-oxazolidinone was dissolved in a mixture of 3 ml of ethanol and 3 ml of 1N sodium hydroxide. The solution was refluxed for 17 hours, diluted with 3 ml of water and concentrated under vacuum to remove the ethanol. The aqueous residue was extracted with two 5 ml portions of dichloromethane and the extracts dried over sodium sulfate. The solvent

was removed to give 0.15 g of solid which was washed with hexane to give 0.13 g of white solid; $[\alpha]_D^{26}$ -10° ±2 (c = 0.507, methanol).

## Reference Example 93

### (S)-2-Amino-4-methyl-(R)-1-(2-thienyl)pentan-1-ol

A 0.81 g portion of (S)-2-(tert-butoxycarbonyl)amino-4-methyl-(R,S)-1-(2-thienyl)pentan-1-ol was dissolved in 5 ml of dichloromethane and 2.1 ml of trifluoroacetic acid added. This mixture was stirred for 3 hours, then poured with stirring into 15 ml of ice- cold 2N sodium hydroxide. The mixture was diluted with 25 ml of dichloromethane, the organic layer separated and the aqueous layer extracted with 20 ml of dichloromethane. The organic layer and extract were combined, washed with saturated sodium chloride solution, dried and the solvent removed in vacuo. The residue was chromatographed on a silica gel column with ethyl acetate:hexane (1:4), giving 0.72 g of (4S-trans)-4-(2-methylpropyl)-5-(2-thienyl)-2-oxazolidinone as a white solid; $[\alpha]_D^{26}$ -141° ±2 (c = 0.570, methanol).

A 0.23 g portion of the above solid was dissolved in 5 ml of 1N sodium hydroxide added. The solution was refluxed for 16 hours and then concentrated in vacuo. The residue was extracted with two 10 ml portions of dichloromethane. The extracts were combined, dried and the solvent removed in vacuo, giving 0.2 g of the desired compound; Rf 0.045 [silica gel; ethyl acetate-hexane (1:2)].

## Reference Example 94

### N-( L -Histidyl)-(S)-2-amino-3-cyclohexyl-(R)-1-(2-thienyl)-propan-1-ol

A mixture of 1.18 g of $N^{\alpha}$-tert-butoxycarbonyl- L -histidine and 0.65 ml of triethylamine in 6 ml of chloroform was stirred and warmed on a steam bath until most of the solid dissolved. To this mixture was added 2.04 g of benzotriazol-1-gloxy-tris(dimethylamino)phosphonium hexafluorophosphate (BOP) in 2.5 ml of chloroform. The mixture was warmed on a steam bath for 3 minutes and 1.0 g of (S)-2-amino-3-cyclohexyl-(R)-1-(2-thienyl)propen-1-ol added. The mixture was stirred at room temperature overnight and refluxed for 5 minutes. The solvent was removed under reduced pressure and the residue dissolved in 25 ml of ethyl acetate. The solution was washed with 10 ml of water, three 10 ml portions of 2M sodium carbonate and 10 ml of brine. The organic layer was dried ($Na_2SO_4$), the solvent removed and the residue dried under vacuum to give 1.7 g of N-[N-(tert-butopycarbonyl)- L -histidyl]-(S)-2-amino-3-cyclohexyl-(R)-1-(2)-thienyl)propan-1-ol as a white foam. To a 0.40 g sample of the preceding compound in one ml of tetrahydrofuran cooled to 0° C, was added 3 ml of ice-cold 4N hydrochloric acid. The resulting solution was chilled at 0° C to 4° C for seven days and filtered. To the filtrate was added 1.5 ml of 10N sodium hydroxide. The mixture was extracted twice with 2 ml of dichloromethane and the extract dried ($Na_2SO_4$). The solution was applied to two 20x20x0.2 cm silica gel plates and the plates developed with dichloromethane-methanol-ammonium hydroxide (9:1.2:02). The product band was removed and extracted with 5% concentrated ammonium hydroxide in methanol. The extract was concentrated under vacuum to give 0.22 g of the product of the Example as a gum.

## Reference Example 95

### (S)-2-Amino-3-cyclohexyl-(R)-1-2[N-[2-(trimethylsilyl)ethoxy]methyl]imidazolyl)propan-1-ol

A solution (cooled to -78° C) of 17.82 g of 1-[[2-(trimethylsilyl)ethoxy]-1H-imidazole in 90 ml of dry tetrahydrofuran under argon was added slowly 36 ml of n-butyllithium in tetrahydrofuran (2.5 molar) and after complete addition the mixture was stirred at -78° C for 1 hour (Solution A).

To a stirred solution of 18.84 g of N-methoxy-N-methyl $N^{\alpha}$-t-butoxycarbonyl- L -cyclohexylalaninamide in 180 ml of dry tetrahydrofuran chilled to -78° C under argon was added 67 ml of secondary butyllithium in hexane (0.85 molar). After the addition the mixture was stirred for 1.5 hours at -78° C (Solution B).

By the use of double-tipped needle technique the solution A was added to the solution B with stir ring at -78° C. The mixture was stirred at -78° C for one hour and allowed to warm to 0° C and kept (ice bath) at 0° C for one hour. The mixture was quenched with 150 ml of saturated ammonium chloride solution and the tetrahydrofuran solvent removed under reduced pressure. The residual aqueous layer was extracted twice with 150 ml of ethyl acetate. The combined extract was washed with saturated sodium chloride and dried ($Na_2SO_4$). The solvent was removed under vacuum to give 35.0 g of dark orange oil. This oil (35 g) was chromatographed on a silica gel column with ethyl acetate-hexane (1:10) to give 17.82 g of 1,1-dimethylethyl-(S)-[1-(cyclohexylmethyl)-2-oxo-2-(2-[N-[2-trimethylsilyl)ethoxy]methyl]imidazolyl)ethyl]-carbamate as a yellow viscous oil; $[\alpha]_D^{26}$ +15° ±1 (c = 1.234, methanol).

To a solution of 19.12 g of the preceding compound in 170 ml of dry tetrahydrofuran cooled to -78° C was added dropwise 85 ml of potassium tri-sec-butylborohydride in tetrahydrofuran (1.0M). After the addition, the mixture was stirred at -78° C for 2 hours and 170 ml of saturated ammonium chloride solution was added. The mixture was allowed to warm to room temperature and the tetrahydrofuran removed under vacuum. The aqueous residue was diluted with 50 ml of hydrogen peroxide (30% by weight in water) added dropwise (exothermic). After the addition was complete, the ice bath was removed and the mixture stirred for one hour. The mixture was cooled to 0° C and 93 g of sodium sulfate added in six equal portions. The mixture was stirred for 15 minutes, the organic layer separated, and the aqueous layer extracted with 200 ml of ethyl acetate. The organic layer and the extract were combined, dried over $Na_2SO_4$ and the solvent removed to give a white solid. The solid was dissolved in a mixture of 400 ml of hexane and 50 ml of ethyl acetate by warming on a steam bath. Cooling gave 14.13 g of (S)-2-tert-butoxycarbonylamin-3-cyclohexyl-(R)-1-(2-(trimeth ylsilyl)ethoxy]methyl]imidazolyl)propan-1-ol as white crystals, mp 97-99° C; $[\alpha]_D^{26}$ +8° ±1 (c = 1.15, methanol).

A 20 g sample of the preceding compound was dissolved in 110 ml of ethanol. To the stirred solution was added 110 ml of 2N hydrochloric acid and the solution heated in an oil bath at 70° C to 75° C for 2 hours. The solution was diluted with 85 ml of water and concentrated under vacuum to remove the ethanol. The aqueous residue was chilled to 0° C and made basic with 2N sodium hydroxide. The mixture was extracted twice with 300 ml of ethyl acetate and the extracts combined. The extract was washed with 250 ml of brine, dried ($Na_2SO_4$) and the solvent removed under vacuum. The residue was dried at 55° C for 5 hours to give 14.1 g of (S)-2-amino-3-cyclohexyl-(R)-1-(2-[N-[2-(trimethylsilyl)ethoxy]methyl]imidazole)-propan-1-ol as a light yellow oil; $[\alpha]_D^{26}$ -8° ±1 (c = 1.05, methanol).

## Reference Example 96

### N-( L -Histidyl)-(S)-2-amino-3-cyclohexyl-(R)-1-2(2-[N-[2-(trimethylsilyl)ethoxy]methyl]imidazolyl)propan-1-ol

To a solution of 30.45 g of imidazole in 150 ml of chloroform under argon was added 12.60 of phenyl dichlorophosphate in 50 ml of chloroform. The mixture was stirred at room temperature for 0.5 hour and then cooled to 0° C. To the cooled mixture was added 15.24 g of $N^{\alpha}$-tert-butoxycarbonyl- L -histidine and the mixture stirred for 2 hours. To the solution was added dropwise 14.1 g of (S)-2-amino-3-cyclohexyl-(R)-1-(2-[N-[2-(trimethylsilyl)ethoxy]methyl]imidazolyl)propan-1-ol in 40 ml of chloroform. The mixture was stirred at 0° C for 44 hours, diluted with 400 ml of water and extracted with one liter of ethyl acetate. The extract was washed with two 350 ml portions of water, 350 ml of saturated sodium carbonate solution and dried ($Na_2SO_4$). The solvent was removed to give 29.0 g of solid. This solid was dissolved in 200 ml of methanol and 100 ml of 1N sodium hydroxide added. The solution was stirred overnight at room temperature, diluted with 100 ml of water and concentrated under vacuum. The mixture was extracted with two 300 ml portions of ethyl acetate and the extract washed with 250 ml of brine and dried ($Na_2SO_4$) the solvent was removed under vacuum to give 24.6 g of a glass.

Reference Example 97

N-( L -Histidyl)-(S)-2-amino-3-cyclohexyl-(R)-1-(2-furanyl)propan-1-ol

As described for Reference Example 94, a mxture of 1.28 g of N-tert-butoxycarbonyl- L -histidine, 0.72 ml of trimethylamine and 0.005 mole of benzotriazol-1-gloxytris(dimethylamino)phosphonium hexafluorophosphate in 10 ml of chloroform was warmed and stirred for 3 minutes. To this mixture was added 1.12 g of (S)-2-amino-3-cyclohexyl-(R)-1-(2-furanyl)propan-1-ol and the mixture stirred for 16 hours. Work up and removal of the N-tert-butoxycarbonyl blocking group as described for Reference Example 94 gave 0.2 g of the product as a glass.

Reference Example 98

N-[N-(Benzyloxycarbonyl)- D , L -[2-(2-methylpropoxy)glycyl]-(S)-2-amino-3-cyclohexyl-(R)-1-(2-pyridinyl)-propan-1-ol

A mixture of 30.33 g of benzyl carbonate, 20.2 g of glyoxylic and in 200 ml of ether was stirred at room temperature overnight. The solution was cooled at 0 °C for 30 minutes, filtered and the solid washed with ether. The solid was dried to give 28.2 g of N-(benzyloxycarbonyl)-2-hydroxyglycine as crystals, mp 194-198 °C.

A mixture of the preceding compound (4.0 g) in 80 ml of 2-methylpropanol and 0.4 ml of concentrated sulfuric and was stirred at room temperature overnight. The solution was diluted with 100 ml of ethyl acetate and washed with 10% sodium bicarbonate, water and brine. The organic layer was dried ($MgSO_4$) and the solvent removed. The residue (5.8 g) was dissolved in chloroform and the solution filtered through a short silica gel column. The filtrate was concentrated under vacuum to give 3.36 g of 2-methylpropyl N-(benzyloxycarbonyl)-2-(2-methylpropoxyl)glycinate as a clear oil.

The preceding compound (3.9 g) was dissolved in 120 ml of methanol and 11.6 ml of 1M sodium hydroxide added. The solution was stirred overnight and 3.9 ml of 3N hydrochloric acid added. The solvent was removed and the residue dissolved in 10% $NaHCO_3$. The mixture was extracted twice with ether. The aqueous layer was acidified with 6N hydrochloric acid, and extracted with dichloromethane. The dichloromethane extract was dried ($MgSO_4$) and the solvent removed to give 2.97 g N-(benzyloxycarbonyl)-2-(2-methylpropoxy)glycine as a white solid, mp 66-68 °C. To a solution of 0.432 g of the preceding compound in 10 ml tetrahydrofuran was added 0.249 g of N,N-carbonyldiimidazole. After stirring at room temperature for 2 hours, 0.30 g of (S)-2-amino-3-cyclohexyl-(R)-1-(2-pyridinyl)propan-1-ol was added and the solution was stirred overnight. The solvent was removed and the residue in dichloromethane washed with 10% $NaHCO_3$. The aqueous layer was extracted with dichloromethane. The organic layer and extract were combined and washed with brine, dried ($Na_2SO_4$) and the solvent removed under vacuum. The residue was dried under vacuum to give 0.653 g of the product as a gum: Mass spectrum (FAB); calc (M + H), 498.2978: Found (M + H), 498.2968.

Reference Example 99

(S)-2-Amino-3-cyclohexyl-(R)-1-(5-acetyl-2-furanyl)propan-1-ol

To a solution of 0.50 g of (4S-trans)-4-(cyclohexylmethyl)-5-(2-furanyl)-2-oxazolidinone in 8 ml of tetrahydrofuran, cooled to -78 °C, was added 1.8 ml of n-butyllithium in hexane (2.2M). After 15 minutes the solution was warmed to room temperature and N-methoxy-N-methyl acetamide in one ml of tetrahydrofuran

was added. The mixture was stirred at room temperature for 3 hours and quenched with 4 ml of saturated ammonium chloride solution and 4 ml of water. The mixture was concentrated under vacuum to remove the tetrahydrofuran and then extracted with 20 ml of ethyl acetate. The extract was washed with 10 ml each of 1N hydrochloric acid, saturated sodium bicarbonate and brine. The organic layer was dried ($Na_2SO_4$) and the solvent removed to give 0.58 g of solid. Flash chromatography on silica gel with ethyl acetate-hexane (1:1) as solvent gave 0.29 g of (4S-trans)-4-(cyclohexylmethyl)-5-(5-acetyl-2-furanyl)-2-oxazolidinone as a cream colored solid; $[\alpha]_D^{26}$ -116° ±1 (c = 0.773, methanol).

The preceding compound was dissolved in a mixture of 4 ml of ethanol and 4 ml of 1N sodium hydroxide and the solution heated at 80° C for 6 hours. The solution was diluted with 4 ml of water, concentrated to remove the ethanol, and extracted twice with 8 ml of dichloromethane. The extract was dried ($Na_2SO_4$) and the solvent removed to give 0.11 g of solid. Flash chromatography on silica gel with 10% methanol in dichloromethane gave 85 mg of product as a yellow solid: $[\alpha]_D^{26}$ +64° ±2 (c = 0.464, methanol).

The following Reference Examples may be prepared by the procedure of Reference Example 99.

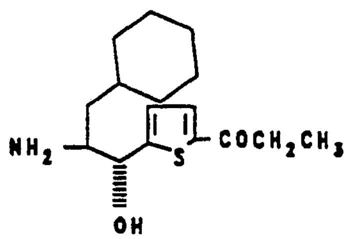

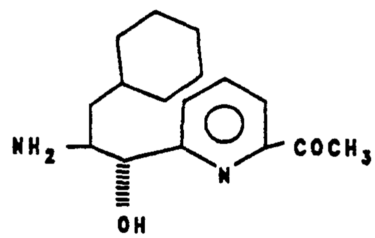

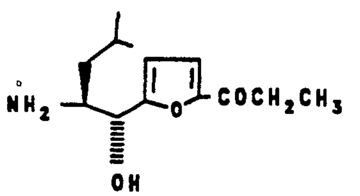

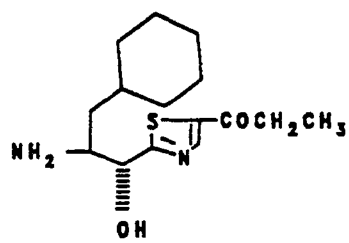

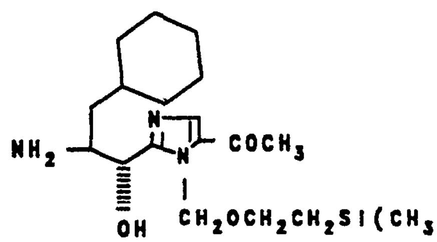

Reference Example 100

(S)-2-Amino-3-cyclohexyl-(R)-1-(5-methoxycarbonyl-2-thiazolyl)-1-(tert-butyldimethylsilyloxy)propane

To a solution of 0.23 g of N-tert-butoxycarbonyl-(S)-2-amino-3-cyclohexyl-(R)-1-(2-thiazolyl)-1-(tert-butyldimethylsilyloxy)propane in 2 ml of tetrahydrofuran under argon at -78° C, was added 0.45 ml of n-butyllithium in hexane (2.2M). After 0.5 hour at -78° C, the mixture was allowed to warm to 0° C and stand for 0.5 hour. Carbon dioxide gas was bubbled through the stirred suspension for 1 hour at 0° C and the mixture diluted with 2 ml of water and 2 ml of saturated ammonium chloride. The mixture was concentrated under vacuum and the residue extracted with 15 ml of diethyl ether. The ether extract was washed twice with 5 ml of 1N sodium hydroxide and with 5 ml of brine. A white precipitate formed in the ether layer and was filtered off and washed with ether. This white precipitate was combined with the sodium hydroxide

washes and the mixture acidified with 6N hydrochloric acid. The acidified mixture was extracted with 15 ml of ethyl acetate and the extract washed with brine and dried ($Na_2SO_4$). The solvent was removed to give a solid which crystallized from isooctane to give 0.20 g of N-tert-butoxycarbonyl-(S)-2-amino-3-cyclohexyl-(R)-1-[2-(5-carboxy)thiazolyl]-1-(tert-butyldimethylsilyloxy)propane.

The preceding compound (0.13 g) in 0.5 ml of tetrahydrofuran was added to a solution of 49 mg of N,N-carbonyldiimidazole in 0.5 ml of tetrahydrofuran under argon. The mixture was stirred at room temperature for one hour and 0.5 ml of dry methanol added. The solution was stirred at room temperature for 17 hours and the solvent removed. The residue in 10 ml of ethyl acetate was washed with two 5 ml portions of 0.5N hydrochloric acid, 5 ml of 1N sodium hydroxide, brine and dried ($Na_2SO_4$). The ethyl acetate soution was filtered through a thin pad of hydrous magnesium silicate and the pad washed with ethyl acetate. The filtrate was evaporated under vacuum to give 0.12 g of N-tert-butoxycarbonyl-(S)-2-amino-3-cyclohexyl-(R)-1-(5-methoxycarbonyl-2-thiazolyl)-1-(tert-butyldimethylsilyloxy)propane as a gum.

The preceding compound (0.12 g) was dissolved in one ml of dichloromethane and 0.18 ml of trifluoroacetic acid added. The solution was stirred at room temperature for 22 hours and then poured into 3 ml of ice-cold 1N sodium hydroxide. The mixture was extracted with 10 ml of dichloromethane and the extract dried ($Na_2SO_4$). The solvent was removed to give 0.095 g of the product as a colorless gum.

## Reference Example 101

### (4S-trans)-4-(Cyclohexylmethyl)-5-(2-pyridinyl)-2-oxazolidinone

As described in Reference Example 83, 20.27 g of 1,1-dimethylethyl-(S)-[1-(cyclohexylmethyl)-2-oxo-2-(2-pyridinyl)ethyl]carbamate in 300 ml of tetrahydrofuran at -78° C was reduced with 155 ml of potassium tri-sec-butyl-borohydride (0.155M) cooled to -78° C. After 5 hours at -78° C, the mixture was quenched with 50 ml of water and after warming to 0° C additional water (200 ml) was added. The mixture was extracted with two 250 ml portions of ethyl acetate and the extract dried ($Na_2SO_4$) and the solvent removed. The residue (41 g) was dissolved in 30 ml of tetrahydrofuran and 150 ml of 6N hydrochloric acid added. The mixture was stirred at room temperature for 5 hours, diluted with 40 ml of water and extracted with 50 ml of ether. The aqueous layer chilled and 110 ml of 10N sodium hydroxide added slowly. The mixture was extracted with three 200 ml portions of dichloromethane and the extract washed with brine (200 ml) and dried over anhydrous potassium carbonate. The solvent was removed to give 15.0 g of solid. This solid was chromatographed on silica gel with a Waters-HPLC Prep-500 apparatus with dichloromethane-methanol-triethylamine (97:2:1) as solvent to give 9.0 g of (S)-2-amino-3-cyclohexyl-(R)-1-(2-pyridinyl)propanol as crystals, mp 53-55° C and 3.0 g of (S)-2-amino-3-cyclohexyl-(S)-1-(2-pyridinyl)propanol as an oil.

A 2.8 g sample (S)-2-amino-3-cyclohexyl-(S)-1-(2-pyridinyl)propan-1-ol was dissolved in 24 ml of dioxane-water (1:1) and 3.14 g of η-butylpyrocarbonate in 13 ml of dioxane added, dropwise, with stirring. The solution was stirred 17 hours, diluted with 20 ml of water and extracted with two 75 ml portions of ethyl acetate. The extract was washed with brine, dried ($Na_2SO_4$) and the solvent removed. The residual oil was chromatographed on silica gel with ethyl acetate-hexane (4:6) as solvent to give 3.0 g of N-(tert-butoxycarbonyl)-(S)-2-amino-3-cyclohexyl-(S)-1-(2-pyridinyl)propan-1-ol as white crystals; $[\alpha]_D^{26}$ -14° ±1 (c = 0.984, chloroform) mp 84-85° C. To the preceding compound (0.334 g) in 10 ml of dichloromethane was added 0.20 ml of triethylamine and the mixture cooled to 5° C. To the solution was added 0.10 ml of methanesulfonyl chloride and the mixture stirred for 1.5 hours at 5° C. The solvent was removed and the residue dissolved in methanol and allowed to stand at room temperature for 24 hours. The mixture was diluted with 20 ml of water and extracted with 50 ml of ethyl acetate. The extract was washed with water, brine, dried ($Na_2SO_4$) and the solvent removed to give a pale yellow oil. Chromatography on silica gel with ethyl acetate-heptane (2:3) gave 0.195 g of (4-S-trans)-4-(cyclohexylmethyl)-5-(2-pyridinyl)-2-oxazolidinone as thick pale yellow oil $[\alpha]_D^{26}$ -46° ±1 (c = 1.08, methanol).

The preceding compound was also prepared by reacting 0.300 g of (S)-2-amino-3-cyclohexyl-(R)-1-(2-pyridinyl)propan-1-ol with 0.49 g of N,N-carbonyldiimidazole in refluxing tetrahydrofuran (25 ml) for 16 hours.

## Reference Example 102

(4S-trans)-4-(cyclohexylmethyl)-5-(2-thiazolyl)-2-oxazolidinone

A solution of 0.24 g of (S)-2-amino-3-cyclohexyl-(R)-1-(2-thiazolyl)propan-1-ol and 0.11 g of N,N-carbonyldiimidazole in 5 ml of tetrahydrofuran was stirred at room temperature for 16 hours and then refluxed for 5 hours. The solvent was removed and the residue dissolved in 15 ml of ethyl acetate. The solution was washed twice with 5 ml of 1N hydrochloric acid, with 5 ml of sodium bicarbonate, with 5 ml of brine and dried ($Na_2SO_4$). The solvent was removed and the residue washed with hexane to give 0.26 g of a colorless glass; $[\alpha]_D^{26}$ -88° ±1 (c = 1.072, methanol).

Reference Example 103

(4S-trans)-4-(Cyclohexylmethyl)-5-[1-[[2-(trimethyl-silyl)ethoxy]methyl]-1H-imidazol-2-yl]-2-oxazolidinone

To a solution of 0.35 g of (S)-2-amino-3-cyclohexylmethyl-(R)-1-(2-[N-[2-(trimethylsilyl)ethoxy]methyl]-imidazolyl)propan-1-ol in 2 ml of diethylcarbonate was added 0.41 g of potassium carbonate and 0.16 g of sodium methoxide. The suspension was heated at 80° C for 21 hours and at 100° C for 6 days. The mixture was quenched with 5 ml of water and extracted with 15 ml of ethyl acetate. The extract was washed with brine, dried ($Na_2SO_4$) and the solvent removed. The residue was chromatographed on silica gel (20 g). The column was eluted with hexane (200 ml) and then with ethyl acetate-hexane (1:1) to give 0.33 g of a colorless gum: $[\alpha]_D^{26}$ -138° ±1 (c = 1.042, methanol).

Reference Example 104

N-( L -Leucyl)-(S)-2-amino-3-cyclohexyl-(R)-1-(2-furanyl)propan-1-ol

To a solution of 1.4 g of imidazole in 18 ml of dichloromethane was added 0.90 ml of phenyl dichlorophosphate in 6 ml of dichloromethane. The mixture was stirred for 20 minutes, cooled to 0° C and a solution of 0.60 g of imidazole, 1.60 g of $N^{\alpha}$-[(benzyloxy)carbonyl]- L -leucine in 6 ml of tetrahydrofuran and 2.4 ml of N,N-dimethylformamide added. The mixture was stirred at 0° C for 40 minutes and then 1.30 g of (S)-2-amino-3-cyclohexyl-(R)-1-(2-furanyl)propan-1-ol added. The mixture was stirred at 0° C to 25° C (ice bath allowed to melt) overnight and the solvent removed. The residue was dissolved in 20 ml of ethyl acetate and washed with water, 2N citric acid, sodium bicarbonate solution and dried ($MgSO_4$). The solution was filtered through a thin pad of hydrous magnesium silicate and the pad washed with several volumes of ethyl acetate. The filtrate was concentrated under vacuum to give 2.3 g of N-[N-(benzyloxy)carbonyl-L -leucyl]-(S)-2-amino-3-cyclohexyl-(R)-1-(2-furanyl)propan-1-ol as an oil.

The preceding compound (1.85 g) and 1.0 g of ammonium formate in 24 ml of methanol under nitrogen was warmed on a steam bath and then the solution was chilled to 0° C. To the mixture (without stirring) was added (by pipette) 0.96 g of 10% palladium on carbon suspended in 5 ml of ethanol. The mixture was stirred at 0° C for one hour, diatomaceous earth added and the mixture filtered. The filter pad was washed with methanol and the filtrate evaporated to dryness. The residue was partitioned between ammonium hydroxide and dichloromethane. The organic layer was separated, dried ($MgSO_4$) and the solvent removed to give 1.24 g of a gum. Crystallization from 5 ml of diisopropyl ether gave 0.74 g of N-( L -leucyl)-(S)-2-amino-3-cyclohexyl-(R)-1-(2-furanyl)propan-1-ol as colorless crystals, mp 83-84° C.

Reference Example 105

N-( L -Histidyl)-(S)-2-amino-3-cyclohexyl-(R)-1-(2-furanyl)propan-1-ol

To a mixture of 1.25 g of imidazole in 0.5 ml of dichloromethane was added 1.2 g of phenyl dichlorophosphate in 5 ml of dichloromethane. After stirring for 25 minutes the mixture was chilled to 0 °C and to the mixture was added a warm solution of 1.65 g of $N^{\alpha}$-(benzyloxycarbonyl)- L -histidine and 0.55 g of imidazole in 2 ml of dry N,N-dimethylformamide. The mixture was diluted to a volume of 10 ml with dichloromethane and stirred at 0 °C for one hour. To the mixture was added 1.25 g of (S)-2-amino-3-cyclohexyl-(R)-1-(2-furanyl)propan-1-ol and the mixture stirred overnight at 0 °C to 25 °C (ice bath allowed to melt). The mixture was concentrated under vacuum and the residue in 20 ml of ethyl acetate washed with 5 ml of water, three 5 ml portions of 1M sodium bicarbonate and brine. The organic layer was dried ($Na_2SO_4$) and the solvent removed to give 2.4 g of N-[N-(benzyloxycarbonyl)- L -histidyl)-(S)-2-amino-3-cyclohexyl-(R)-1-(2-furanyl)propan-1-ol as a gum.

A mixture of the preceding gum (2.4 g), 1.52 g of ammonium formate, 0.37 ml of formic acid (90%) and 30 ml of methanol under nitrogen was chilled to 0 °C and then a slurry of 1.2 g of 10% palladium on carbon in ethanol was added by pipette. The cooled mixture was stirred 2.5 hours and filtered through diatomaceous earth and evaporated. To the residue was added one ml of concentrated ammonium hydroxide. The mixture was extracted successively with 10 ml, 5 ml and 5 ml portions of ethyl acetate. The combined extracts were dried ($Na_2SO_4$) and the solvent removed to give 1.2 g of zo a glass. This glass was chromatographed on a silica gel column with solvent dichloromethane-methanol-triethylamine (94:6:2). Cuts containing product were combined, concentrated to dryness and partitioned between 10 ml of 2N ammonium hydroxide and 5 ml of dichloromethane. The organic layer was separated and the aqueous layer extracted with two 5 ml portions of dichloromethane. The organic layer and extracts were combined, dried ($Na_2SO_4$) and the solvent removed to give 0.22 g of solid; Mass spectrum (FAB): calc (M + H), 361. Found (M + H), 361.

Reference Example 106

(S)-2-Amino-3-cyclohexyl-(R)-1-(2-thiazolyl)-1-[(tert-butyl)(dimethylsilyl)oxy]propane

To a stirred solution of 10.0 g of (S)-2-(tert-butoxycarbonyl)amino-3-cyclohexyl-(R)-1-(2-thiazolyl)-1-(tert-butyldimethylsilyloxy)propane in 50 ml of dichloromethane cooled to 0 °C under argon was added 16.9 ml of trifluoroacetic acid over 15 minutes. The solvent was stirred at room temperature for 4 hours, cooled to 0 °C and 120 ml of ice-cold 2N sodium hydroxide added slowly. To the mixture was added 100 ml of dichloromethane and the organic layer separated. The aqueous layer was extracted with two 100 ml portions of dichloromethane. The organic layer and extracts were combined, dried ($MgSO_4$) and the solvent removed under vacuum to give 7.5 g (96%) of crystals, mp 63-65 °C; $[\alpha]_D^{26}$ +19 ° ±1 (c = 1.062, methanol).

Reference Example 107

(S)-2-Amino-3-cyclohexyl-(R)-1-(1H-pyrazol-3-yl)-1-[[(1,1-dimethylethyl)dimethylsilyl]oxy]propane

A mixture of 7.00 g of 1,1-dimethylethyl-(S)-(2-cyclohexyl-1-formylethyl)carbamate, 4.97 g of t-butyldimethylsilyl chloride, 7.03 g of potassium cyanide and 0.150 g of zinc iodide in 150 ml of dry acetonitrile under argon was stirred vigorously at room temperature for 48 hours. The solvent was removed under vacuum and the residue triturated with ether, filtered and the solid washed with ether. The filtrate and wash were combined and washed with water and saturated sodium chloride solution. The organic layer was dried

($Na_2so_4$) and the solvent removed to give 10.7 g of a gum. This gum was chromatographed by HPLC on a Water-Prep 500 instrument on silica gel with 2.5% ethyl acetate in hexane as eluent. The first fractions collected were concentrated to dryness to give 3.27 g of 1,1-dimethylethyl (1S,2R)[2-cyano-1-(cyclohexylmethyl)-2-[[(1,1-dimethylethyl)dimethylsilyl]oxy]ethyl]carbamate as a gum; $[\alpha]_D^{26}$ -27° ±1 (c = 1.148, methanol). Concentration of the last fractions from the column gave 1,1-dimethylethyl (1S,2S)[2-cyano-1-(cyclohexylmethyl)-2-[[(1,1-dimethylethyl)dimethylsilyl]oxy]ethyl]carbamate as a gum; $[\alpha]_D^{26}$ -57° ±1 (c = 1.0267, methanol).

To 1.72 g of 1,1-dimethylethyl (1S,2R)[2-cyano-1-(cyclohexylmethyl)-2-[[(1,1-dimethylethyl)-dimethylsilyl]oxy]ethyl]carbamate in dried anhydrous ether under argon, cooled to 0° C, was added (via syringe) 3.74 ml of methylmagnesium bromide in ether (2.3M $CH_3MgBr$ in ether). The cooling bath was removed and the mixture stirred at room temperature for 24 hours. The mixture was quenched with saturated ammonium chloride solution, stirred 3 hours, and acidified to pH 4.0 with 1N citric acid solution. The mixture was stirred vigorously at room temperature for 6.5 hours, the organic layer separated, dried ($Na_2SO_4$) and the solvent removed. The residue (1.90 g of oil) was chromatographed on a 2.8cmx24cm silica gel column with ethyl acetate-hexane (1:9) as eluent (20 ml cuts). Cuts 5-7 were combined and the solvent removed to give 1.41 g of 1,1-dimethylethyl (1S,2R)[1-(cyclohexylmethyl)-2-[[(1,1-dimethylethyl)-dimethylsilyl]oxy]-3-oxobutyl]carbamate as a pale yellow gum; $[\alpha]_D^{26}$ -44° ±1 (c = 1.071, methanol); anal. calcl: C, 63.9; H, 10.5; N, 3.4. Found: C, 63.8; H, 10.4; N, 3.4.

A solution of the preceding compound (1.40 g) in 3 ml of tert-butoxy bis(dimethylamino)methane (Bredereck's Reagent) under argon was stirred at room temperature for 26.5 hours. The yellow solution was evaporated to dryness under a high vacuum to give a yellow gum. This gum was chromatographed on a silica gel column (2.8cmx21cm) with ethyl acetate-hexane (1:1) as eluent (30 ml cuts). Cuts 4-15 were combined, the solvent removed and the residue dried under high vacuum at 40° C to give 1.38 g of 1,1-dimethylethyl (1S,2R)[1-(cyclohexylmethyl)-5-dimethylamino)-2-[[(1,1-dimethylethyl)dimethylsilyl]oxy]-3-oxo-4-pentenyl]carbamate as a thick syrup; $[\alpha]_D^{26}$ +3° ±1 (c = 1.375, methanol); anal calcl: C, 64.1; H, 10.3; N, 6.0; Si, 6.0. Found: C, 63.8; H, 10.2; N, 5.7; Si, 5.8.

To the preceding compound (1.38 g) in 11 ml of ethanol was added 0.162 g of hydrazine hydrate and 0.342 ml of acetic acid. The solution was stirred under argon at room temperature for 20 hours and the solvent removed under vacuum to give 1.29 g of (S)-2-(tert-butoxycarbonyl)amino-3-cyclohexyl-(R)-1-(1H-pyrazol-3-yl)-1-[[(1,1-dimethylethyl)dimethylsilyl]oxy]propane as a gum. Crystallization from hexane gave white crystals, mp 136-138° C.

The preceding compound (1.2 g) in 10 ml of dichloromethane and 2.3 ml of trifluoroacetic acid was stirred at room temperature for 4 hours. The solution was washed with 16.6 ml of 2N sodium hydroxide and diluted with 15 ml of dichloromethane. The organic layer was separated and the aqueous layer extracted with two 15 ml portions of dichloromethane. The organic layer and extracts were combined, dried ($Na_2SO_4$) and the solvent removed. The residue was crystallized from hexane to give 0.70 g of crystals (in two crops). Chromatography on silica gel with ethyl acetate as eluent gave a solid which was crystallized from hexane to give 0.379 g of (S)-2-amino-3-cyclohexyl-(R)-1-(1H-pyrazol-3-yl)-1-[[(1,1-dimethylethyl)dimethylsilyl]oxy]-propane as white crystals, mp 125-127° C; $[\alpha]_D^{26}$ +22° ±1 (c = 1.081, methanol).

## Reference Example 108

## N-Diethoxyphosphinyl)- L -phenylalanine

A solution of 16.5 g of L -phenylalanine and 50ml of triethylamine in 30 ml of water and 20 ml of ethanol under argon was chilled to 0° C. To this stirred solution was added dropwise a solution of diethyl phosphite (13.8 g) in 40 ml of carbon tetrachloride. After the addition, the mixture was stirred at room temperature for 3 hours and acidified with 3N hydrochloric acid (pH 2). The mixture was extracted with three 200 ml portions of ethyl acetate, the extract dried ($Na_2SO_4$) and the solvent removed under vacuum. The residue was dried under vacuum to give 23.5 g of an orange gum; $[\alpha]_D^{26}$ +11° ±1 (c = 1.056, chloroform).

## Reference Example 109

N-(Dibenzyloxyphosphinyl)- L -phenylalanine

A solution of 8.2 g of L -phenylalanine and 25 ml of triethylamine in 15 ml of water and 10 ml of ethanol under argon was chilled to 0° C. To this stirred solution was added dropwise 13.1 g of dibenzyl phosphite in 20 ml of carbon tetrachloride. After the addition, the mixture was stirred at room temperature for 3 hours and the mixture was concentrated under vacuum, diluted with ethyl acetate (100 ml), cooled to 0° C and 50 ml of 3N hydrochloric acid added dropwise. The organic layer was separated and the aqueous layer extracted twice with 100 ml of ethyl acetate. The organic layer and extracts were combined, dried ($Na_2SO_4$) and the solvent removed. The residue was dried under vacuum to give 20.7 g of yellow gum.

Reference Example 110

N-[N-(Dibenzyloxyphosphinyl)- L -phenylalanyl]- L -leucine

To a solution of 8.5 g of imidazole in 55 ml of dichloromethane under argon was added slowly a solution of 5.3 g of phenyl dichlorophosphate in 15 ml of dichloromethane. The suspension was stirred at room temperature for 0.5 hour, cooled to 0° C and a solution of 10.62 g of N-(dibenzyloxyphosphinyl)- L -phenylalanine in 15 ml of dichloromethane added slowly. After stirring at 0° C for one hour L -leucine methyl ester hydrochloride (4.5 g) was added in small portions. The mixture was stirred at 0° C for 5 hours and allowed to warm to room temperature. The mixture was washed with 1N hydrochloric acid and the aqueous layer extracted with dichloromethane. The organic layer and extract were combined, washed with 0.5N hydrochloric acid, water and saturated sodium bicarbonate solution and dried ($Na_2SO_4$). The solvent was removed to give 11.2 g of a gum. Chromatography on silica gel with solvent ethyl acetate-hexane (1:1) followed by solvent ethyl acetate:hexane (2:1) gave 4.54 g of solid. Trituration with hexane (40 ml) gave 4.2 g of N-[N-(dibenzyloxyphosphinyl)- L -phenylalanyl]- L -leucine, methyl ester as crystals , mp 90-92° C; $[\alpha]_D^{26}$ -17° ±1 (c = 1.059, methanol).

To the preceding compound (4.0 g) in 50 ml of methanol was added dropwise 14 ml of 1N sodium hydroxide and the mixture stirred 5 hours. An additional 7 ml of 1N sodium hydroxide was added and the mixture stirred at room temperature for 2.5 hours, chilled 2 days at 4° C and concentrated under vacuum. The aqueous residue was extracted with ether (3 times with 20 ml), and the aqueous layer acidified with 2N hydrochloric acid and extracted with two 50 ml portions of dichloromethane. The extract was dried ($Na_2SO_4$) and the solvent removed to give 3.2 g of a white solid. Trituration with hexane gave 2.7 g of white crystals, mp 50-53° C; $[\alpha]_D^{26}$ -11±1 (c = 1.084, methanol).

Reference Example 111

N-[N-[(Phenylmethoxy)carbonyl]- L -phenylalanyl- L -2-(1-methylethoxy)glycyl]-(S)-2-amino-3-cyclohexyl-(R)-1-(2-thiazolyl)propan-1-ol

To a solution of N,N-carbonyldiimidazole (0.12 g) in 3 ml of dichloromethane under argon was added a solution of N-[(phenylmethoxy)carbonyl- L -phenylalanyl- D , L -2-(1-methylethoxy)glycine (0.31 g) in one ml of tetrahydrofuran and the mixture stirred one hour. To this solution was added 0.18 g of (S)-2-amino-3-cyclohexyl-(R)-1-(2-thiazolyl)-1-(tert-butyldimethylsilyloxy)propane in one ml of tetrahydrofuran. The mixture was stirred at room temperature for 2 hours, diluted with 3 ml of water and the solvent removed. The residue was diluted with 20 ml of ethyl acetate and 5 ml of 1N hydrochloric acid. The organic layer was separated, washed with 10 ml of 0.5N hydrochloric acid, 10 ml of saturated sodium bicarbonate, brine and

dried ($Na_2SO_4$). The solution was diluted with 10 ml of hexane and filtered through a thin pad of hydrous magnesium silicate. The pad was washed with 10 ml of ethyl acetate-hexane (1:1) and the filtrate concentrated to dryness to give 0.4 g of a foamy solid. This solid (0.22 g) in one ml of tetrahydrofuran and 0.35 ml of tetra(n-butyl)ammonium fluoride (1.0M in tetrahydrofuran) was stirred at room temperature for one hour, diluted with 2 ml of water and the solvent was removed. The residue was extracted with 10 ml of ethyl acetate and the extract washed with 3 ml of 0.5N hydrochloric acid, 3 ml of saturated sodium bicarbonate, brine and dried ($Na_2SO_4$). The solvent was removed and the residue chromatographed on silica gel by gradient elution with ethyl acetate-hexane (2:3) to ethyl acetate-hexane (1:1). Fractions containing the faster moving component were combined and the solvent removed to give 0.10 g of the product of the Example as a white solid; $[\alpha]_D^{26}$ -37° ±1 (c = 0.660, chloroform). Fractions containing the slower moving component gave 0.10 g of N-[N-[(phenylmethoxy)carbonyl- L -phenylalanyl- D -2-(1-methylethoxy)glycyl]-(S)-2-amino-3-cyclohexyl-(R)-1-(2-thiazolyl)propan-1-ol as a white solid; $[\alpha]_D^{26}$ +6° ±2 (c = 0.639, chloroform).

## Reference Example 112

### N-[N-[(Phenylmethoxy)carbonyl]- L -phenylalanyl- L -2-(isopropylthio)glycyl]-(S)-2-amino-4-methyl-(R)-1-(2-thiazolyl)pentan-1-ol

A mixture of 0.5 g of N-[(phenylmethoxy)carbonyl]- L -phenylalanyl- L -2-hydroxyglycine, 0.624 ml of 2-propanethiol, 0.150 ml of concentrated sulfuric acid in 5 ml of glacial acetic acid was stirred under argon at room temperature for 3 days. The mixture was poured into ice water and extracted with ethyl acetate. The combined extracts were washed with water and the solvent removed. The residue was dissolved in ethyl acetate, dried ($MgSO_4$) and the solvent removed. The residue was dried under high vacuum over solid potassium hydroxide to give a viscous oil. The oil was dissolved in ethyl acetate and the solvent removed (repeated several times). Drying under high vacuum gave 0.46 g of N-[(phenylmethoxy)carbonyl]- L -phenylalanyl-2-(isopropylthio)glycine as a foam: Mass spectrum (FAB); calc (M+H), 431: Found (M+H), 431.

To the preceding compound (0.150 g) in 5 ml of dichloromethane under argon was added 86.3 mg of N-ethoxycarbonyl-2-ethoxy-1,2-dihydroquinoline (EEDQ). After stirring 15 minutes, 69.8 mg of (S)-2-amino-4-methyl-(R)-1-(2-thiazolyl)pentan-1-ol in 1.3 ml of dichloromethane was added and the mixture was stirred overnight at room temperature. The mixture was washed with 3N hydrochloric acid, 10% sodium bicarbonate, brine and dried ($MgSO_4$). The solvent was removed to give 0.180 g of a foam. Chromatography on silica gel of a 0.49 g sample with ethyl acetate-dichloromethane (2:3) separated the two diastereomers. Fractions containing the faster moving compound were combined and the solvent removed. Drying under vacuum gave 0.160 g of the product of Example as a clear foam; $[\alpha]_D^{26}$ -5° ±1 (c = 1.097, methanol), and 0.10 g of N-[N-[(phenylmethoxy)carbonyl]- L -phenylalanyl- D -2-(isopropylthio)glycyl]-(S)-2-amino-4-methyl-(R)-1-(2-thiazolyl)pentan-1-ol as a white solid; $[\alpha]_D^{26}$ -34° ±1 (c = 1.00, methanol).

## Reference Example 113

### N-[N-[(Phenylmethoxy)carbonyl]- L -2-(1-methylethoxy)glycyl]-(S)-2-amino-3-cyclohexyl-(R)-1-(2-pyridinyl)-propan-1-ol

A mixture of 0.30 g of N-[(phenylmethoxy)carbonyl- L -phenylalanyl- D , L -2-(1-methylethoxy)glycine, 0.179 g of N-ethoxycarbonyl-2-ethoxy-1,2-dihydroquin oline (EEDQ) in 10 ml of dichloromethane was stirred for 30 minutes. To the solution was added 0.169 g of (S)-2-amino-3-cyclohexyl-(R)-1-(2-pyridinyl)propan-1-ol in 2 ml of dichloromethane and the mixture stirred overnight. The solution was washed with 10% sodium bicarbonate, brine, dried ($MgSO_4$) and the solvent removed to give 0.520 g of solid. A 1.40 g sample was chromatographed on silica gel on a Waters-Prep 500 HPLC apparatus with solvent ethyl acetate-dichloromethane (2:3) as eluent to give 0.10 g of the product of the Example as a white amorphous solid; $[\alpha]_D^{26}$ -27° ±1 (c = 1.058, methanol) and 0.093 g of N-[N-[(phenylmethoxy)carbonyl]- L -phenylalanyl- D -2-(1-methylethoxy)glycyl]-(S)-2-amino-3-cyclohexyl-(R)-1-(2-pyridinyl)propan-1-ol as a white solid; $[\alpha]_D^{26}$ +20±1 (c = 1.05, methanol).

## Reference Example 114

### N-[N-[(Phenylmethoxy)carbonyl]- L -phenylalanyl- L -2-(isopropylthio)glycyl]-(S)-2-amino-3-cyclohexyl-(R)-1-(2-pyridinyl)propan-1-ol

To a solution of N-[(phenylmethoxy)carbonyl]- L -phenylalanyl- D , L -2-(isopropylthio)glycine in 15 ml of dry tetrahydrofuran under argon was added 0.219 g of N,N-carbonyldiimidazole and the mixture stirred at room temperature for 2.5 hours. To this solution was added 0.254 g of (S)-2-amino-3-cyclohexyl-(R)-1-(2-pyridinyl)propan-1-ol and the mixture was stirred at room temperature overnight. The solvent was removed and the residue dissolved in dichloromethane and the solution washed with 10% sodium bicarbonate and brine. The aqueous layer was extracted with dichloromethane and the organic layer and extracts combined, dried ($Na_2SO_4$) and the solvent removed. The residue (0.83 g) was chromatographed on silica gel with 45% ethyl acetate in dichloromethane as solvent. Fractions containing the fast moving component were combined to give 0.26 g of the product of the Example as a solid foam; $[\alpha]_D^{26}$ 18° ±1 (c = 0.868, methanol), and 0.19 g of N-[N-[(phenylmethoxy)carbonyl]- L -phenylalanyl- D -2-(isopropylthio)glycyl]-(S)-2-amino-3-cyclohexyl-(R)-1-(2-pyridinyl)propan-1-ol as solid yellow foam; $[\alpha]_D^{26}$ -8° ±1 (c = 1.205, methanol).

## Reference Example 115

### 1,1-Dimethylethyl[(S)-1-(cyclohexylmethyl-(R,S)-2-[[(1,1-dimethylethyl)dimethylsilyl]oxy]-2-(2-methyl-4-pyrimidinyl)ethyl]carbamate

To a suspension of 0.050 g of acetamidine acetate in 2 ml of ethanol was added 9.6 mg of sodium. After the sodium had reacted, 0.100 g of 1,1-dimethylethyl(1S,2R,S)[1-(cyclohexylmethyl)-5-(dimethylamino)-2-[[(1,1-dimethylethyl)dimethylsilyl]oxy]-3-oxo-4-pentenyl]carbamate was added and the mixture refluxed overnight. The solvent was removed and the residue chromatographed on thick layer silica gel plate with ethyl acetate-hexane (1:1) as solvent to give 4 mg of product as a gum: Mass spectrum (chemical ionization): $MH^+$ = 464.

## Reference Example 116

### Ethyl (4S-trans)-5-[4-cyclohexylmethyl)-2-oxo-5-oxazolidinyl]-4-oxazolecarboxylate

To a solution of 1.0 g of (4S-trans-4-(cyclohexylmethyl)-5-(2-furanyl)-2-oxazolidinone in 24 ml of acetonitrile and 24 ml of carbon tetrachloride was added a solution of 8.56 g of sodium metaperiodate in 60 ml of water and then 40 mg of ruthenium dioxide monohydrate. The mixture was stirred vigorously at room temperature for 3 hours, filtered through diatomaceous earth and the filter pad washed with 80 ml of dichloromethane. The organic layer of the filtrate was separated and the aqueous layer extracted with 80 ml of dichloromethane. The organic layer and extract were combined, dried ($Na_2SO_4$) and the solvent removed. The residue was dissolved in ethyl acetate (30 ml) and extracted with two 15 ml portions of saturated sodium bicarbonate solution. The basic aqueous extract was acidified with 6N hydrochloric acid

and extracted with two 50 ml portions of ethyl acetate. The ethyl acetate extract was dried ($MgSO_4$), filtered through diatomaceous earth and the filtrate evaporated to give 0.91 g of (4S-trans)-4-(cyclohexylmethyl)-2-oxo-5-oxazolidinecarboxylic acid as foamy solid.

The preceding compound (0.11 g) in 0.5 ml of tetrahydrofuran was added to a solution of N,N-carbonyldiimidazole (89 mg) in one ml of tetrhydrofuran and the solution stirred at room temperature for one hour. To the solution was added ethyl isocyanoacetate (71 mg) and 1,8-diazabicyclo[5.4.0]undec-7-ene and the mixture stirred at room temperature for 22 hours. The solvent was removed and the residue dissolved in 10 ml of ethyl acetate. This solution was washed with 5 ml each of 1N hydrochloric acid, water, saturated sodium bicarbonate and brine, dried ($NaSO_4$) and the solvent removed. The residue was triturated with hexane to give 60 mg of the product of the Example as a gummy solid; $[\alpha]_D^{26}$ -49° ±2 (c = 0.409, chloroform).

Reference Example 117

(S)-2-Amino-3-cyclohexyl-(R,S)-1-(5-isoxazolyl)-1-[[(1,1-dimethylethyl)dimethylsilyl]oxo]propane

A mixture of 1,1-dimethylethyl(1S,2R,S)[1-(cyclohexylmethyl)-5-(dimethylamino)-2-[[(1,1-dimethylethyl)-dimethylsilyl]oxy]-3-oxo-4-pentenyl]carbamate (0.10 g) and 14 mg of hydroxylamine hydrochloride in 2.5 ml of ethanol was refluxed for one hour and the solvent removed. The residue was chromatographed on a thick layer silica gel plate with hexane-ethyl acetate (85:15) as solvent to give a gum. This gum was dissolved in 2 ml of dry ethanol and 15 µL of hydrogen chloride in dioxane (4N) added. The mixture was refluxed for 4 hours under argon and the solvent removed. The residue was dissolved in dichloromethane and the solution washed with saturated sodium bicarbon ate solution. The organic layer was dried ($Na_2SO_4$) and the solvent removed to give the product of the Example as a gum; Mass spectrum ($MH^+$) 339.

Reference Example 118

(4S-trans)-4-(Cyclohexylmethyl)-5-(3-pyridazinyl-2-oxazolidinone

To a stirred solution under argon of 0.50 g of (4S-trans)-4-(cyclohexylmethyl)-5-(2-furanyl)-2-oxazolidinone in 25 ml of methanol containing 0.425 g of sodium carbonate chilled to -50° C was added dropwise (over 30 minutes) 0.11 ml of bromine in 10 ml of methanol. After the addition, the solution was allowed to warm to room temperature (30 minutes) and the solution was filtered and the filtrate concentrated to dryness to give (4S-trans)-4-(cyclohexylmethyl)-5-(2,5-dimethoxy-2-furanyl)-2-oxazolidinone as a gum (0.48 g).

The preceding compound (0.48 g) was suspended in 3 ml of methanol and 10 ml of 1% aqueous acetic acid added. The mixture was refluxed for 10 minutes, chilled to room temperature and one ml of hydrazine hydrate added. The mixture was refluxed for one hour, cooled, neutralized with sodium bicarbonate and extracted with ethyl acetate. The organic layer was washed with water, brine, dried ($Na_2SO_4$) and the solvent removed to give a gum (0.35 g). Chromatography on silica gel with solvent ethyl acetate-hexane (3:1) gave 0.110 g of the product of the Example as an off-white foam (hygroscopic): IR; 2950, 2850, 1760, 1580 $cm^{-1}$; Mass Spectrum (CI); ($MH^+$) 262; ($M+NH_4^+$), 279.

As described in Reference Example 64, the compounds of Reference Examples 11, 21 and 27 may be reacted with 2-trimethylsilylthiazole to give the following Reference Compounds:

N-[N-[(Phenylmethoxy)carbonyl]- L -phenylalanyl- L -2-(2-methylpropoxy)glycyl]-(S)-2-amino-4-methyl-(R)-1-(2-thiazolyl)pentan-1-ol

N-[N-[(Phenylmethoxy)carbonyl]- L -phenylalanyl- L -2-(1-methylethoxy)glycyl]-(S)-2-amino-4-methyl-(R)-1-(2-thiazolyl)pentan-1-ol

N-[N-[(Phenylmethoxy)carbonyl]- L -phenylalanyl- L -2-(phenylmethoxy)glycyl]-(S)-2-amino-4-methyl-(R)-1-(2-thiazolyl)pentan-1-ol.

The following examples illustrate the preparation of compounds of our invention.

## Example 1

### N-[N-(Diethoxyphosphinyl)-L-phenylalanyl-L-leucyl]-(S)-2-amino-4-methyl-(R)-1-(2-thiazolyl)pentan-1-ol

To a solution of 46 mg of N-(L-phenylalanyl-L-leucyl)-(S)-2-amino-4-methyl-(R)-1-(2-thiazolyl)pentan-1-ol in one ml of dichloromethane under argon was added 12 mg of dry triethylamine and 20 mg of diethyl phosphorochloridate (diethyl chlorophosphate). The mixture was stirred at room temperature for 18 hours and then diluted with 10 ml of ethyl acetate. The mixture was washed with 2 ml of 1N hydrochloric acid, 2 ml of saturated sodium bicarbonate solution and 2 ml of saturated sodium chloride solution. The organic layer was dried and the solvent removed under vacuum to give a solid which was washed with hexane. The product was obtained as a white solid, $[\alpha]_D^{26} = -43^\circ$ (c = 0.328; methanol).

## Example 2

### N-[N-(Diphenoxyphosphinyl)-L-phenylalanyl-L-leucyl]-(S)-2-amino-4-methyl-(R)-1-(2-thiazolyl)pentan-1-ol

To a solution of 69 mg of N-(L-phenylalanyl-L-leucyl)-(S)-2-amino-4-methyl-(R)-1-(2-thiazolyl)pentan-1-ol in 2 ml of dichloromethane under argon was added 18 mg of dry triethylamine and 45 mg of diphenyl phosphorochloridate (diphenyl chlorophosphate). The mixture was stirred at room temperature for 2 days (white precipitate formed). The mixture was filtered and the solid washed with water and methanol. The solid was dried under vacuum to give 90 mg of product as a white solid; $[\alpha]_D^{26}$ -28° (c = 0.49, dimethylsulfoxide).

## Example 3

### N-[N-[[Methoxy][phenoxy]phosphinyl]-L-phenylalanyl-L-leucyl]-(S)-2-amino-4-methyl-(R)-1-(2-thiazolyl)-pentan-1-ol

To a solution of 92 mg of N-(L-phenylalanyl-L-leucyl)-(S)-2-amino-4-methyl-(R)-1-(2-thiazolyl)pentan-1-ol in 2 ml of dichloromethane was added 45 mg of phosphorochloridic acid, methyl phenyl ester. The mixture was stirred under argon at room temperature for 27 hours. The mixture was diluted with 10 ml of ethyl acetate and washed with 1N hydrochloric acid (2x3 ml), saturated sodium bicarbonate (3 ml) and dried. The solution was filtered through a thin pad of hydrous magnesium silicate and the pad washed with ethyl acetate (2x2 ml). The filtrate was concentrated under vacuum to give 0.11 g of white solid; $[\alpha]_D^{26}$ -40° ±2 (c = 0.622, methanol).

## Example 4

### N-[N-(Dibutoxyphosphinyl)-L-phenylalanyl-L-leucyl]-(S)-2-amino-4-methyl-(R)-1-(2-thiazolyl)-pentan-1-ol

To solution of 46 mg of N-( L -phenylalanyl- L -leucyl)-(S)-2-amino-4-methyl-(R)-1-(2-thiazolyl)pentan-1-ol in one ml of dichloromethane under argon is added 12 mg of dry triethylamine and 0.11 mmole of dibutyl phosphorochloridate . The mixture is stirred at room temperature for 24 hours and then diluted with 10 ml of ethyl acetate. The mixture is washed with 2 ml portions of 1N hydrochloric acid, saturated sodium bicarbonate and saturated sodium chloride. The organic layer is dried, the solvent removed under vacuum and the residue triturated with hexane to give the product as a white solid.

## Example 5

### N-[N-(Diethoxyphosphinyl)- L -phenylalanyl- L -leucyl]-(S)-2-amino-3-cyclohexyl-(R)-1-(2-thiazolyl)propan-1-ol

To a solution of 0.31 g of N-( L -phenylalanyl- L -leucyl)-(S)-2-amino-3-cyclohexyl-(R)-1-(2-thiazolyl)-1-[(t-butyl)(dimethyl)silyloxy]propane in 5 ml of dichloromethane was added 0.07 g of triethylamine and 0.11 g of diethyl phosphorochloridate. This mixture was stirred for 28 hours and then diluted with 30 ml of ethyl acetate. The mixture was washed with two 6 ml portions of 0.5N hydrochloric acid, two 10 ml portions of 10% potassium carbonate and dried. The solution was filtered through a thin pad of hydrous magnesium silicate and the filter cake washed with ethyl acetate. The filtrate and wash were combined and concentrated in vacuo, giving 0.4 g of a white foamy solid; $[\alpha]_D^{26}$ -40° ±4 (c = 0.272, methanol).

To a 0.3 g portion of the above solid in one ml of tetrahydrofuran was added 0.44 ml of 1M tetra-n-butylammonium fluoride in tetrahydrofuran. This solution was stirred for 5 hours and then diluted with 10 ml of 10% sodium bicarbonate solution and 20 ml of ethyl acetate. The organic layer was separated, washed with 5 ml each of 0.5N hydrochloric acid, saturated sodium bicarbonate solution and saturated sodium chloride solution, dried and filtered through a thin pad of hydrous magnesium silicate. The pad was washed with ethyl acetate and the combined filtrate and wash concentrated in vacuo, giving a solid. This solid was triturated with hexane, giving 0.26 g of the desired product as a white solid; $[\alpha]_D^{26}$ -50° ±2 (c = 0.590, methanol).

## Example 6

### N-[N-([Methoxy][phenylmethoxy]phosphinyl)- L -phenylalanyl- L -leucyl]-(S)-2-amino-3-cyclohexyl-(R)-1-(2-thiazolyl)propan-1-ol

To a solution of 0.12 g of N-( L -phenylalanyl- L -leucyl)-(S)-2-amino-3-cyclohexyl-(R)-1-(2-thiazolyl)-1-[(t-butyl)(dimethyl)silyloxy]propane in 2 ml of dichloromethane was added 88 mg of phosphorochloridic acid, methyl phenylmethyl ester and 32 mg of 2,6-dimethylpyridine. This solution was stirred under argon for 2 hours and 0.22 g of phosphorochloridic acid, methyl phenylmethyl ester added. After stirring 2 hours the mixture was worked up as described in Example 5, giving 0.16 g of foamy white solid.

The above procedure was repeated, using 0.24 g of N-( L -phenylalanyl- L -leucyl)-(S)-2-amino-3-cyclohexyl-(R)-1-(2-thiazolyl)-1-[(t-butyl)(dimethyl)silyloxy]propane, 0.1 g of triethylamine and 0.22 g of phosphorochloridic acid, methyl phenylmethyl ester, giving 0.27 g of foamy white solid.

The above two solids were combined and chromatographed on silica gel with ethyl acetate:hexane (1:1), giving 0.18 g of N-[N-(R)[[methoxy][phenylmethoxy]phosphinyl]- L -phenylalanyl- L -leucyl]-(S)-2-amino-3- cyclohexyl-(R)-1-(2-thiazolyl)-1-[(t-butyl)(dimethyl)silyloxy]propane and 0.18 g of N-[N-(S)[[methoxy][phenylmethoxy]phosphinyl]- L -phenylalanyl- L -leucyl]-(S)-2-amino-3-cyclohexyl-(R)-1-(2-thiazolyl)-1-[(t-butyl)(dimethyl)silyloxy]propane as foamy white solids.

Each of the above diastereomers was treated separately but identically as follows: An 80 mg portion was dissolved in 0.5 ml of tetrahydrofuran, the solution cooled to 0° C, and 0.2 ml of 1M tetra-n-butylammonium fluoride in tetrahydrofuran added. After stirring for 1.5 hours each mixture was worked up as described in Example 5, giving 50 mg of N-[N-(R)[[methoxy][phenylmethoxy]phosphinyl]- L -phenylalanyl- L -leucyl]-(S)-2-amino-3-cyclohexyl-(R)-1-(2-thiazolyl)propan-1-ol as a white solid; $[\alpha]_D^{26}$ -

45° ±2 (c = 0.48, methanol); and 50 mg of N-[N-(S)[[methoxy][phenylmethoxy]phosphinyl]-L-phenylalanyl-L-leucyl]-(S)-2-amino-3-cyclohexyl-(R)-1-(2-thiazolyl)propan-1-ol as a white solid; $[\alpha]_D^{26}$ -50° ±4 (c = 0.253, methanol).

## Example 7

### N-[N-([Hydroxy][phenoxy]phosphinyl)-L-phenylalanyl-L-leucyl]-(S)-2-amino-3-cyclohexyl-(R)-1-(2-thiazolyl)propan-1-ol

As described for Example 6, a mixture of 0.31 g of N-(L-phenylalanyl-L-leucyl]-(S)-2-amino-3-cyclohexyl-(R)-1-(2-thiazolyl)-1-[(t-butyl)(dimethyl)silyloxy]propane in 2 ml of dichloromethane and 61 mg of triethylamine was reacted with 0.12 g of phosphorochloridic acid, methyl phenyl ester, giving 0.41 g of N-[N-([methoxy][phenoxy]phosphinyl)-L-phenylalanyl-L-leucyl]-(S)-2-amino-3-cyclohexyl-(R)-1-(2-thiazolyl)-1-[(t-butyl)(dimethyl)silyloxy]propane as a foamy white solid; $[\alpha]_D^{26}$ -40° ±2 (c = 0.429, methanol).

A 0.31 g portion of the above solid was dissolved in 0.5 ml of tetrahydrofuran and 0.42 ml of a 1M solution of tetra-n-butylammonium fluoride in tetra hydrofuran was added. The mixture was stirred for 6 hours and then diluted with 20 ml of ethyl acetate and 5 ml of 10% sodium bicarbonate. The organic layer was washed with 5 ml each of 0.5N hydrochloric acid, saturated sodium bicarbonate and saturated sodium chloride. The organic layer was dried and the solvent removed in vacuo. The residue was stirred with 10 ml of dry diethyl ether for 0.5 hour and then filtered, giving 0.22 g of the desired product as a white solid; $[\alpha]_D^{26}$ -66° ±1 (c = 1.159, methanol).

## Example 8

### N-[N-[[Methoxy][phenylmethoxy]phosphinyl]-L-phenylalanyl-L-leucyl]-(S)-2-amino-4-methyl-(R)-1-(2-thienyl)pentan-1-ol

A 0.81 g portion of (S)-2-tert-butoxycarbonylamino-4-methyl(R,S)-1-(2-thienyl)pentan-1-ol (Reference Example 55) was dissolved in 5 ml of dichloromethane and 2.1 ml of trifluoroacetic acid added. This mixture was stirred for 3 hours and then poured into 15 ml of ice-cold 2N sodium hydroxide with stirring. The mixture was diluted with 25 ml of dichloromethane, the organic layer separated and the aqueous layer extracted with 20 ml of dichloromethane. The organic layer and extract were combined, washed with saturated sodium chloride solution, dried and the solvent removed in vacuo, giving 1.06 g of a white solid. A 0.23 g portion of this solid was dissolved in 5 ml of ethanol and 5ml of 1N sodium hydroxide added. This solution was refluxed for 16 hours and then concentrated in vacuo. The residue was extracted with two 10 ml portions of dichloromethane. The extracts were combined, dried and the solvent removed in vacuo, giving 0.20 g of (S)-2-amino-4-methyl-(R)-1-(2-thienyl)pentan-1-ol as a white solid.

A 0.1 g portion of the above solid was coupled with N-(tert-butoxycarbonyl)-L-phenylalanyl-L-leucine as described in Reference Example 43, giving 0.31 g of solid. This solid was dissolved in 2 ml of methanol and 1.0 ml of 1N sodium hydroxide added. After stirring for one hour, the mixture was diluted with 2 ml of water and the methanol removed in vacuo. The aqueous suspension was extracted with ethyl acetate. The extract was washed with saturated sodium chloride solution, dried and filtered through a thin pad of hydrous magnesium silicate. The pad was washed with ethyl acetate and the filtrate and wash combined. The solvent was removed and the residue triturated with hexane, giving 0.27 g of N-[N-(t-butoxycarbonyl)-L-phenylalanyl-L-leucyl]-(S)-2-amino-4-methyl-(R)-1-(2-thienyl)pentan-1-ol as a white solid.

This solid was stirred in a mixture of 2 ml of dichloromethane and one ml of trifluoroacetic acid for 3 hours. The mixture was diluted with 10 ml of dichloromethane , washed with 1N sodium carbonate, dried and the solvent removed in vacuo. The residue was reacted with phosphorochloridic acid, methyl phenylmethyl ester as described in Example 6, giving the desired product as a foamy white solid.

Example 9

N-[N-(Diethoxyphosphinyl)-L-prolyl-L-phenylalanyl-L-leucyl]-(S)-2-amino-4-methyl-(R)-1-(2-thiazolyl)pentan-1-ol

N-[N-(tert-Butoxycarbonyl)-L-prolyl-L-phenylalanyl-L-leucyl]-(S)-2-amino-4-methyl-(R)-1-(2-thiazolyl)pentan-1-ol (Reference Example 48) is deblocked with trifluoroacetic acid:dichloromethane (1:1) at room temperature and the mixture concentrated in vacuo. The residue is reacted with diethyl phosphorochloridate in dichloromethane, in the presence of triethylamine as described in Example 1, to give the desired product as a solid.

Example 10

N-[N-(Diphenoxyphosphinyl-L-phenylalanyl-L-2-(2-methylpropoxy)glycyl]-(1R,2S)-2-amino-3-cyclohexyl-1-(2-thiazolyl)propan-1-ol

N-[(Phenylmethoxy)carbonyl]-L-phenylalanyl-L-2-(2-methylpropoxy)glycyl-(1R,2S)-2-amino-3-cyclohexyl-1-(2-thiazolyl)propan-1-ol is deblocked with anhydrous hydrogen bromide in glacial acetic acid at room temperature and the mixture concentrated in vacuo. The residue is reacted with diphenyl phosphorochloridate in dichloromethane in the presence of triethylamine as described in Example 2, to give the desired product as a solid.

Example 11

N-[N-(Diethoxyphosphinyl)-L-phenylalanyl-L-leucyl]-(S)-2-amino-4-methyl-(R)-1-(1-methyl-1H-1,2,4-triazol-5-yl)pentan-1-ol

N-[N-(Benzyloxycarbonyl)-L-phenylalanyl-L-leucyl]-(S)-2-amino-4-methyl-(R)-1-(1-methyl-1H-1,2,4-triazol-5-yl)pentan-1-ol is deblocked with anhydrous hydrogen bromide in glacial acetic acid at room temperature and the mixture concentrated in vacuo. The residue is reacted with diethyl phosphorochloridate in dichloromethane at room temperature in the presence of triethylamine, as described in Example 1, to give the desired product as a solid.

Example 12

N-[N-(Diethoxyphosphinyl)-L-phenylalanyl-L-2-(2-methylpropoxy)glycyl]-(S)-2-amino-4-methyl-(R)-1-(1-methyl-1H-1,2,4-triazol-5-yl)pentan-1-ol

N[N-(Benzyloxycarbonyl)-L-phenylalanyl-L-2-(2-methylpropoxy)glycyl]-(S)-2-amino-4-methyl-(R)-1-(1-methyl-1H-1,2,4-triazol-5-yl)pentan-1-ol is deblocked by stirring with palladium on carbon in ethanol under a hydrogen atmosphere. The mixture is filtered under nitrogen to remove the catalyst and the filtrate concentrated in vacuo. The residue is reacted with diethyl phosphorochloridate in dichloromethane at room temperature in the presence of triethylamine as described in Example 1 to give the desired product as a

solid.

## Example 13

### N-[N-(Diethoxyphosphinyl)- L -phenylalanyl- L -2-(2-methylpropoxy)glycyl]-(S)-2-amino-3-phenyl-(R)-1-(2-thiazolyl)propan-1-ol

N-[N-(Benzyloxycarbonyl)- L -phenylalanyl- L -2-(2-methylpropoxy)glycyl]-(S)-2-amino-3-phenyl-(R)-1-(2-thiazolyl)propan-1-ol (Reference Example 63) is deblocked by stirring in ethanol under a hydrogen atmosphere with palladium on carbon. This mixture is filtered under nitrogen and the filtrate concentrated in vacuo. The residue is reacted with diethyl phosphorochloridate in dichloromethane in the presence of triethylamine as described in Example 1, giving the desired product as a solid.

## Example 14

### N-[N-(Diethoxyphosphinyl)- L -phenylalanyl- L -2-(cyclohexyloxy)glycyl]-(S)-2-amino-4-methyl-(R)-1-(2-thiazolyl)pentan-1-ol

N-[N-(Benzyloxycarbonyl)- L -phenylalanyl- L -2-(cyclohexyl)glycyl]-(S)-2-amino-4-methyl-(R)-1-(2-thiazolyl)pentan-1-ol (Reference Example 65) is deblocked by stirring in ethanol under a hydrogen atmosphere with palladium on carbon. This mixture is filtered under nitrogen and the filtrate concentrated in vacuo. The residue is reacted with diethyl phosphorochloridate in dichloromethane in the presence of triethylamine as described in Example 1, giving the desired product as solid.

## Example 15

### N-[N-(Diphenoxyphosphinyl)- L -phenylalanyl- L -2-(1-methylethoxy)glycyl]-(S)-2-amino-4-methyl-(R)-1-(2-thiazolyl)pentan-1-ol

N-[N-(Benzyloxycarbonyl)- L -phenylalanyl- L -2-(1-methylethoxy)glycyl]-(S)-2-amino-4-methyl-(R)-1-(2-thiazolyl)pentan-1-ol (Reference Example 66) is deblocked by stirring with palladium on carbon in ethanol under a hydrogen atmosphere. This mixture is filtered under nitrogen and the filtrate concentrated in vacuo. The residue is reacted with diphenyl phosphorochloridate in dichloromethane in the presence of triethylamine as described in Example 2, giving the desired product as a solid.

## Example 16

### N-[N-(Diethoxyphosphinyl)glycyl- L -phenylalanyl- L -methionyl]-(S)-2-amino-4-methyl-(R)-1-(2-thiazolyl)pentan-1-ol

To a suspension of 0.317 g of N-[N-(tert-butoxycarbonyl)glycyl- L -phenylalanyl- L -methionyl]-(S)-2-amino-4-methyl)-(R)-1-(2-thiazolyl)pentan-1-ol (Reference Example 70) in a mixture of 3 ml of dichloromethane and 3 ml of trifluoroacetic acid, was added 3 ml of dry tetrahydrofuran. This mixture was stirred for one hour, then concentrated in vacuo to an oil. This oil was triturated with 3 ml of 1N sodium hydroxide, giving 0.15 g of solid which was collected and washed with water. The solid was dissolved in 2 ml of tetrahydrofuran and 34 mg of diethyl phosphorochloridate and 0.05 ml of triethylamine added. This mixture was stirred for 2 hours, then the solvent was removed in vacuo giving a gum. The gum was dissolved in 2 ml of ethyl acetate and washed with 2N citric acid and 1N sodium bicarbonate. The organic layer was dried and the solvent removed, giving 0.14 g of a gum. This gum was purified on thick layer silica gel plates with ethyl acetate:ethanol (19:1) as solvent, giving 40 mg of the desired product.
Analysis Calculated: C, 51.9; H, 6.8; N, 10.4; P, 4.6.
Found: C, 52.2; H, 6.6; N, 9.3; P, 4.5.

## Example 17

N-[N-[(5-Methoxy-1,3,2-dioxaphosphorin-2-yl)-P-oxide]-L-phenylalanyl-L-leucyl]-(S)-2-amino-3-cyclohexyl-(R)-1-(2-thiazolyl)propan-1-ol

To a solution of 0.12 g of N-(L-phenylalanyl-L-leucyl)-(S)-2-amino-3-cyclohexyl-(R)-1-(2-thiazolyl)-1-[-(t-butyl)(dimethyl)silyloxy]propane in 2 ml of dichloromethane was added 40 mg of triethylamine followed by 56 mg of 2-chloro-5-methoxy-1,3,2-dioxaphosphorinone, 2-oxide. This mixture was stirred for 15 hours, then diluted with 15 ml of ethyl acetate and washed with 5 ml of 1N hydrochloric acid, 2x5 ml of 10% sodium carbonate and 5 ml of saturated sodium chloride solution. The organic layer was dried and evaporated. The residual gum was washed with hexane and dried in vacuo, giving 0.19 g of a foamy white solid. This solid was dissolved in one ml of tetrahydrofuran, then cooled to 0° C and 0.2 ml of 1M tetra-n-butylammonium fluoride in tetrahydrofuran added. This mixture was stirred for 2 hours at 0° C and then diluted with 5 ml of 10% sodium bicarbonate solution and 10 ml of ethyl acetate. The organic layer was separated and washed with 5 ml of 0.5N hydrochloric acid, 5 ml of saturated sodium bicarbonate solution, 5 ml of saturated sodium chloride solution and dried. The solvent was removed in vacuo and the residue triturated with ether-hexane, giving 0.16 g of the desired product as a white solid; $[\alpha]_D^{26}$ -46° ±1 (c = 0.718, methanol).

The compounds of Reference Examples 35, 37, 45, 46, 48, 57, 58 and 59 may be deblocked as described in Reference Example 49 and reacted with diethyl phos phorochloridate as described in Example 1 to give the following compounds:
N-[N-(Diethoxyphosphinyl)-L-phenylalanyl-L-leucyl]-(S)-2-amino-3-phenyl-(S)-1-(2-thiazolyl)propan-1-ol
N-[N-(Diethoxyphosphinyl)-L-phenylalanyl-L-leucyl]-(S)-2-amino-3-phenyl-(R)-1-(2-thiazolyl)propan-1-ol
N-[N-(Diethoxyphosphinyl)-L-phenylalanyl-L-histidyl]-(S)-2-amino-4-methyl-(R)-1-(2-thiazolyl)pentan 1-ol
N-[N-(Diethoxyphosphinyl)-L-phenylalanyl-L-histidyl]-(S)-2-amino-3-cyclohexyl-(R)-1-(2-thiazolyl)propan-1-ol
N-[N-(Diethoxyphosphinyl)-L-prolyl-L-phenylalanyl-L-leucyl]-(S)-2-amino-4-methyl-(R)-1-(2-thiazolyl)-propan-1-ol
N-[N-(Diethoxyphosphinyl)-L-phenylalanyl-L-leucyl]-(S)-2-amino-4-methyl-(R,S)-1-(2-furanyl)pentan-1-ol
N-[N-(Diethoxyphosphinyl)-L-phenylalanyl-L-leucyl]-5-[(S)-2-amino-(R,S)-1-hydroxy-4-methylpentyl]-2-thiophenecarboxamide
N-[N-(Diethoxyphosphinyl)-L-phenylalanyl-L-leucyl]-(S)-2-amino-4-methyl-(R,S)-1-(2-thienyl)pentan-1-ol.

## Example 18

N-[N-(Diethoxyphosphinyl)-L-phenylalanyl-L-leucyl]-(S)-2-amino-3-cyclohexyl-(R)-1-(2-furanyl)propan-1-ol

A. 1,1-Dimethylethyl (S)-[1-(cyclohexylmethyl)-2-(2-furanyl)-2-oxoethyl]carbamate

A solution of 1.57 g of N-methyl-N-methyl-$N^{\alpha}$-t-butoxycarbonyl-L-cyclohexylalaninamide in 15 ml of dry tetrahydrofuran was cooled to -78° C under argon. To the solution was added dropwise 5.9 ml of secondary butyllithium (0.85M in hexane). The viscous mixture was stirred at -78° C for 1.5 hour and then warmed to 0° C and stirred for 5 minutes. (Solution A)

A solution of 0.73 ml of furan in 5 ml of dry tetrahydrofuran was cooled to 0° C and 3.8 ml of n-butyllithium (2.35M in hexane) added. The yellow suspension was stirred at 0° C for 1.7 hour and then allowed to warm to room temperature for 15 minutes (yellow solution B).

The yellow solution B was added to solution A and the mixture stirred at 0° C for 1.5 hours. The mixture was quenched with 5 ml of saturated aqueous ammonium chloride and the solvent tetrahydrofuran removed under vacuum. The residue was diluted with 50 ml of ethyl acetate and 20 ml of 1N hydrochloric acid. The organic phase was separated and washed successively with 20 ml of 1N hydrochloric acid, 20 ml of water, 20 ml of saturated sodium bicarbonate, 20 ml of brine and dried over sodium sulfate. The solvent was removed under vacuum to give 1.63 g of a light brown gum. This gum was dissolved in ether-hexane (1:5) and the solution filtered through a thin pad of hydrous magnesium silicate. The pad was washed with ether-hexane (1:5) and the filtrate concentrated. The residue was triturated with hexane to give 1.23 g of light yellow crystals; $[\alpha]_D^{26}$ +41° ±1 (c = 1.14, methanol).

B. (S)-2-(N-tert-Butoxycarbonyl)amino-3-cyclohexyl-(R,S)-1-(2-furanyl)propan-1-ol

A solution of 0.16 g of 1,1-dimethylethyl (S)-[1-(cyclohexylmethyl)-2-(2-furanyl)-2-oxoethyl]carbamate in 2 ml of dry tetrahydrofuran and 0.2 ml of methanol was cooled to 0° C under argon and 23 mg of sodium borohydride added. The solution was stirred at 0° C for one hour and quenched with 2 ml of saturated aqueous ammonium chloride. The organic solvent was removed under vacuum and the residue diluted with 5 ml of saturated aqueous ammonium chloride. The organic solvent was removed under vacuum and the residue diluted with 5 ml of water and extracted with 10 ml of ethyl acetate. The organic layer was separated, washed successively with 5 ml of 0.5N hydrochloric acid, 5 ml of saturated sodium bicarbonate, 5 ml of brine and dried over sodium sulfate. The solvent was removed under vacuum to give 0.19 g of gummy solid.

C. (4S-trans)-4-(Cyclohexylmethyl)-5-(2-furanyl)-2-oxazolidione

To a solution of 0.23 g of (S)-2-(N-tert-butoxycarbonyl)amino-3-cyclohexyl-(R,S)-1-(2-furanyl)propan-1-ol in 3 ml of dichloromethane was added 0.06 ml of trifluoroacetic acid. The solution was stirred for 23 hours at room temperature, washed with 1N sodium hydroxide, dried over sodium sulfate and the solvent removed to give 0.17 g of solid.

D. (S)-2-Amino-3-cyclohexyl-(R)-1-(2-furanyl)propan-1-ol

A 0.15 g sample of (4S-trans)-4-(cyclohexylmethyl)-5-(2-furanyl)-2-oxazolidinone was dissolved in a mixture of 3 ml of ethanol and 3 ml of 1N sodium hydroxide. The solution was refluxed for 17 hours, diluted with 3 ml of water and concentrated under vacuum to remove the ethanol. The aqueous residue was extracted with two 5 ml portions of dichloromethane and the extracts dried over sodium sulfate. The solvent was removed to give 0.15 g of solid which was washed with hexane to give 0.13 g of white solid; $[\alpha]_D^{26}$ -10° ±2 (c = 0.507, methanol).

E.

A mixture of 0.12 g of phenyl dichlorophosphate and 0.12 g of imidazole in 2.5 ml of dichloro methane was stirred at room temperature for one hour and then cooled to -15° C under argon. N-(tert-butoxycarbonyl)-L-phenylalanyl-L-leucine (0.21 g) was added in portions and the mixture stirred for one hour. To the above mixture was added 80 mg of (S)-2-amino-3-cyclohexylmethyl-(R)-1-(2-furanyl)propan-1-ol in 0.5 ml of dichloromethane. The mixture was stirred at -15° C for 23 hours, and diluted with 15 ml of ethyl acetate and 10 ml of 0.5N hydrochloric acid. The organic layer was separated and washed successively with 5 ml of 0.5 N hydrochloric, 5 ml of 1N sodium hydroxide, 5 ml of brine and dried over sodium sulfate.

The solvent was removed under vacuum to give 0.24 g of solid. This solid was dissolved in 2 ml of methanol and one ml of 1N sodium hydroxide and the mixture stirred for one hour. The mixture was diluted with 4 ml of water and concentrated to remove the methanol. The residue was extracted with 10 ml of ethyl acetate and the extract dried over sodium sulfate. The extract was filtered through a thin pad of hydrous magnesium silicate and the pad washed with ethyl acetate. The filtrate was concentrated under vacuum to give 0.16 g of solid, washing the solid with hexane gives 0.15 g of the product of the Example as a white solid: $[\alpha]_D^{26}$ -31° ±2 (c = 0.58, methanol).

As described for Reference Example 49, the preceding compound in dichloromethane is treated with trifluoroacetic acid to give N-(L-phenylalanyl-L-leucyl)-(S)-2-amino-3-cyclohexyl-(R)-1-(2-furanyl)propan-1-ol as a solid. This solid (0.10 g) in dichloromethane is reacted with diethyl phosphorochloridate as described for Example 1 to give a white solid.

## Example 19

### N-[N-Diethoxyphosphinyl)-L-phenylalanyl-L-leucyl]-(S)-2-amino-4-methyl-(R)-1-(2-imidazolyl)pentan-1-ol

A solution of 18.4 g of 1-[[2-(trimethylsilyl)ethoxy]methyl]-1H-imidazole in 80 ml of tetrahydrofuran under argon was chilled to -78° C and to the solution was added 37.2 ml of n-butyllithium in hexane (2.5M). After stirring at -78° C for 1.5 hours, the cooling bath removed for 5 minutes, and the mixture quenched with 30 ml of saturated ammonium chloride solution. The tetrahydrofuran was removed under vacuum and the aqueous residue diluted with 30 ml of water and then extracted with 300 ml of ethyl acetate. The organic layer was washed with saturated sodium bicarbonate (50 ml), saturated sodium chloride solution and dried ($Na_2SO_4$). The solvent was removed under vacuum to give 29.0 g of an amber oil. Chromatography on silica gel with ethyl acetate-hexane(2:3) gave 8.2 g of solid. This solid was chromatographed on silica gel with ethyl acetate-hexane (3:2) as an eluent to give 4.14 g of (S)-2-(t-butoxycarbonyl)amino-4-methyl-(R)-1-(2-[N-[2-(trimethylsilyl)ethoxy]methyl]imidazolyl)pentan-1-ol as a white solid; $[\alpha]_D^{26}$ +8° ±2 (c = 0.583, methanol).

To 0.21 g of the preceding compound in one ml of dichloromethane was added 0.39 ml of trifluoroacetic acid and the solution stirred for 2 hours at room temperature under argon. The mixture was diluted with 10 ml of dichloromethane and poured into ice-cold 1N sodium hydroxide. The organic layer was separated and the aqueous layer extracted with dichloromethane. The organic layer and extracts were combined, dried ($Na_2SO_4$) and the solvent removed to give 0.15 g (S)-2-amino-4-methyl-(R)-1-(2-[N-[2-(trimethylsilyl)ethoxy]methyl]imidazolyl)pentan-1-ol as a white solid.

A mixture of 0.13 g of phenyl dichlorophosphate and 0.20 g of imidazole in 4 ml of dichloromethane was stirred for 0.5 hour and then cooled to -15° C. To this mixture was added 0.23 g of N-t-butoxycarbonyl-L-phenylalanyl-L-leucine in 0.5 ml of dichloromethane and the mixture stirred at -15° C for 1.5 hours. To this mixture was added 0.15 g of (S)-2-amino-4-methyl-(R)-1-(2-[N-[2-(trimethylsilyl)ethoxy)methyl]-imidazolyl)pentan-1-ol in 0.5 ml of dichloromethane. The mixture was stirred at -15° C for 18 hours and at room temperature for 3 hours. The mixture was diluted with 30 ml of ethyl acetate, washed with water, 10% potassium bicarbonate, saturated sodium chloride solution and dried ($Na_2SO_4$). The solvent was removed under vacuum to give 0.24 g of N-[N-(t-butoxycarbonyl)-L-phenylalanyl-L-leucyl]-(S)-2-amino-4-methyl-(R)-1-2-[N-(2-(trimethylsilyl)methoxy]methyl]imidazolyl)pentan-1-ol as a white solid.

The preceding compound was dissolved in one ml of tetrahydrofuran. To this solution was added one ml of tetra-n-butyl-ammonium fluoride in tetrahydrofuran (1.0M), 0.2 ml of triethylamine and 0.2 ml of water. The mixture was refluxed for 24 hours. An additional one ml of tetra-n-butylammonium fluoride in tetrahydrofuran (1M) was added and the mixture refluxed for 24 hours. After adding one ml of water the tetrahydrofuran was removed under vacuum and the aqueous residue extracted with ethyl acetate. The extract was washed with water and saturated sodium chloride solution, dried ($Na_2SO_4$) and the solvent removed. The residue (0.169 g) was chromatographed on 15 g of silica gel with ethyl acetate as eluent to give 60 mg of N-[N-(t-butoxycarbonyl-L-phenylalanyl-L-leucyl]-(S)-2-amino-4--methyl-(R)-1-(2-imidazolyl)pentan-1-ol as a white solid.

The preceding solid in dichloromethane and trifluoroacetic acid is stirred for 20 hours at room temperature and worked up as described for Reference Example 49 to give N-(L-phenylalanyl-L-leucyl)-(S)-2-amino-4-methyl-(R)-1-(2-imidazolyl)pentan-1-ol as a white solid. As for Example 1, the preceding

compound is treated with diethyl phosphorochloridate to give the product of the Example as a white solid.

## Example 20

### N-[N-(Diisopropyloxyphosphinyl)-L-phenylalanyl-L-leucyl]-(S)-2-amino-4-methyl-(R)-1-(2-imidazolyl)pentan-1-ol

A 0.10 g sample of N-[N-(t-butoxycarbonyl)-L-phenylalanyl-L-leucyl]-(S)-2-amino-4-methyl-(R)-1-(2-[N-[2-(trimethylsilyl)ethoxy]methyl]imidazolyl)pentan-1-ol in one ml of dichloromethane and 0.18 ml of trifluoroacetic acid is stirred for 20 hours and worked-up as for Reference Example 49 to give 55 mg of N-(L-phenylalanyl-L-leucyl)-(S)-2-amino-4-methyl-(R)-1-(2-[N-[2-(trimethylsilyl)ethoxy]methyl]imidazolyl)-pentan-1-ol as a white solid.

The preceding compound is reacted with diisopropyl phosphorochloridate as described for Example 1 to give N-[N-(diisopropyloxyphosphinyl)-L-phenylalanyl-L-leucyl]-(S)-2-amino-4-methyl-(R)-1-(2-[N-[2-(trimethylsilyl)ethoxy]methyl]imidazolyl)pentan-1-ol as a white solid.

The preceding compound (50 mg) is dissolved in one ml of tetrahydrofuran and one ml of tetra-n-butylammonium fluoride in tetrahydrofuran (1.0M), 0.2 ml of triethylamine and 0.2 ml of water added. The mixture is refluxed for 24 hours and the solvent removed under vacuum. The residue in water is extracted with ethyl acetate, the extract washed with water, saturated sodium chloride solution and dried. The solvent is removed under vacuum and the residue chromatographed on silica gel to give the product of the Example as a white solid.

## Example 21

### N-[N-(Diethoxyphosphenyl)-L-phenylalanyl-L-leucyl]-(S)-2-amino-4-methyl-(R)-1-(2-pyridinyl)pentan-1-ol

A. (S)-2-tert-Butoxycarbonylamino-4-methyl-(R,S)-1-(2-pyridinyl)pentan-1-ol.

To 2-bromopyridine (3.16 g, 20 mm) in 20 ml of tetrahydrofuran at -78° and under argon was added slowly 11.2 ml (20 mm) of 2M n-butyllithium in tetrahydrofuran. The solution was stirred for 5 minutes then 2.1 g (10 mm) of N-tert-butoxycarbonyl-L-leucinal in 10 ml of tetrahydrofuran was added. The mixture was stirred for 35 minutes then quenched with 10 ml of saturated sodium sulfate solution, warmed to room temperature and poured into 50 ml of water. The mixture was extracted with ethyl acetate. The pale yellow organic layer was dried (sodium sulfate) and evaporated under reduced pressure. The product was chromatographed over silica with 30% ethyl acetate in hexane followed by 50% ethyl acetate in hexane to give 0.620 g of yellow oil; $[\alpha]_D^{26}$ -21° ±3 (c = 0.329, dichloromethane ) (mixture of diastereomers).

B. (S)-2-tert-Butoxycarbonylamino-4-methyl-(R)-1-(2-pyridinyl)pentan-1-ol, O-acetate and (S)-2-tert-butoxycarbonylamino-4-methyl-(S)-1-(2-pyridinyl)pentan-1-ol, O-acetate.

(S)-2-tert-butoxycarbonylamino-4-methyl-(R,S)-1-(2-pyridinyl)pentan-1-ol (4.12 g) in 5 ml of acetic anhydride and 15 ml of methylene chloride was stirred at room temperature for 22 hours. Excess acetic anhydride was removed under reduced pressure. The residue was dissolved in methylene chloride and washed with saturated aqueous sodium bicarbonate. The methylene chloride layer was dried over sodium sulfate and the solvent removed under vacuum. The residue was chromatographed over 200-400 mesh silica gel with 30% ethyl acetate in hexane to give 1.55 of (S)-2-tert-butoxycarbonylamino-4-methyl-(S)-1-(2-pyridinyl)pentan-1-ol, O-acetate as crystals, mp 83-85° C: $[\alpha]_D^{26}$ -62° ±1 (c = 1.01, dichloromethane) and 1.4 g of (S)-2-tert-butoxycarbonylamino-4-methyl-(R)-1-(2-pyridinyl)pentan-1-ol, O-acetate as an amber oil; $[\alpha]_D^{26}$

+3° ±1 (c = 1.066, methanol ).

C. (S)-2-amino-4-methyl-(S)-1-(2-pyridinyl)pentan-1-ol, dihydrochloride and (S)-2-amino-4-methyl-(R)-1-(2-pyridinyl)pentan-1-ol, dihydrochloride.

(S)-2-tert-butoxycarbonylamino-4-methyl-(S)-1-(2-pyridinyl)pentan-1-ol, O-acetate (1.35 g) was dissolved in a mixture of 3 ml of ethanol and 8 ml of 5N sodium hydroxide and the mixture stirred at room temperature for 2 hours. The mixture was extracted with dichloromethane, the extract dried and the solvent removed to give 1.17 g of solid. To this solid in 20 ml of dichloromethane was added 5 ml of dichloromethane saturated with anhydrous hydrochloric acid. The solvent was removed and the solid dissolved in ethanol. Dilution with ether and chilling gave 0.36 g of crystals, mp 211-213°C (dihydrochloride).

(S)-2-tert-butoxycarbonylamino-4-methyl-(R)-1-(2-pyridinyl)pentan-1-ol, O-acetate (3.32 g) was dissolved in a mixture of 3 ml of methanol and 8 ml of 5N sodium hydroxide. The mixture was stirred at 10°C for 2 hours and extracted with dichloromethane. The extract was dried over sodium sulfate and the solvent removed under vacuum to give 2.69 g of an oil. To the oil was added 15 ml of 2N anhydrous hydrochloric acid in ethyl acetate. After stirring at room temperature over night, the solvent was removed under vacuum to give 2.4 g of (S)-2-amino-4-methyl-(R)-1-(2-pyridinyl)pentan-1-ol, dihydrochloride, mp 152-154°C.

D. N-[N-(tert-butoxycarbonyl)- L -phenylalanyl- L -leucyl]-(S)-2-amino-4-methyl-(S)-1-(2-pyridinyl)pentan-1-ol and N-[N-(tert-butoxycarbonyl)- L -phenylalanyl- L -leucyl]-(S)-2-amino-4-methyl-(R)-1-(2-pyridinyl)-pentan-1-ol.

To a solution containing 0.769 g of N-tert-butoxycarbonyl- L -phenylalanyl- L -leucine in 10 ml of tetrahydrofuran at -10°C under argon was added 0.660 ml (6mm) of N-methylmorpholine. After a few minutes isobutyl chloroformate (0.268 ml) and 0.542 g of (S)-2-amino-4-methyl-(R)-1-(2-pyridinyl)pentan-1-ol, dihydrochloride was added. The mixture was stirred at -10°C for 1.5 hours, then at room temperature for 5 hours. The salts were filtered and the filtrate evaporated under reduced pressure. The residue was chromatographed over 200-400 mesh silica with 50/50 ethyl acetate-hexane to give 0.360 g of white crystals. mp 154-156°; $[\alpha]_D^{26}$ -13° ±1 (c = 0.630, methanol) (2S, 1R) diastereomer.

In a similar manner N-tert-butoxycarbonyl- L -phenylalanyl- L -leucine was coupled to (S)-2-amino-4-methyl-(S)-1-(2-pyridinyl)pentan-1-ol, dihydrochloride to give N-[N-(tert-butoxycarbonyl)- L -phenylalanyl- L -leucyl]-(S)-2-amino-4-methyl-(S)-1-(2-pyridinyl)pentan-1-ol as crystals mp 141-142°C; $[\alpha]_D^{26}$ -42° ±2 (c = 0.401, methanol).

As described for Reference Example 49, a 0.20 g sample of N-[N-(tert-butoxycarbonyl)- L -phenylalanyl- L -leucyl]-(S)-2-amino-4-methyl-(R)-1-(2-pyridinyl)-pentan-1-ol in dichloromethane is treated with trifluoroacetic acid to give N-[ L -phenylalanyl- L -leucyl]-(S)-2-amino-4-methyl-(R)-1-(2-pyridinyl)-pentan-1-ol as a solid. Without purification this solid is reacted with diethyl phosphorochloridate as described for Example 1 to give the product of the example as a white solid.

## Example 22

### N-[N-(Diethoxyphosphinyl)- L -phenylalanyl- L -leucyl]-(S)-2-amino-3-cyclohexyl-(R)-1-(2-pyridinyl) propan-1-ol

To a solution of 3.16 g of 2-bromopyridine in 20 ml of dry tetrahydrofuran cooled to -78°C under argon was added 11.6 ml of a 2.0 molar n-butyllithium in tetrahydrofuran. After 5 minutes, N-methoxy-N- methyl $N^{\alpha}$-t-butoxycarbonyl- L -cyclohexylalaninamide (3.14 g) in 20 ml of tetrahydrofuran was added under extremely dry conditions. The dark solution was stirred for one hour at -78°C. The mixture was quenched with saturated aqueous sodium sulfate solution (20 ml), warmed to room temperature and poured into 50 ml of water. The mixture was extracted with ethyl acetate and the ethyl acetate layer dried over anhydrous sodium sulfate and the solvent removed under vacuum. The residue was chromatographed on a silica gel

column with ethyl acetate-hexane (1:1) as solvent to give 2.01 g of 1,1-dimethylethyl (S)[1-(cyclohexylmethyl)-2-oxo-2-(2-pyridinyl)ethyl]carbamate as an oil; $[\alpha]_D^{26}$ +35° ±1 (c = 0.812, methanol).

A solution of 1.35 g of the preceding compound in 30 ml of dry tetrahydrofuran under argon was chilled to -78° C and 7.5 ml of lithium tri-sec-butylborohydride in tetrahydrofuran (1.0M). The mixture was stirred for 5 hours at -78° C and 12 hours at room temperature. The cooled mixture (ice bath) was quenched with water (8 ml) and 8 ml of 30% hydrogen peroxide slowly added dropwise. The mixture was extracted with ethyl acetate, dried over sodium sulfate and the solvent removed under vacuum. The residue was chromatographed on silica gel column with ethyl acetate-hexane (2:3) to give 0.27 g of (S)-2-tert-butoxycarbonylamino-3-cyclohexyl-(S)-1-(2-pyridinyl)-propan-1-ol as a white solid; $[\alpha]_D^{26}$ +4° ±1 (c = 1.18, methanol), and 0.25 g of (4S-trans)-4-(cyclohexylmethyl)-5-(2-pyridinyl)-2-oxazolidinone as a clear oil: $[\alpha]_D^{26}$ -40° ±1 (c = 1.16, methanol). A 3.2 g sample of the preceding oil was dissolved in 60 ml of ethanol and 60 ml of 1N sodium hydroxide. The mixture was refluxed for 17 hours, diluted with 60 ml of water and concentrated to remove the ethanol. The mixture was extracted with dichloromethane (3 x 80 ml) and the extract dried over sodium sulfate. The solvent was removed to give 2.54 g of (S)-2-amino-3-cyclohexyl-(R)-1-(2-pyridinyl)-propan-1-ol as crystals, mp 53-56° C.

N-tert-butoxycarbonyl-L-phenylalanyl-L-leucine (0.769 g) in 10 ml of dry tetrahydrofuran was cooled to -10° C under argon and 0.660 ml of N-methylmorpholine added. After stirring for 2 minutes, 0.268 ml of isobutyl chloroformate was added followed by 0.534 g of (S)-2-amino-3-cyclohexyl-(R)-1-(2-pyridinyl)propan-1-ol. The mixture was stirred at -10° C for one hour and then at room temperature for 5 hours. The solid was filtered off and the filtrate evaporated under vacuum. The residue was chromatographed over silica gel with ethyl acetate-hexane (3:7) as eluent and then with ethyl acetate-hexane (1:1) to elute the product. Fractions containing product were concentrated to give 0.41 g of N-[N-tert-butoxycarbonyl-L-phenylalanyl-L-leucyl]-(S)-2-amino-3-cyclohexyl-(R)-1-(2-pyridinyl)propan-1-ol as crystals, mp 140-142° C; $[\alpha]_D^{26}$ -20° ±1 (c = 1.05, methanol).

As described for Reference Example 49, a 0.20 g sample of the preceding compound in dichloromethane is treated with trifluoroacetic acid to give N-[L-phenylalanyl-L-leucyl]-(S)-2-amino-3-cyclohexyl-(R)-1-(2-pyridinyl)propan-1-ol as a solid. This solid is reacted with diethyl phosphorochloridate as described for Example 1 to give a white solid; $[\alpha]_D^{26}$ -46° ±2 (c = 1.0, methanol).

## Example 23

### N-[N-(Diethoxyphosphinyl)-L-phenylalanyl-L-leucyl]-(S)-2-amino-3-cyclohexyl-(S)-1-(2-pyridinyl) propan-1-ol

To a solution of 1.93 g of (S)-2-tert-butoxycarbonylamino-3-cyclohexyl-(S)-1-(2-pyridinyl)propan-1-ol in 10 ml of ethyl acetate was added 25 ml of 2N anhydrous hydrochloric acid in ethyl acetate. The mixture was stirred for 45 minutes, diluted with hexane and the solid filtered off. The solid was washed with hexane to give 1.04 g of (S)-2-amino-3-cyclohexyl-(S)-1-(2-pyridinyl)-propanol, hydrochloride as crystals.

To a solution of 0.378 g of N-tert-butoxycarbonyl-L-phenylalanyl-L-leucine in 10 ml of dry tetrahydrofuran at -10° C under argon was added 0.330 ml of N-methylmorpholine. To the solution was added 0.134 ml of isobutyl chloroformate followed by 0.37 g of (S)-2-amino-3-cyclohexyl-(S)-1-(2-pyridinyl)-propan-1-ol, hydrochloride . The mixture was stirred at -10° C for 2 hours, the solids filtered off and the filtrate evaporated under vacuum. The residue was chromatographed over silica gel with ethyl acetate-hexane(1:1) to give 0.122 g of N-[N-(t-butoxycarbonyl)-L-phenylalanyl-L-leucyl]-(S)-2-amino-3-cyclohexyl-(S)-1-(2-pyridinyl)propan-1-ol as an oil; $[\alpha]_D^{26}$ -43° ±1 (c = 1.101, methanol).

As described for Example 49, a 0.10 g sample of the preceding compound in dichloromethane is treated with trifluoroacetic acid to give N-[N-L-phenylalanyl-L-leucyl]-(S)-2-amino-3-cyclohexyl-(S)-1-(2-pyridinyl)propan-1-ol as a solid. This solid is reacted with diethyl phosphorochloridate as described for Example 1 to give a white solid.

## Example 24

N-[N-(Diethoxyphosphinyl)- L -phenylalanyl- L -histidyl]-(S)-2-amino-3-cyclohexyl-(R)-1-(2-thiazolyl) propan-1-ol

To a mixture of 0.59 g of $N^{\alpha}$-t-butoxycarbonyl- L-histidine in 6 ml of dichloromethane was added 0.32 ml of triethylamine and 1.02 g of benzotriazol-1-yloxytris(dimethylamino)phosphonium hexafluorophosphate. After stirring one minute 0.53 g of (S)-2-amino-3-cyclohexyl-(R)-1-(2-thiazolyl)propan-1-ol was added. The mixture was stirred over night and the solvent removed. The residue was dissolved in 20 ml of ethyl acetate and washed successively with 5 ml of water, saturated sodium bicarbonate and saturated sodium chloride solution. The organic layer was dried ($MgSO_4$) and passed through a short pad of hydrous magnesium silicate. The pad was washed with ethyl acetate and the filtrate concentrated under vacuum to give 1.1 g of solid. The solid was dissolved in dichloromethane and filtered through diatomaceous earth. The filtrate was concentrated under vacuum to give 0.98 g of N-[N-t-butoxycarbonyl)- L-histidyl]-(S)-2-amino-3-cyclohexyl-(R)-1-(2-thiazolyl)propan-1-ol as a solid (glass). This glass in one ml of ethyl acetate was treated with 10 ml of 2N anhydrous hydrochloric acid in ethyl acetate and the mixture stirred for 2 hours. The mixture was diluted with 10 ml of hexane, chilled and filtered to give crystals. The crystals were dissolved in 3 ml of water and 0.4 ml of 15N ammonium hydroxide added. The mixture was extracted with dichloromethane and then with ethyl acetate. The combined extracts were dried (MgSO4) and the solvent removed to give 0.4 g of N-[ L-histidyl]-(S)-2-amino-3-cyclohexyl-(R)-1-(2-thiazolyl)propan-1-ol as a gum.

A mixture of 3.30 g of L -phenylalanine, 10 ml of triethylamine, 6 ml of water and 4 ml of ethanol was cooled to 0° C under argon. To the stirred mixture was added dropwise a mixture of 2.76 g of diethyl phosphite in 8 ml of carbon tetrachloride. The mixture was stirred over night (16 hours) and acidified with 3N hydrochloric acid to pH 2. The mixture was extracted three times with 40 ml of ethyl acetate, the extract dried ($MgSO_4$) and the solvent removed under vacuum to give 5.4 g of N-(diethoxyphosphinyl)- L -phenylalanine as a viscous oil.

To a sample (1 mmol) of the preceding compound in dichloromethane (5 ml) at -10° C is added triethylamine (2.5 ml) followed by dropwise addition of as solution of N,N-dicyclohexylcarbodiimide (one mmol) and N-hydroxysuccinimide (1 mmol) in 5 ml of dichloromethane. To this mixture is added 1 mmol of N-[ L-histidyl]-(S)-2-amino-3-cyclohexyl-(R)-1-(2-thiazolyl)propan-1-ol in one ml of dichloromethane. The mixture is stirred at 20° C for 5 hours, filtered and the filtrate is washed with water, saturated sodium bicarbonate, water and dried. The solvent is removed to give a white solid; $[\alpha]_D^{26}$ -26° ±1 (c = 1.0, chloroform).

## Example 25

N-[N-(Diisopropyloxyphosphinyl)- L -phenylalanyl- L -leucyl]-(S)-2-amino-3-cyclohexyl-(R)-1-(2-pyridinyl) propan-1-ol

To a solution of 0.324 g of $N^{\alpha}$-t-butoxycarbonyl- L-leucine in 6 ml of tetrahydrofuran chilled to -15° C was added 0.429 ml of N-methylmorpholine. After one minute 0.174 ml of isobutyl chloroformate was added followed by the addition of 0.399 g of (S)-2-amino-3-cyclohexyl-(R)-1-(2-pyridinyl)-propan-1-ol, hydrochlorride. The mixture was stirred at -15° C for one hour and at room temperature for 2 hours. The solids were filtered off and the filtrate evaporated under vacuum. The residual oil (0.30 g) was chromatographed over silica gel with ethyl acetate-hexane (2:3) to give 0.25 g of N-[N-(tert-butoxycarbonyl)- L-leucyl]-(S)-2-amino-3-cyclohexyl-(R)-1-(2-pyridinyl)propan-1-ol as crystals, mp 52-54° C; $[\alpha]_D^{26}$ -51° ±3 (c = 0.361, methanol).

As described for Reference Example 49, the preceding compound in dichloromethane is treated with trifluoroacetic acid to give N-( L-leucyl)-(S)-2-amino-3-cyclohexyl-(R)-1-(2-pyridinyl)propan-1-ol as a solid. This compound is reacted with N-(diisoproxyphosphinyl)- L-phenylalanine, activated with phenyl dichlorophosphate as described for Reference Example 110 to give a white solid.

## Example 26

N-[N-(Diethoxyphosphinyl)- L -phenylalanyl- L-histidyl]-(S)-2-amino-4-methyl-(R)-1-(2-thiazolyl)-pentan-1-ol

To a suspension of 2.4 g of $N^{\alpha}$-t-butoxycarbonyl- L-histidine in 24 ml of dichloromethane was added 1.3 ml of triethylamine, followed by the addition of 4.2 g of benzotriazol-1-yloxytris(dimethylamino)-phosphonium hexafluorophosphate (BOP). The mixture was stirred under argon for 2 minutes and 1.73 g of (S)-2-amino-4-methyl-(R)-1-(2-thiazolyl)pentan-1-ol added. The mixture was stirred for 4 days and the solvent removed. The residue was dissolved in 20 ml of ethyl acetate and the solution washed with 10 ml of water, three 10 ml portions of 1M sodium bicarbonate, 5 ml of 2N citric acid and 20 ml of 1M sodium bicarbonate. The organic layer was dried ($MgSO_4$) and then filtered through a thin pad of hydrous magnesium silicate (pad washed with ethyl acetate). The filtrate was concentrated under vacuum to give 2.7 g of N-[N-(t-butoxycarbonyl)- L-histidyl]-(S)-2-amino-4-methyl-(R)-1-(2-thiazolyl)pentan-1-ol as a foam. This foam (2.7 g) was dissolved in 5 ml of ethyl acetate and to the solution was added 30 ml of 2N anhydrous hydrochloric acid in ethyl acetate. The mixture was stirred for 2 hours (crystals separated) and 35 ml of hexane added. The mixture was filtered to give 4.55 g of N-( L-histidyl)-(S)-2-amino-4-methyl-(R)-1-(2-thiazolyl)pentan-1-ol, dihydrochloride as white hygroscopic crystals. The crystals were dissolved in 9 ml of water and 3 ml of concentrated ammonium hydroxide added. The mixture was extracted with ethyl acetate, the extract dried ($MgSO_4$) and the solvent removed under vacuum to give 1.23 g of N-( L-histidyl)-(S)-2-amino-4-methyl-(R)-1-(2-thiazolyl)pentan-1-ol-as a foam.

A 3.01 g sample of N-(diethoxyphosphinyl)- L -phenylalanine (see Example 24 for preparation) in 5 ml was added slowly with stirring to a prepared mixture of 3.40 g of imidazole, 2.2 g of phenyl dichlorophosphate in 25 ml dichloromethane (chilled in ice bath). The mixture was stirred at 0° C for 1 hour and then 10 mmole of N-( L -histidyl)-(S)-2-amino-4-methyl-(R)-1-(2-thiazolyl)pentan-1-ol is added. The mixture is stirred at 0° C for 5 hours and at room temperature for one hour. The mixture is diluted with dichloromethane and washed with water, 1M sodium bicarbonate, water and saturated sodium chloride solution. The organic layer is dried and the solvent removed to give a solid.

## Example 27

N-[N-(Diethoxyphosphinyl)- L -phenylalanyl- L-leucyl-(S)-2-amino-3-cyclohexyl-(R)-1-(2-imidazolyl) propan-1-ol

A solution (cooled to -78° C) of 17.82 g of 1-[[2-(trimethylsilyl)ethoxy]methyl]-1H-imidazole in 90 ml of dry tetrahydrofuran under argon was added slowly 36 ml of n-butyllithium in tetrahydrofuran (2.5 molar). After complete addition the mixture was stirred at -78° C for one hour (Solution A).

To a stirred solution of 18.84 g of N-methoxy-N-methyl $N^{\alpha}$-t-butoxycarbonyl- L-cyclohexylalaninamide in 180 ml of dry tetrahydrofuran chilled to -78° C under argon was added 67 ml of secondary butyllithium in hexane (0.85 molar). After the addition the mixture was stirred for 1.5 hours at -78° C (Solution B).

By the use of double-tipped needle technique the solution A was added to the solution B with stirring at -78° C. The mixture was stirred at -78° C for one hour and allowed to warm to 0° C and kept (ice bath) at 0° C for one hour. The mixture was quenched with 150 ml of saturated ammonium chloride solution and the tetrahydrofuran solvent removed under reduced pressure. The residual aqueous layer was extracted twice with 150 ml of ethyl acetate. The combined extract was washed with saturated sodium chloride and dried ($Na_2SO_4$). The solvent was removed under vacuum to give 35.0 g of dark orange oil. This oil (35 g) was chromatographed on a silica gel column with ethyl acetate-hexane (1:10) to give 17.82 g of 1,1-dimethylethyl (S)-[1-(cyclohexylmethyl)-2-oxo-2-(2-[N-[2-(trimethylsilyl)ethoxy]methyl]imidazolyl)ethyl]-carbamate as a yellow viscous oil.

To a solution of 9.02 g of the preceding compound in 80 ml of dry tetrahydrofuran cooled to -78° C was added dropwise 40 ml of potassium tri-sec-butylborohydride in tetrahydrofuran (1.0M). After the addition, the mixture was stirred at -78° C for 2 hours and 80 ml of saturated ammonium chloride solution was added. The mixture was allowed to warm to room temperature and the tetrahydrofuran removed under vacuum. The aqueous residue was diluted with 20 ml of water and extracted with 200 ml of ethyl acetate. The extract was washed twice with 80 ml of saturated ammonium chloride solution, 80 ml of saturated sodium carbonate solution and 80 ml of sodium chloride solution. The organic layer was dried ($Na_2SO_4$)

and the solvent removed to give 15.2 g of thick gum. This gum in a mixture of 100 ml of ethyl acetate and 80 ml of water was cooled in an ice bath and 20 ml of 30% hydrogen peroxide was added slowly. The mixture was stirred at room temperature for one hour, cooled in an ice bath and then solid sodium sulfite (45 g) added slowly in small portions. The organic layer was separated and the aqueous layer extracted with 100 ml of ethyl acetate. The combined organic layer and extract was dried ($Na_2SO_4$) and the solvent removed to give a light yellow solid (10.0 g). This solid was chromatographed over silica gel with ethyl acetate-hexane (2:3) to give 4.5 g of (S)-2-tert- butoxycarbonylamino-3-cyclohexyl-(R)-1-(2-[N-[2-(trimethylsilyl)ethoxy]methyl]imidazolyl)propan-1-ol as a white solid; $[\alpha]_D^{26}$ +9° ±1 (c = 1.034, methanol).

The preceding compound in dichloromethane is treated with trifluoroacetic acid as described for Reference Example 49 to give (S)-2-amino-4-cyclohexyl-(R)-1-(2-[N-(2-(trimethylsilyl)ethoxy]methyl]-imidazolyl)propan-1-ol as a solid; $[\alpha]_D^{26}$ -8° ±1 (c = 1.05, methanol.

To a solution of 3.40 g of imidazole in 25 ml of dichloromethane under argon was added slowly a solution of 2.20 g of phenyl dichlorophosphate in 5 ml of dichloromethane. The suspension was stirred for 0.5 hour and then cooled in an ice-bath to 0° C. To the mixture is added a solution of 3.01 g of N-(diethoxyphosphinyl)-L-phenylalanine in 5 ml of dichloromethane. The mixture was stirred at 0° C for one hour and 1.82 g of L-leucine methyl ester hydrochloride added in small portions. The mixture was stirred at 0° C for 5 hours and at room temperature for one hour. The mixture is diluted with dichloromethane and washed with 30 ml of 0.5N hydrochloric acid. The aqueous layer is extracted with 20 ml of dichloromethane and the organic layer and extract combined. The combined extract is washed with 20 ml of 0.5N hydrochloric acid, with 10 ml of water, 20 ml of saturated sodium bicarbonate and dried. The solvent is removed under vacuum to give 3.5 g of gum. This gum was chromatographed on silica gel with ethyl acetate-hexane (1:1) as eluent to give 2.11 g of N-[N-(diethoxyphosphinyl)- L-phenylalanyl]- L-leucine methyl ester, mp 92-94° C; $[\alpha]_D^{26}$ -27° ±1 (c = 1.035, methanol).

To solution of 1.71 g of N-[N-(diethoxyphosphinyl)- L-phenylalanyl]- L-leucine methyl ester in 8 ml of methanol cooled was added 4.8 ml of 1N sodium hydroxide. The mixture was stirred under argon at 0° C for 22 hours. The methanol was removed under vacuum and the aqueous residue acidified with 1N hydrochloric acid to pH 1 with stirring in 60 ml of ethyl acetate. The ethyl acetate layer was separated, washed with 20 ml of brine and dried over sodium sulfate. The solvent was removed and the solid washed with hexane to give 1.66 g of N-[N-(diethoxyphosphinyl)- L-phenylalanyl]- L-leucine as a white solid; $[\alpha]_D^{26}$ -21° ±1 (c = 1.111, methanol).

A sample of this solid (1 mmol) is activated with phenyl dichlorophosphate and imidazole in dichloromethane as described above and to the mixture is added (S)-2-amino-3-cyclohexyl-(R)-1-(2-[N-(2-(trimethylsilyl)ethoxy)methyl]imidazolyl)propan-1-ol. The mixture is stirred over night and the solvent removed under vacuum. To the residue is added water and ethyl acetate. The organic layer is separated, washed with water, 1N hydrochloric acid, 1M sodium bicarbonate, saturated sodium chloride solution and dried. The solvent is removed under vacuum and to the residue is added tetrahydrofuran, tetra-n-butylammonium fluoride in tetrahydrofuran (1.0M), water and triethylamine. The mixture is refluxed for 24 hours, the solvent removed and the residue in water extracted with ethyl acetate. The extract is washed with water and saturated sodium chloride, dried and the solvent removed to give the product as a solid; $[\alpha]_D^{26}$ -25±1 c = 1.0, methanol).

## Example 28

### N-[N-(Diethoxyphosphinyl)- L-phenylalanyl- L-leucy]-(S)-2-amino-3-cyclohexyl-(R)-1-(2-thiazolyl) propan-1-ol

To 0.65 g of phenyl dichlorophosphate in 1 ml of dichloromethane under argon was added 1.02 g of imidazole in 12 ml of dichloromethane. The mixture was stirred at room temperature for 0.5 hour and then cooled to -15° C. To this stirred mixture was added 1.24 g of N-[N-diethoxyphosphinyl)- L-phenylalanyl]- L-leucine and the mixture stirred at -15° C for one hour. To this mixture was added 0.71 g of (S)-2-amino-3-cyclohexyl-(R)-1-(2-thiazolyl)-1-[[(t-butyl)(dimethyl)silyl]oxy]propane in 2 ml of dichloromethane and the mixture stir red at -15° C for 29 hours. Water (10 ml) was added and the dichloromethane removed under vacuum. To the aqueous residue was added 30 ml of ethyl acetate. The organic layer was separated and washed with 10 ml of 1N hydrochloric acid, 20 ml of 0.5N hydrochloric acid, 5 ml of brine, two 10 ml

portions of 1N sodium hydroxide and 5 ml of brine. The organic layer was dried over sodium sulfate and passed through a thin pad of hydrous magnesium silicate. The pad was washed with ethyl acetate and the filtrate concentrated under vacuum to give 1.53 g of N-[N-(diethoxyphosphinyl)-L-phenylalanyl-L-leucyl]-(S)-2-amino-3-cyclohexyl-(R)-1-(2-thiazolyl)-1-[[(t-butyl)(dimethyl)silyl]oxy]propane as a foamy solid; $[\alpha]_D^{26}$ -40° ±1 c = 1.067, methanol). To a sample (1.40 g) of the preceding compound in 5 ml of tetrahydrofuran chilled to 0° C was added 2.1 ml of tetra-n-butylammonium fluoride in tetrahydrofuran (1.0M). The mixture was stirred at room temperature for 2 hours and diluted with 5 ml of saturated sodium bicarbonate. The tetrahydrofuran was removed under vacuum and the residue partitioned between 40 ml of ethyl acetate and 10 ml of water. The organic layer was separated, washed with 10 ml of 0.5N hydrochloric acid, 10 ml of saturated sodium bicarbonate, 10 ml of brine, dried over sodium sulfate and filtered. The filtrate was passed through a thin pad of hydrous magnesium silicate and the pad washed with ethyl acetate. The filtrate was concentrated under vacuum to give a solid which was washed with hexane to give 1.17 g of product as a white solid, mp 164-165° C; $[\alpha]_D^{26}$ -50° ±2 (c = 1.0, methanol).

As described for Example 28, the following Examples 29-33 were prepared.

## Example 29

### N-[N-(Diethoxyphosphinyl)-L-phenylanyl-L-leucyl]-(S)-2-amino-3-cyclohexyl-(R)-1-(5-methoxycarbonyl-2-thiazolyl)propan-1-ol

N-[(Diethoxyphosphinyl)-L-phenylalanyl-L-leucine (0.12 g) was reacted with (S)-2-amino-3-cyclohexyl-(R)-1-(5-methoxycarbonyl-2-thiazolyl)-1-[[(t-butyl)(dimethyl)silyl]oxy]propane (0.082 g) to give 0.16 g of N-[N-(diethoxyphosphinyl)-L-phenylalanyl-L-leucyl]-(S)-2-amino-3-cyclohexyl-(R)-1-(5-methoxycarbonyl-2-thiazolyl)-1-[[(t-butyl)(dimethyl)silyl]oxy]propane (0.16 g); $[\alpha]_D^{26}$ -64° ±1 (c = 0.788, methanol). Reaction of the preceding compound (0.11 g) with tetra-n-butylammonium fluoride in tetrahydrofuran (1.0M) gave 0.090 g of the product of the Example as a white powder; $[\alpha]_D^{26}$ -92° ±2 (c = 0.560, methanol).

## Example 30

### N-[N-(Diethoxyphosphinyl)-L-phenylalanyl-L-leucyl]-(S)-2-amino-3-cyclohexyl-(R)-1-(5-acetyl-2-furanyl)-propan-1-ol

N-[(Diethoxyphosphinyl)-L-phenylalanyl-L-leucine (0.14 g) was reacted with (S)-2-amino-3-cyclohexyl-(R)-1-(5-acetyl-2-furanyl)propan-1-ol (0.060 g) to give 0.10 g of the product of the Example as a white solid; $[\alpha]_D^{26}$ +3° ±1 (c =0.6890, methanol).

## Example 31

### N-[N-(Diethoxyphosphinyl)-L-phenylalanyl-L-leucyl]-(S)-2-amino-3-cyclohexyl-(R)-1-(2-furanyl)propan-1-ol

N-[N-(Diethoxyphosphinyl)-L-phenylalanyl-L-leucine (0.62 g) was reacted with (S)-2-amino-3-cyclohexyl-(R)-1-(2-furanyl)propan-1-ol (0.22 g) to give 0.60 g of the product of the Example as a white solid; $[\alpha]_D^{26}$ -34° ±1 (c = 1.05, methanol).

## Example 32

### N-[N-(Diethoxyphosphinyl)- L -phenylalanyl- L -leucyl]-(S)-2-amino-3-cyclohexyl-(R)-1-(1H-pyrazol-3-yl)-propan-1-ol

N-[N-(Diethoxyphosphinyl)- L -phenylalanyl- L -leucine (0.19 g) was reacted with (S)-2-amino-3-cyclohexyl-(R)-1-(1H-pyrazol-3-yl)-1-[[(1,1-dimethylethyl)dimethylsilyl]oxy]propane (0.10 g) to give N-[N-(diethoxyphosphinyl)- L -phenylalanyl- L -leucyl]-(S)-2-amino-3-cyclohexyl-(R)-1-(1H-pyrazol-3-yl)-1-[[(1,1-dimethylethyl)dimethylsilyl]oxy]propane (0.12 g) as a white solid; $[\alpha]_D^{26}$ -32±2 (c = 0.547, chloroform).

The preceding compound (80 mg) in 0.5 ml of tetrahydrofuran was reacted with 0.60 ml of tetra-n-butylammonium fluoride (1.0M in tetrahydrofuran) for 6 hours to give 55 mg of the product of the Example as a white solid; $[\alpha]_D^{26}$ -33° ±2 (c = 0.417, methanol).

## Example 33

### N-[N-(Dibenzyloxyphosphinyl)- L -phenylalanyl- L -leucyl]-(S)-2-amino-3-cyclohexyl-(R)-1-(2-thiazolyl) propan-1-ol

N-[(Dibenzylphosphinyl)- L -phenylalanyl- L -leucine (0.24 g) was reacted with (S)-2-amino-3-cyclohexyl-(R)-1-(2-thiazolyl)-1-[[(t-butyl)(dimethyl)silyl]oxy]propane (0.11 g) to give 0.28 g of N-[N-(dibenzyloxyphosphinyl)- L -phenylalanyl- L -leucyl]-(S)-2-amino-3-cyclohexyl-(R)-1-(2-thiazolyl)-1-[[(t-butyl)(dimethyl)silyl]oxy]propane as a white solid; $[\alpha]_D^{26}$ -31° ±1 (c = 0.797, methanol).

The preceding compound (0.21 g) in one ml of tetrahydrofuran and 0.29 ml of tetra-n-butylammonium fluoride in tetrahydrofuran (1.0M) at room temperature for one hour gave 0.18 g of the product of the Example as a white solid; $[\alpha]_D^{26}$ -38° ±1 (c = 0.608, methanol).

## Example 34

### N-[N-(Diethoxyphosphinyl)- L -phenylalanyl- L -leucyl]-(S)-2-amino-3-cyclohexyl-(R)-1-(2-thiazole-5-carboxylic acid)propan-1-ol

N-[N-(Diethoxyphosphinyl)- L -phenylalanyl- L -leucyl]-(S)-2-amino-3-cyclohexyl-(R)-1-(5-methoxycarbonyl-2-thiazolyl)propan-1-ol (40 mg) in one ml of methanol and one ml of 1N sodium hydroxide was stirred at room temperature for 2 hours and concentrated under vacuum. The aqueous residue was acidified with 1N hydrochloric acid and the suspension extracted with 10 ml of ethyl acetate. The extract was washed with brine, dried and evaporated to give 39 mg of the product as a solid; $[\alpha]_D^{26}$ -89° ±2 (c = 0.485, methanol).

## Example 35

### N-[N-(Diethoxyphosphinyl)- L -phenylalanyl- L -histidyl]-(S)-2-amino-3-cyclohexyl-(R)-1-(2-pyridinyl) propan-1-ol

A solution of N-(diethoxyphosphinyl)- L -phenylalanine (0.62 g) in one ml of dichloromethane was added to a chilled (0 °C) mixture of 0.43 g of diethylchlorophosphate and 0.68 g of imidazole in 6 ml of dichloromethane and the mixture stirred 45 minutes at 0 °C. To the solution was added 0.30 g of N-( L -histidyl)-(S)-2-amino-3-cyclohexyl-(R)-1-(2-pyridinyl)propan-1-ol in 2 ml of dichloromethane and the mixture stirred at 0 °C for 23 hours. To the mixture was added 10 ml of 1N sodium hydroxide and the dichloromethane removed under vacuum. The aqueous layer was decanted from the gummy precipitate and the gum rinsed twice with 10 ml portions of water. The gum was dissolved in 6 ml of methanol and 3 ml of 1N sodium hydroxide added. After stirring for 0.5 hour, 3 ml of water was added and the methanol removed under vacuum. The aqueous layer was decanted from the precipitated solid and the solid dissolved in 25 ml of ethyl acetate. This solution was washed with 5 ml of 1N sodium hydroxide, 10 ml of brine, dried ($Na_2SO_4$) and the solvent removed to give 0.44 g of solid. Trituration with ether gave 0.42 g of white solid which was chromatographed on silica gel with dichloromethane-methanol-ammonium hydroxide (10:1:0.5) as eluent to give 0.40 g of white solid; $[\alpha]_D^{26}$ -12° ±1 (c = 0.910, methanol).

## Example 36

### N-[N-(Diethoxyphosphinyl)- L -phenylalanyl- L -histidyl]-(S)-2-amino-3-cyclohexyl-(R)-1-(2-imidazolyl)propan-1-ol

As described for Example 34, N-(diethoxyphosphinyl)- L -phenylalanine (0.30 g) was coupled with N-( L -histidyl)-(S)-2-amino-3-cyclohexyl-(R)-1-(2-[N-(2-trimethylsilyl)ethoxy)methyl]imidazolyl)propan-1-ol (0.18 g) to give 0.13 g of a solid after work-up as for Example 34. This solid was dissolved in hexamethylphosphoramide (2 ml) and 0.07 ml of ethylenediamine and 0.85 ml of a solution of tetra-n-butylammonium fluoride in tetrahydrofuran (1.0M) added. The mixture was stirred at room temperature for 2.5 days and concentrated. This solution was added to a stirred solution of saturated sodium bicarbonate (20 ml). The suspension was filtered and the solid washed with two 2 ml portions of water and dried in air. Trituration with diethyl ether-ethyl acetate (2:1) gave 60 mg of product as a white solid; $[\alpha]_D^{26}$ -17° ±2 (c = 0.495, methanol).

## Example 37

### N-[N-(Diethoxyphosphinyl)- L -3-(1′-naphthyl)alanyl- L -leucyl]-(S)-2-amino-3-cyclohexyl-(R)-1-(2-furanyl)propan-1-ol

A solution of 0.25 g of L -3-(1′-naphthyl)alanine in 5 ml of methanol was saturated with anhydrous hydrogen chloride gas at 0 °C and then stirred overnight. The solvent was removed under vacuum and methanol added and removed several times. The residue in diethyl ether was chilled and the precipitate filtered off to give 0.260 g of methyl- L -3-(1′-naphthyl)alanate hydrochloride as a white solid; $[\alpha]_D^{26}$ +29° ±1 (c = 1,036, methanol) .

To a slurry of the preceding compound (1.27 g) in 55 ml of dichloromethane under argon was added 1.46 ml of triethylamine and 0.763 g of diethyl phosphoryl chloride. The mixture was stirred at room temperature overnight and then washed with 10% sodium bicarbonate solution and brine. The solvent was removed, the residue dissolved in ethyl acetate-dichloromethane (1:1) and the solution filtered through a thin pad of hydrous magnesium silicate. The pad was washed with ethyl acetate-dichloromethane (1:1) and the filtrate concentrated to give 1.76 g of crystals. Recrystallization from diisopropyl ether gave 1.62 g of methyl N-(diethoxyphosphinyl)- L -3-(1′-naphthyl)alanate as white crystals, mp 110-112 °C; $[\alpha]_D^{26}$ -23±1 (c = 1.093, methanol).

To the preceding compound (0.350 g) in 15 ml of methanol was added 2.88 ml of 1M sodium hydroxide and the mixture stirred for 2.5 hours at room temperature. Acid (3N HCl) was added until the mixture was acidic and the solvent removed. The residue was extracted with dichloromethane, the extracts dried

($MgSO_4$) and the solvent removed to give N-(diethylphosphinyl)- L -3-(1′-naphthyl)alanine.

The preceding compound (residue) was dissolved in 12 ml of dichloromethane under argon and 96.9 mg of triethylamine and 0.424 g of benzotriazol-1-yloxytris(dimethylamino)phosphonium hexafluorophosphate (BOP) added. After one minute 0.293 g N-( L -leucyl)-(S)-2-amino-3-cyclohexyl-(R)-1-(2-furanyl)propan-1-ol was added and the mixture was stirred at room temperature overnight. The solvent was removed and the residue in ethyl acetate washed with 2M citric acid, 10% sodium bicarbonate, water and dried ($MgSO_4$). The solvent was removed to give 0.60 g of tan solid. This solid was dissolved in ethyl acetate and filtered through a pad of silica gel. The pad was washed with ethyl acetate and the filtrate evaporated to give 0.47 g of crystals; $[\alpha]_D^{26}$ -46° ±1 (c = 1.134, methanol); Mass spectrum (FAB): calc. 692.3441; Found: 692.3428.

Following the above procedure the following compounds may be prepared:

N-N-(Diethoxyphosphinyl)- L -3-(1′-naphthyl)alanyl- L -leucyl]-(S)-2-amino-3-cyclohexyl-(R)-1-(5-acetyl-2-furanyl)propan-1-ol

N-[N-(Diethoxyphosphinyl)- L -3-(1′-naphthyl)alanyl- L -leucyl]-(S)-2-amino-3-cyclohexyl-(R)-1-(5-ethoxycarbonyl-2-furanyl)propan-1-ol

N-[N-(Diethoxyphosphinyl)- L -3-(1′-naphthyl)alanyl- L -leucyl]-(S)-2-amino-3-cyclohexyl-(R)-1-(5-chloro-2-furanyl)propan-1-ol

N-[N-(Diethoxyphosphinyl)- L -3-(1′-naphthyl)alanyl- L -leucyl]-(S)-2-amino-3-cyclohexyl)-(R)-1-(5-trifluoroacetyl-2-furanyl)propan-1-ol.

## Example 38

### N-[N-(Diethoxyphosphinyl)- L -(4-methoxyphenyl)alanyl- L -leucyl]-(S)-2-amino-3-cyclohexyl-(R)-1-(2-furanyl)propan-1-ol

To a stirred slurry of 0.88 g of L -(4-methoxyphenyl)alanine methyl ester hydrochloride in 40 ml of dichloromethane under argon was added 1.09 ml of triethylamine and 0.572 ml of diethyl phosphoryl chloride. The mixture was stirred overnight and then washed with 10% sodium bicarbonate solution and brine. The organic layer was concentrated under vacuum. The residue in ethyl acetate-dichloromethane (1:1) was filtered through a thin pad of hydrous magnesium silicate and the pad washed with ethyl acetate-dichloromethane (1:1) and the filtrate concentrated to dryness to an oil which crystallizes. The crystals were recrystallized from diisopropylether to give 0.932 g of methyl N-(diethoxyphosphinyl- L -(4-methoxyphenyl)-alanate as white crystals, mp 58.5-60.5° C; $[\alpha]_D^{26}$ -6° ±1 (c = 0.947, methanol).

To the preceding compound (0.179 g) in 10 ml of methanol was added 1.30 ml of 1M sodium hydroxide and the solution stirred for 1.5 hour. An additional 0.52 ml of 1M sodium hydroxide was added and after 45 minutes, 3N hydrochloric acid was added until mixture was acidic and the solvent removed. The residue was extracted with dichloromethane. The extracts dried ($MgSO_4$) and the solvent removed to give N-(diethoxyphosphinyl)- L -(4-methoxyphenyl)alanine as a gum.

This gum under argon was dissolved in 6 ml of dichloromethane and 52.3 mg of triethylamine and 0.229 g of benzotriazol-1-yloxytris(dimethylamino)phosphonium hexafluorophosphite (BOP). After stirring for one minute, 0.145 g of N-( L -leucyl)-(S)-2-amino-3-cyclohexyl-(R)-1-(2-furanyl)propan-1-ol was added and the mixture stirred overnight. The solvent was removed and the residue, dissolved in ethyl acetate, was washed with water. The organic layer was dried ($MgSO_4$) and the solvent removed. The residue was chromatographed on silica gel with solvent ethyl acetate-dichloromethane (1:1) to ethyl acetate (gradient elution) as eluent. The fractions containing product were combined and the solvent removed to give 0.170 g of a white foam; $[\alpha]_D^{26}$ -30° ±1 (c = 1.00, methanol).

Following the above procedure the following compounds may be prepared:

N-[N-(Diethoxyphosphinyl)- L -(4-methoxyphenyl)alanyl- L -leucyl]-(S)-2-amino-3-cyclohexyl-(R)-1-(5-bromo-2-furanyl)propan-1-ol

N-[N-(Diethoxyphosphinyl)- L -(4-methoxyphenyl)alanyl- L -leucyl]-(S)-2-amino-3-cyclohexyl-(R)-1-(5-acetyl-2-furanyl)propan-1-ol

N-[N-(Diethoxyphosphinyl)- L -(4-methoxyphenyl)alanyl- L -leucyl]-(S)-2-amino-3-cyclohexyl-(R)-1-(5-chloro-2-furanyl)propan-1-ol

N-[N-(Diethoxyphosphinyl)- L -(4-methoxyphenyl)alanyl- L -leucyl]-(S)-2-amino-3-cyclohexyl-(R)-1-(5-trifluoroacetyl-2-furanyl)propan-1-ol
N-[N-(Diethoxyphosphinyl)- L -(4-methoxyphenyl)alanyl- L -leucyl]-(S)-2-amino-3-cyclohexyl-(R)-1-(5-isopropoxycarbonyl-2-furanyl)propan-1-ol
N-[N-(Diethoxyphosphinyl)- L -(4-methoxyphenyl)alanyl- L -leucyl]-(S)-2-amino-3-cyclohexyl-(R)-1-(5-furan-2-carboxamide).

## Claims

1. A compound of the formula:

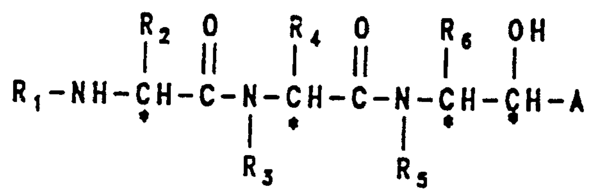

wherein $R_1$ is

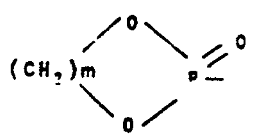

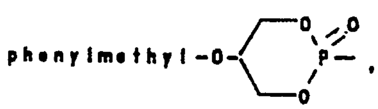

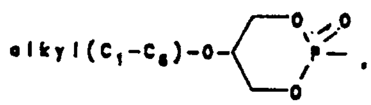

$[alkyl(C_1\text{-}C_6)O]_2\overset{O}{\overset{\|}{P}}$-,
$(phenyl\text{-}O)_2\overset{O}{\overset{\|}{P}}$-,
$[alkyl(C_1\text{-}C_6)][alkyl(C_1\text{-}C_6)O]\overset{O}{\overset{\|}{P}}$-,
$(phenylmethyl\text{-}O)_2\overset{O}{\overset{\|}{P}}$-,
$[alkyl(C_1\text{-}C_6)O][phenyl\text{-}O]\overset{O}{\overset{\|}{P}}$-,
$[phenyl(CH_2)_n][alkyl(C_1\text{-}C_6)O]\overset{O}{\overset{\|}{P}}$-,
$[phenyl(CH_2)_n][phenyl\text{-}O]\overset{O}{\overset{\|}{P}}$-,
$[alkyl(C_1\text{-}C_6)][phenylmethyl\text{-}O]\overset{O}{\overset{\|}{P}}$-,

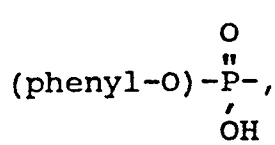

R- L -prolyl, R- L -thioprolyl, R- L -seryl, R- L -methionyl, R- L -alanyl, R- L -phenylalanyl, R- L -(S-benzyl)cysteinyl, R- L -histidyl or R-glycyl, where n is an integer 1-4; m is an integer 2-4 and R is selected

from one of the groups defined by $R_1$; $R_2$ is phenylmethyl, $-CH_2$(2-thienyl), $-CH_2$(3-indolyl), 4-methoxybenzyl or $-CH_2$-naphthyl; $R_3$ is hydrogen or methyl; $R_4$ is 4-(imidazolyl)$CH_2X$-, alkyl($C_1$-$C_8$), $-(CH_2)_n-NH_2$, phenylmethyl , cyclohexylmethyl, -X-alkyl($C_1$-$C_8$), X- cyclohexyl , $-(CH_2)_n$-X-alkyl($C_1$-$C_3$), $-X-CH_2CH_2N$[alkyl-($C_1$-$C_3$)$]_2$ (where X is -O- or -S-) and moieties of the formulae:

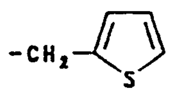

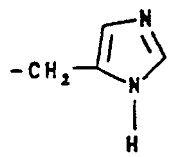

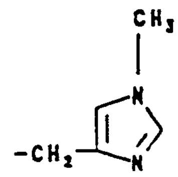

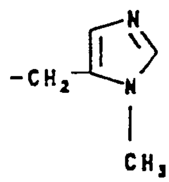

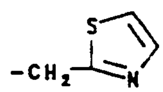

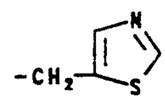

or 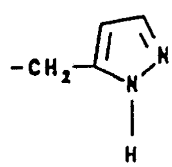

$R_5$ is hydrogen, or methyl; $R_6$ is alkyl($C_1$-$C_6$), phenylmethyl, cyclohexylmethyl, $-(CH_2)_n$-X-alkyl($C_1$-$C_3$) or

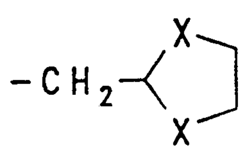

and A is

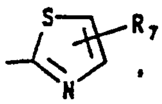 , 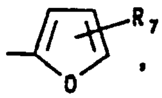 , 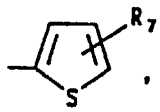 , 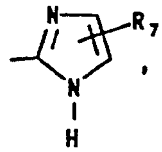 ,

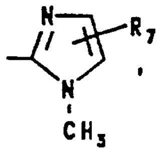 , 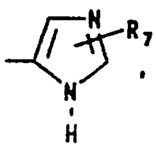 , 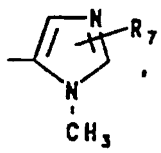 , 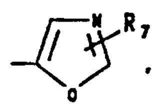 ,

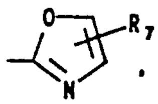 , 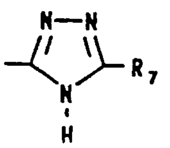 , 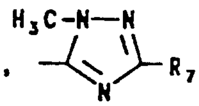 , 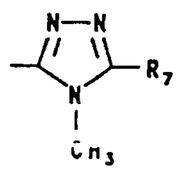 ,

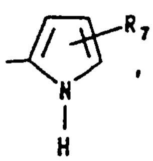 , 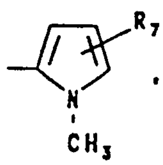 , 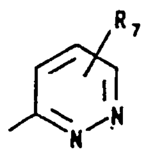 , 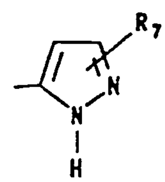 ,

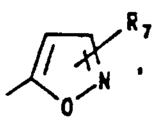 , 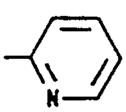 , 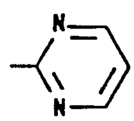 , 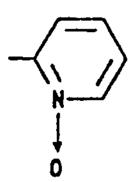 ,

or 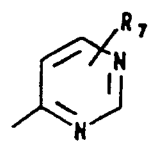

where $R_7$ is hydrogen, alkyl($C_1$-$C_3$), Br, Cl, F or $COR_8$, where $R_8$ is H, $CF_3$, $NH_2$, OH, -O-alkyl($C_1$-$C_4$), -NH-alkyl($C_1$-$C_4$), alkyl($C_1$-$C_6$), $CH_2OH$, -N[alkyl($C_1$-$C_3$)]$_2$ or

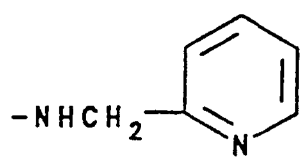

and wherein the asterisks denote asymmetric carbon atoms.

2. A compound according to Claim 1 wherein the α-amino acids have the natural L configuration.

3. A compound according to Claim 2 wherein the C-terminal unit is of the formula

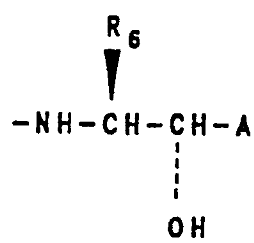

wherein $R_1$ is

$[alkyl(C_1\text{-}C_6)O]_2\text{-}\overset{O}{\overset{\|}{P}}\text{-}$,

$(phenyl\text{-}O)_2\overset{O}{\overset{\|}{P}}\text{-}$,

$[alkyl(C_1\text{-}C_6)][alkyl(C_1\text{-}C_6)O]\overset{O}{\overset{\|}{P}}\text{-}$,

$(phenylmethyl\text{-}O)_2\overset{O}{\overset{\|}{P}}\text{-}$, or

$[alkyl(C_1\text{-}C_6)O][phenyl\text{-}O]\overset{O}{\overset{\|}{P}}\text{-}$; $R_2$ is phenylmethyl, (3-indolyl)$CH_2$, (2-thienyl)$CH_2$ or $CH_2$-naphthyl, $R_3$ is hydrogen, $R_4$ is alkyl($C_1$-$C_8$),

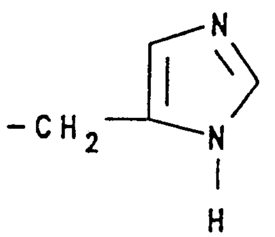
,

O-alkyl($C_1$-$C_8$), S-alkyl($C_1$-$C_8$) or O-$CH_2CH_2N$[alkyl($C_1$-$C_3$)]$_2$; $R_5$ is hydrogen; $R_6$ is alkyl($C_1$-$C_8$), cyclohexyl methyl, or

4. The compound according to Claim 3, N-[N-(diethoxyphosphinyl)- L -phenylalanyl- L -histidyl]-(S)-2-amino-3-cyclohexyl-(R)-1-(2-imidazolyl)propan-1-ol.

5. The compound according to Claim 3, N-[N-(diethoxyphosphinyl)- L -phenylalanyl- L -leucyl]-(S)-2-amino-3-cyclohexyl-(R)-1-(2-thiazolyl)propan-1-ol.

6. The compound according to Claim 3, N-[N-(diethoxyphosphinyl)- L -phenylalanyl- L -histidyl]-(S)-2-amino-3-cyclohexyl-(R)-1-(2-thienyl)propan-1-ol.

7. The compound according to Claim 3, N-[N-(diethoxyphosphinyl)- L -phenylalanyl- L -histidyl]-(S)-2-amino-3-cyclohexyl-(R)-1-(2-furanyl)propan-1-ol.

8. The compound according to Claim 3, N-[N-(dibenzyloxyphosphinyl)- L -phenylalanyl- L -histidyl]-(S)-2-amino-3-cyclohexyl-(R)-1-(2-thiazolyl)propan-1-ol.

9. The compound according to Claim 3, N-[N-(diethoxyphosphinyl)- L -3-(1′-naphthyl)alanyl- L -histidyl]-(S)-2-amino-3-cyclohexyl-(R)-1-(2-imidazolyl)propan-1-ol.

10. A process for preparing a compound of the formula

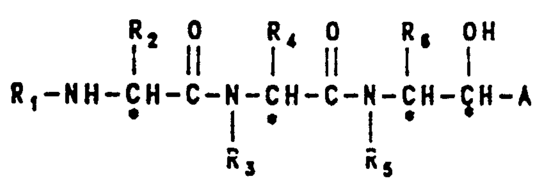

wherein $R_1$ is

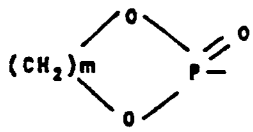 , 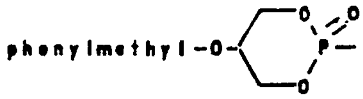 ,

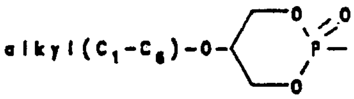 ,

$[alkyl(C_1-C_6)O]_2\overset{O}{\overset{\|}{P}}-$,

$(phenyl-O)_2\overset{O}{\overset{\|}{P}}-$,

$[alkyl(C_1-C_6)][alkyl(C_1-C_6)O]\overset{O}{\overset{\|}{P}}-$,

$(phenylmethyl-O)_2\overset{O}{\overset{\|}{P}}-$,

$[alkyl(C_1-C_6)O][phenyl-O]\overset{O}{\overset{\|}{P}}-$,

$[phenyl(CH_2)_n][alkyl(C_1-C_6)O]\overset{O}{\overset{\|}{P}}-$,

$[phenyl(CH_2)_n][phenyl-O]\overset{O}{\overset{\|}{P}}-$,

$[alkyl(C_1-C_6)][phenylmethyl-O]\overset{O}{\overset{\|}{P}}-$,

$$(phenyl-O)-\underset{OH}{\overset{O}{\overset{\|}{P}}},$$

R-$\underline{\underline{L}}$-prolyl, R-$\underline{\underline{L}}$-thioprolyl, R-$\underline{\underline{L}}$-seryl, R-$\underline{\underline{L}}$-methionyl, R-$\underline{\underline{L}}$-alanyl, R-$\underline{\underline{L}}$-phenylalanyl, R-$\underline{\underline{L}}$-(S-benzyl)cysteinyl, $R_1$-$\underline{\underline{L}}$-histidyl or $R_1$-$\underline{\underline{L}}$-glycyl, where n is an integer 1-4; m is an integer 2-4 and R is selected from one of the groups defined by $R_1$; $R_2$ is phenylmethyl, $-CH_2$(2-thienyl), $-CH_2$(3-indolyl), 4-methoxybenzyl or $-CH_2$-naphthyl; $R_3$ is hydrogen or methyl; $R_4$ is 4-(imidazolyl)$CH_2X$-, alkyl($C_1$-$C_8$), $-(CH_2)_n-NH_2$, phenylmethyl, cyclohexylmethyl , -X-alkyl($C_1$-$C_8$), X-cyclohexyl, $-(CH_2)_n$-X-alkyl($C_1$-$C_3$), $-X-CH_2CH_2N$[alkyl($C_1$-$C_3$)]$_2$ (where X is -O- or -S-) or moieties of the formulae:

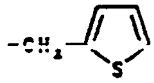 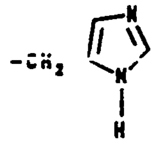 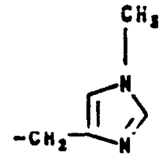

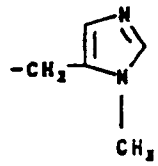 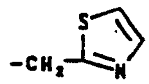 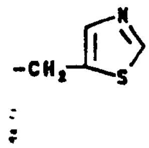

or 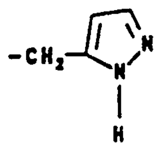

$R_5$ is hydrogen or methyl; $R_6$ is alkyl($C_1$-$C_6$), phenylmethyl, cyclohexylmethyl, $-(CH_2)_n$-X-alkyl($C_1$-$C_3$) or

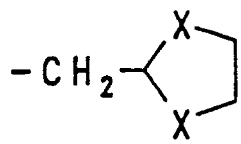

and A is

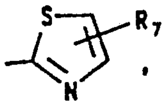, 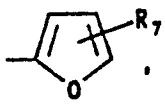, 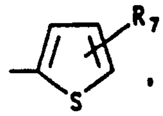, 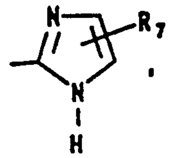,

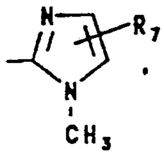, 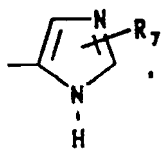, 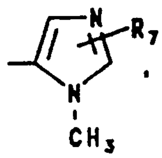, 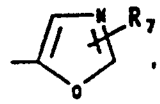,

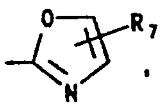, 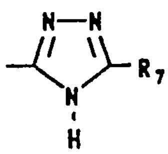, 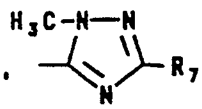, 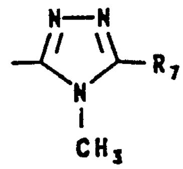,

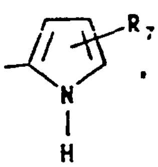, 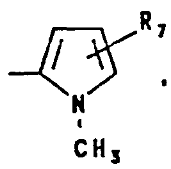, 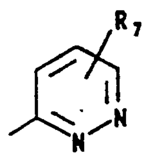, 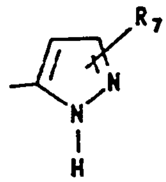,

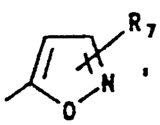, 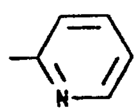, 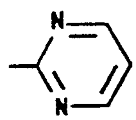, 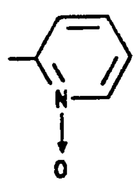,

or 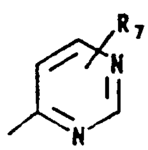

which comprises activating a compound of the formula,

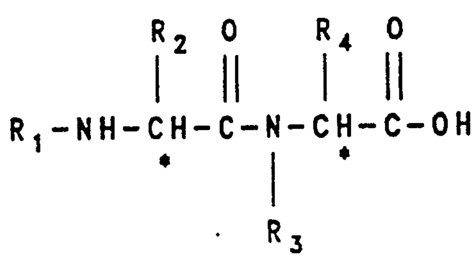

with a peptide coupling reagent of N,N-dicyclohexylcarbodiimide, N-N′-dicyclohexylcarbodiimide plus -hydroxysuccinimide, N-N′-dicyclohexylcarbodiimide plus 1-hydroxy-benzotriazol, 1-benzotriazolyl diethyl phosphate, 1-chloro-N,N-2-trimethyl-1-propen-1-amine, diphenyl phosphoryl azide, diethylphosphorochloridate, di-loweralkyl($C_1$-$C_8$) phosphorochloridates, diphenyl phosphorochloridate, phenyl phosphorodichloridate benzotriazol-1-yloxytris(dimethylamino)phosphonium hexafluorophosphate , N,N′-bis[2-oxo-3-oxazolidinyl]phosphorodiamidic chloride, diphenylphosphinyl chloride, diethoxyphosphoryl cyanide, N,N-carbonyldiimidazole, or isobutyl chloroformate plus N-methylmorpholine, 2-chloro-1-methylpyridinium iodide to give an intermediate of the formula

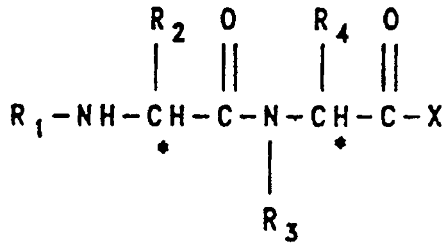

(wherein X is an activating moiety); reacting the intermediate at -10° C to 25° C in tetrahydrofuran, dioxane, dichloromethane or ethyl acetate with a 1-amino-2-hydroxy compound of formula

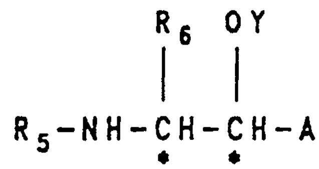

wherein Y is hydrogen or a removable blocking group of trimethylsilyl, t-butyldimethyl-silyl, tetrahydropyranyl, acetyl, or benzoyl and when Y is a protecting group removing the hydroxyl protecting group.